# EUROPEAN PATENT SPECIFICATION

(11) **EP 3 302 440 B1**
(45) Date of publication and mention of the grant of the patent: **06.01.2021**
(21) Application number: 16729739.9
(22) Date of filing: 03.06.2016
(51) Int. Cl.: A61K 9/20, A61K 9/28, A61K 38/10, A61P 1/00, A61P 29/02, A61P 13/10

(54) **MODIFIED OR TARGETED RELEASE FORMULATIONS OF LINACLOTIDE**
MODIFIZIERTE ODER GEZIELTE FREISETZUNGSFORMULIERUNGEN VON LINACLOTID
FORMULATIONS À LIBÉRATION MODIFIÉE OU CIBLÉE DE LINACLOTIDE

(30) Priority: 05.06.2015 US 201562171462 P; 20.05.2016 US 201662339462 P
(43) Date of publication of application: 11.04.2018
(73) Proprietor: Ironwood Pharmaceuticals, Inc., Boston, MA 02110 (US)
(72) Inventor: HASHASH, Ahmad, Southborogh, MA 01772 (US)
(74) Representative: Mathys & Squire
(86) International application number: PCT/US2016/035886
(87) International publication number: WO 2016/197042

(56) References cited:
- WO-A1-2015/089326
- WO-A2-2012/021715
- US-A1- 2010 048 489
- US-A1- 2011 059 903
- US-A1- 2013 012 454
- US-A1- 2014 242 158

### SEQUENCE LISTING

<110> Ironwood Pharmaceuticals, Inc.
<120> Modified or Targeted Release Formulations of Linaclotide
<130> 223355/158PCT1/395990
<140> tbd
   <141> 2016-06-03
<150> 62/171,462
   <151> 2016-05-20
<150> 62/339,462
   <151> 2015-06-05
<160> 9
<170> PatentIn version 3.5
<210> 1
   <211> 14
   <212> PRT
   <213> Artificial Sequence
<220>
   <223> Synthetically generated peptide
<220>
   <221> DISULFID
   <222> (1)..(6)
<220>
   <221> DISULFID
   <222> (2)..(10)
<220>
   <221> DISULFID
   <222> (5)..(13)
<400> 1
   Cys Cys Glu Tyr Cys Cys Asn Pro Ala Cys Thr Gly Cys Tyr
   1               5                   10
<210> 2
   <211> 14
   <212> PRT
   <213> Artificial Sequence
<220>
   <223> Synthetically generated peptide
<220>
   <221> MISC_FEATURE
   <222> (1)..(1)
   <223> Xaal is a Cys, wherein the alpha-amine of the Cys is deaminated.
<220>
   <221> DISULFID
   <222> (1)..(6)
   <223> Disulfide bond is between the deaminated alpha-amine of Xaal and Cys6.
<220>
   <221> DISULFID
   <222> (2)..(10)
<220>
   <221> DISULFID
   <222> (5)..(13)
<400> 2
   Xaa Cys Glu Tyr Cys Cys Asn Pro Ala Cys Thr Gly Cys Tyr
   1               5                   10
<210> 3
   <211> 14
   <212> PRT
   <213> Artificial Sequence
<220>
   <223> Synthetically generated peptide
<220>
   <221> MISC_FEATURE
   <222> (1)..(6)
   <223> Trisulfide bond between Cys1 and Cys6.
<220>
   <221> DISULFID
   <222> (2)..(10)
<220>
   <221> DISULFID
   <222> (5)..(13)
<400> 3
   Cys Cys Glu Tyr Cys Cys Asn Pro Ala Cys Thr Gly Cys Tyr
   1               5                   10
<210> 4
   <211> 14
   <212> PRT
   <213> Artificial Sequence
<220>
   <223> Synthetically generated peptide
<220>
   <221> DISULFID
   <222> (1)..(6)
<220>
   <221> DISULFID
   <222> (2)..(10)
<220>
   <221> MISC_FEATURE
   <222> (5)..(13)
   <223> Trisulfide bond between Cys5 and Cysl3.
<400> 4
   Cys Cys Glu Tyr Cys Cys Asn Pro Ala Cys Thr Gly Cys Tyr
   1               5                   10
<210> 5
   <211> 14
   <212> PRT
   <213> Artificial Sequence
<220>
   <223> Synthetically generated sequence
<220>
   <221> DISULFID
   <222> (1)..(6)
<220>
   <221> MISC_FEATURE
   <222> (2)..(10)
   <223> Trisulfide bond between Cys2 and Cys10.
<220>
   <221> DISULFID
   <222> (5)..(13)
<400> 5
   Cys Cys Glu Tyr Cys Cys Asn Pro Ala Cys Thr Gly Cys Tyr
   1               5                   10
<210> 6
   <211> 14
   <212> PRT
   <213> Artificial Sequence
<220>
   <223> Synthetically generated peptide
<220>
   <221> MISC_FEATURE
   <222> (1)..(1)
   <223> Xaal is a Cys, wherein the alpha-amine of the Cys which is cross-linked to the amine group of Cys2 to form an imidazolidinone 5 membered ring at the N-terminus of the peptide.
<220>
   <221> DISULFID
   <222> (1)..(6)
   <223> Disulfide bond is between the imidazolidinone 5-membered ring of Xaal and Cys6.
<220>
   <221> DISULFID
   <222> (2)..(10)
<220>
   <221> DISULFID
   <222> (5)..(13)
<400> 6
   Xaa Cys Glu Tyr Cys Cys Asn Pro Ala Cys Thr Gly Cys Tyr
   1               5                   10
<210> 7
   <211> 14
   <212> PRT
   <213> Artificial Sequence
<220>
   <223> Synthetically generated peptide
<220>
   <221> DISULFID
   <222> (1)..(6)
<220>
   <221> DISULFID
   <222> (2)..(10)
<220>
   <221> DISULFID
   <222> (2)..(13)
<400> 7
   Cys Cys Glu Tyr Cys Cys Asp Pro Ala Cys Thr Gly Cys Tyr
   1               5                   10
<210> 8
   <211> 14
   <212> PRT
   <213> Artificial Sequence
<220>
   <223> Synthetically generated peptide
<220>
   <221> MOD_RES
   <222> (1)..(1)
   <223> ACETYLATION
<220>
   <221> DISULFID
   <222> (1)..(6)
<220>
   <221> DISULFID
   <222> (2)..(10)
<220>
   <221> DISULFID
   <222> (5)..(13)
<400> 8
   Cys Cys Glu Tyr Cys Cys Asn Pro Ala Cys Thr Gly Cys Tyr
   1               5                   10
<210> 9
   <211> 13
   <212> PRT
   <213> Artificial Sequence
<220>
   <223> Synthetically generated peptide
<220>
   <221> DISULFID
   <222> (1)..(6)
<220>
   <221> DISULFID
   <222> (2)..(10)
<220>
   <221> DISULFID
   <222> (5)..(13)
<400> 9
   Cys Cys Glu Tyr Cys Cys Asn Pro Ala Cys Thr Gly Cys
   1               5                   10

## Description

### FIELD OF THE INVENTION

The present invention relates to delayed release pharmaceutical compositions comprising linaclotide or pharmaceutically acceptable salts thereof, as well as to various methods and processes for the preparation and use of the compositions.

### BACKGROUND OF THE INVENTION

Various formulation techniques have been used to develop delayed release compositions for pharmaceutically active agents including the use of enteric coatings or pH responsive polymers. However, the specific components of these compositions vary greatly and depend significantly on the particular pharmaceutically active agent and the desired properties. For example, the formulation must be compatible with the pharmaceutically active agent and also provide the necessary dissolution performance and stability properties.

U.S. Patents 7,304,036 and 7,371,727 disclose peptides that act as agonists of the guanylate cyclase C (GC-C) receptor for the treatment of gastrointestinal (GI) disorders. One particular peptide disclosed is linaclotide, which consists of the following amino acid sequence: Cys Cys Glu Tyr Cys Cys Asn Pro Ala Cys Thr Gly Cys Tyr. Linaclotide has the chemical structure of:

Linaclotide is orally administered and has been approved in the U.S. by the FDA for the treatment of irritable bowel syndrome with constipation (IBS-c) and chronic idiopathic constipation (CIC). In humans, linaclotide has been shown to affect GI physiology including reducing visceral pain, reducing bloating and accelerating GI transit which can lead to increased stool frequency and improved stool consistency. Orally administered linaclotide acts locally by binding to and activating GC-C receptors at the luminal surface of the intestine. The GC-C receptor is a key regulator in mammals of intestinal function and is found throughout the luminal surface of the GI tract. The GC-C receptor responds to the endogenous hormones, guanylin and uroguanylin, and to enteric bacterial peptides from the heat stable enterotoxin family (ST peptide). When linaclotide binds to the GC-C receptor, there is an intracellular elevation of the second messenger, cyclic GMP (c-GMP), and an increase in chloride and bicarbonate secretion, resulting in an increase in intestinal fluid secretion and reducing abdominal pain.

As approved by the FDA, linaclotide is administered in an oral, solid, immediate-release capsule formulation manufactured by filling drug-layered beads into gelatin capsules. Due to the high expression of GC-C receptors throughout GI tract, linaclotide from an immediate release formulation activates the GC-C receptor starting from the upper GI tract, resulting in significant amount of intestinal fluid being brought to the lower GI tract. To reduce or mitigate this effect, delayed release compositions are needed which have targeted release of linaclotide in the distal or lower segment of the gastrointestinal tract. Targeting the lower GI for linaclotide release may help avoid excess fluid secretion but at the same time maintain or improve linaclotide efficacy for treating abdominal and bowel symptoms of GI disorders.

An estimated 13 million adults in the U.S. have IBS-c and 35 million adults have CIC. An estimated 15 million adults also suffer from IBS-m. Currently, 6.3 million adults are seeking care and are not satisfied with current treatments for IBS-m. Abdominal or visceral pain is a pre-dominant symptom reported by patients suffering from IBS, CIC, and other gastrointestinal disorders. As a result, there is a need for therapies that provide improved relief of visceral pain and greater tolerability for adults suffering from IBS-c, IBS-m, and CIC.

Linaclotide has been previously demonstrated to reduce visceral pain which is thought to be mediated by increasing cGMP in the GI tract. Animal studies have shown that orally administered linaclotide can treat colonic hypersensitivity and hyperalgesia. However, due to the reducing environment of the intestinal tract, it is known that much of the oral linaclotide dose is degraded prior to reaching the distal colon. In human volunteers treated with linaclotide, only about 5% of the oral dose is found in feces. Delayed release ("DR") compositions of linaclotide that target the lower GI may improve linaclotide's efficacy towards relieving pain associated with various GI disorders by allowing for delivery of a higher dose of linaclotide to the colon. Such DR compositions of linaclotide would have the potential to release linaclotide predominantly (or fully) in the lower GI. As a result, for example, the DR formulation or composition may have an increased capacity to treat lower GI associated disorders. Moreover, it may have a capacity to cause lower incidences of adverse events (such as diarrhea) than the immediate-release dosage form, e.g., because it would cause lower overall intestinal fluid secretion by not activating GC-C receptors in the upper GI. This would occur while maintaining or even improving linaclotide efficacy for treating symptoms of GI disorders, such as pain.

Despite the need for delayed release compositions of linaclotide, difficulties exist in preparing such formulations due to the intrinsic chemical instability of linaclotide, for example, by moisture induced degradation reactions such as hydrolysis, deamidation, isomerization, and multimerization. These difficulties may be exacerbated when producing formulations having lower dosages of linaclotide.

Accordingly, there is an existing and continual need for delayed release compositions that provide stable and reliable delivery of linaclotide to targeted areas of the gastrointestinal tract.

US 2011/059903 A1 relates to stable formulations comprising linaclotide.

### SUMMARY OF THE INVENTION

The invention is defined by the claims. Any subject-matter falling outside the scope of the claims is provided for information purposes only.

In particular, the invention concerns a delayed release composition comprising an enteric coated tablet, wherein the tablet comprises:
linaclotide;
Ca²⁺;
histidine;
and polyvinyl alcohol (PVA),
wherein the linaclotide is present in the composition in an amount between 30 µg and 300 µg. The invention also concerns a unit dosage form comprising the above composition, a method of making the above composition, the composition prepared by this method, the above compositions for use in a method of treating a gastrointestinal disorder in a patient, the above compositions for use in a method of treating or relieving pain in a patient, and the above compositions for use in a method of treating overactive bladder syndrome, bladder hypersensitivity or colitis induced bladder afferent hyperactivity in a patient.

More generally, the present disclosure relates to stable, solid, oral dosage forms of linaclotide which exhibit delayed release of linaclotide to the lower gastrointestinal tract. In addition, the present disclosure provides methods of treating conditions by administering these delayed release compositions. The delayed release compositions of the present disclosure may be used to treat various conditions, but is particularly suited to treat gastrointestinal disorders, such as irritable bowel syndrome ("IBS") (for example, IBS with constipation "IBS-c", IBS with diarrhea "IBS-d", or mixed IBS with constipation and diarrhea "IBS-m"), constipation (for example, chronic idiopathic constipation), colon cancer, diverticulitis, interstitial cystitis, and abdominal or visceral pain.

According to some embodiments of the present disclosure, methods are disclosed for the treatment of a gastrointestinal disorder or pain comprising administering to a patient in need thereof, a composition disclosed herein.
**E1.** According to a first aspect, there is provided a delayed release composition comprising an enteric coated tablet, wherein the tablet comprises:
   i. linaclotide;
   ii. Ca²⁺;
   iii. histidine; and
   iv. polyvinyl alcohol (PVA),
   wherein the linaclotide is present in the composition in an amount between 30 µg and 300 µg.
**E2.** The delayed release composition of E1, wherein the linaclotide is present in an amount of 30 µg.
**E3.** The delayed release composition of E1-E2, wherein the linaclotide is present in an amount of 100 µg.
**E4.** The delayed release composition of E1-E3, wherein the linaclotide is present in an amount of 300 µg.
**E5.** The delayed release composition of E1-E4, wherein the tablet comprises at least 1.25% (w/w) of PVA.
**E6.** The delayed release composition of E1-E4, wherein the tablet comprises at least 1.49% (w/w) of PVA.
**E7.** The delayed release composition according to any one of E1 - E6, wherein the tablet comprises at least 0.44% (w/w) of CaCl₂.
**E8.** The delayed release composition according to any one of E1 - E7, wherein the tablet comprises at least 0.71% (w/w) of CaCl₂.
**E9.** The delayed release composition according to any one of E1 - E8, wherein the tablet comprises at least 0.93% (w/w) of histidine.
**E10.** The delayed release composition according to any one of E1 - E9, wherein the tablet comprises at least 1.49% (w/w) of histidine.
**E11.** The delayed release composition according to any one of E1-E10, wherein the enteric coating comprises methyl acrylate-methacrylic acid copolymers (e.g. Eudragit®).
**E12.** The delayed release composition according to any one of E1-E11, wherein the enteric coating comprises Eudragit® S100, Eudragit®L100, or Eudragit® S100/Eudragit®L100 mixture.
**E13.** The delayed release composition according to any one of E1 - E12, further comprising a protective polymer film or subcoating.
E14. The delayed release composition of E13, wherein the subcoating comprises Opadry II®.
**E15.** A unit dosage form comprising the delayed release composition of any one of El - E14.
**E16.** A method of making the composition of any one of E1 - E15, comprising:
   preparing a linaclotide base, pregranulated filler, and placebo base;
   blending and compressing the linaclotide base, pregranulated filler, and placebo base into a tablet; and applying an enteric coating to the tablet.
**E17.** The method of E16, wherein the pregranulated filler is prepared through wet granulation and dried before blending and compressing into a tablet.
**E18.** The method of E16 or E17, wherein the method further comprises applying a subcoat to the tablet.
**E19.** The method of E18, wherein the subcoat comprises Opadry II®.
**E20.** The method of E16-E19, wherein the enteric coating comprises Eudragit® S100, Eudragit®L100, or Eudragit® S100/Eudragit®L100 mixture.
**E21.** A composition prepared by the method of any one of E16 - E20.
**E22.** A composition of any one of E1 -E14 or E21 for use in a method of treating a gastrointestinal disorder in a patient.
**E23.** The composition for use of E22, wherein the gastrointestinal disorder is selected from the group consisting of: irritable bowel syndrome (IBS), constipation, a functional gastrointestinal disorder, gastroesophageal reflux disease, functional heartburn, dyspepsia, diverticulitis, visceral pain, abdominal pain, gastroparesis, chronic intestinal pseudo-obstruction, colonic pseudo-obstruction, Crohn's disease, ulcerative colitis, and inflammatory bowel disease.
**E24.** The composition for use of E22, wherein the gastrointestinal disorder is constipation.
**E25.** The composition for use of E24, wherein the constipation is chronic constipation, idiopathic constipation, chronic idiopathic constipation, constipation due to post-operative ileus, or constipation caused by opiate use.
**E26.** The composition for use of E22, wherein the gastrointestinal disorder is irritable bowel syndrome (IBS).
**E27.** The composition for use of E26, wherein the irritable bowel syndrome is constipation-predominant irritable bowel syndrome (IBS-c), diarrhea-predominant irritable bowel syndrome (IBS-d) or mixed irritable bowel syndromes (IBS-m).
**E28.** The composition for use of E22, wherein the gastrointestinal disorder is dyspepsia.
**E29.** The composition for use of E22, wherein the gastrointestinal disorder is gastroparesis.
**E30.** The composition for use of E29, wherein said gastroparesis is idiopathic, diabetic or post-surgical gastroparesis.
**E31.** The composition for use of E22, wherein the gastrointestinal disorder is chronic intestinal pseudo obstruction.
**E32.** The composition for use of E22, wherein the gastrointestinal disorder is Crohn's disease.
**E33.** The composition for use of E22, wherein the gastrointestinal disorder is ulcerative colitis.
**E34.** The composition for use of E22, wherein the gastrointestinal disorder is inflammatory bowel disease.
**E35.** The composition for use of E22, wherein the gastrointestinal disorder is visceral pain.
**E36.** The composition for use of E22, wherein the gastrointestinal disorder is diverticulitis.
**E37.** The composition for use of E22, wherein the gastrointestinal disorder is abdominal pain.
**E38.** The composition for use of E22, wherein the gastrointestinal disorder is cancer selected from colorectal/local metastasized colorectal cancer, gastric cancer, intestinal polyps, gastrointestinal tract cancer, cancer or pre-cancerous growths or metastatic growths of epithelial cells or polyps of colorectal tissue.
**E39.** A composition of any one of E1-E14 or E21 for use in a method of treating or relieving pain in a patient.
**E40.** The composition for use of E39, wherein the pain is selected from visceral pain; diverticulitis pain; pelvic; abdominal pain; or pain associated with gastrointestinal disorders, venereal diseases, bladder pain syndrome, or interstitial cystitis.
**E41.** The composition for use of E39, wherein the pain is selected from general abdominal pain, diverticular disease, pain associated with irritable bowel syndrome (IBS), chronic or acute radiation proctopathy (also referred to as radiation proctitis), rectal pain, chronic proctalgia, proctalgia fugax, anal pain, chronic anal fissure, post-operative anal pain, overactive bladder syndrome, stress incontinence, interstitial cystitis, bladder pain syndrome, pain associated with cancer, pain associated with gastrointestinal tract neoplasms, general pelvic pain, endometriosis, orchialgia, chronic prostatitis, prostatodynia, vulvodynia, urethral syndrome, penile pain, perianal pain, and pain associated with ulcerative colitis, ulcerative proctitis, or Crohn's disease.
**E42.** A composition of any one of E1-E14 or E21 for use in a method of treating overactive bladder syndrome, bladder hypersensitivity or colitis induced bladder afferent hyperactivity in a patient.
**E43.** A composition of any one of E1-E14 or E21 for use in a method for increasing intestinal motility in a patient.
**E44.** A composition of any one of E1-E14 or E21 for use in a method of increasing guanylate cyclase C (GC-C) receptor activity in a biological sample or organism, comprising contacting said biological sample or organism with said composition.
**E45.** A method of making the composition of any of E1- E14, comprising:
   i. preparing an aqueous solution comprising linaclotide, or a pharmaceutically acceptable salt thereof; and
   ii. applying the aqueous solution to a pharmaceutically acceptable carrier.
**E46.** A composition prepared by the method of E45.

### BRIEF DESCRIPTION OF THE FIGURES

Figure 1 shows the release profile at various pHs for delayed release tablet compositions comprising 100µg linaclotide.
Figure 2 shows bladder afferent and compliance responses to ramp distension in CVH mice treated for 14 days with once daily oral gavage of vehicle (black, N=6) or linaclotide (grey, N=13).
Figure 3 shows bladder afferent responses to: A, carbachol (1µM) p≤0.001, B, capsaicin (10µM) p ≤S0.01, C, αβMe-ATP (30µM) p≤0.001 in CVH mice with once daily oral gavage of vehicle (black, n=7) or linaclotide (grey, n=7) (2-way ANOVA Bonferroni multiple comparisons test).
Figure 4 shows electrophysiological properties of retrogradely traced bladder thoracolumbar (TL) DRG neurons from CVH mice with once daily oral gavage of vehicle (n=27) or linaclotide (n=25). A, linaclotide significantly increases the rheobase compared to vehicle group (p=0.02) B, linaclotide significantly decreases the number of action potentials at 2X rheobase compared to vehicle group (p=0.02) C, linaclotide has no effect on resting membrane potential compared to mice receiving vehicle gavage.
Figure 5 shows rheobase of retrogradely traced bladder thoracolumbar (TL) DRG neurons from healthy mice (n=19), CVH mice (n=22), and CVH mice with once daily oral gavage of vehicle (n=27) or linaclotide (n=25). Rheobase is significantly reduced in CVH mice and this effect is reversed by oral gavage of linaclotide (p≤0.05, one-way ANOVA).
Figure 6 shows stability testing and percent/negative loss of linaclotide over time for representative formulations of linaclotide.
Figure 7 shows stability testing and percent gain of the α-ketone degradant product ("Cys¹-α-Ketone") of linaclotide over time for representative formulations of linaclotide.
Figure 8 shows stability testing and percent gain of the immidazolidinone degradant product ("Cys1-IMD") of linaclotide over time for representative formulations of linaclotide.
Figure 9 shows stability testing and percent/negative loss of linaclotide over time for Formulations I and 64B of linaclotide.

### DETAILED DESCRIPTION OF THE INVENTION

Delayed release oral dosage forms of linaclotide (collectively, "DR") are provided herein. Until now, the only approved formulation of linaclotide is a capsule that exhibits immediate release ("IR"). These IR dosage forms release most or all of the linaclotide contained therein in the upper GI. This, in turn, causes GC-C receptor activation and fluid secretion in both the upper GI and to a lesser extent in the lower GI. The difference between upper and lower GI activation and fluid secretion by the IR dosage form is due, in part, to the fact that linaclotide (once released from the dosage form) undergoes proteolytic digestion and loses some or all capacity to activate GC-C receptors, particularly by the time it reaches the lower GI (such as the ileum, terminal ileum, ileocecal valve, or colon).

The DR dosage forms described herein release most or all of the linaclotide contained therein within the lower GI, such as proximate to the ileocecal valve or within the colon (and less or no release in the stomach, duodenum and/or jejunum). Therefore, the inventive dosage forms have a capacity to achieve lower overall fluid secretion than IR dosage forms in the upper GI, while improving or still maintaining excellent efficacy for treating IBS-c, CIC, and for modulating the gastrointestinal pain sensory signaling. IBS patients report lower left quadrant abdominal pain as a symptom of their disorder, so it is believed that the pain of IBS originates from the colon. Moreover, the DR dosage forms are believed to be ideally suited for treating lower GI-associated diseases and disorders. Because the DR dosage forms will not release any (or a small percentage) of its linaclotide in the stomach and upper GI (which can cause rapid digestion of the linaclotide in the intestine), some preferred embodiments of the DR dosage form will incorporate low doses of linaclotide (as compared to the amounts in the approved IR form) but will maintain the same efficacy levels as the IR in treating IBS-c and CIC symptoms.

In general, guanylate cyclase C (GC-C) receptor is a transmembrane receptor that is located on the apical surface of epithelial cells in the stomach and intestine. The receptor has an extracellular ligand-binding domain, a single transmembrane region and a C-terminal guanylyl cyclase domain. When a ligand binds to the extracellular domain of GC-C, the intracellular catalytic domain catalyzes the production of cGMP from GTP. In vivo, this increase in intracellular cGMP initiates a cascade of events that leads to increased secretion of chloride and bicarbonate into the intestinal lumen, increased luminal pH, decreased luminal sodium absorption, increased fluid secretion, and acceleration of intestinal transit. cGMP is secreted bi-directionally from the epithelium into the submucosa and lumen. Normally, the pH of the GI tract gradually increases from stomach (pH 1.5-3) to terminal ileum (pH 7-8) before it drops in the colon to pH 5.5-7.0.

Linaclotide binds to the intestinal GC-C receptor which is a regulator of fluid and electrolyte balance in the intestine. Linaclotide is a peptide that consists of the amino acid sequence Cys₁ Cys₂ Glu₃ Tyr₄ Cys₅ Cys₆ Asn₇ Pro₈ Ala₉ Cys₁₀ Thr₁₁ Gly₁₂ Cys₁₃ Tyr₁₄. Any desired form of linaclotide may be used in the composition, for example, any pharmaceutically acceptable salt or hydrate of the peptide, any isolated and/or purified form thereof, or any disulfide form thereof. Linaclotide has disulfide bonds between Cys₁ and Cys₆, Cys₂ and Cys₁₀, and Cys₅ and Cys₁₃.

The DR compositions containing linaclotide can be used to treat a variety of disorders. In various embodiments, the patient is suffering from a gastrointestinal disorder; the patient is suffering from a disorder selected from the group consisting of: gastrointestinal motility disorders, chronic intestinal pseudo-obstruction, colonic pseudo-obstruction, inflammatory bowel disease, Crohn's disease, duodenogastric reflux, dyspepsia, functional dyspepsia, nonulcer dyspepsia, a functional gastrointestinal disorder, functional heartburn, gastroesophageal reflux disease (GERD), gastroparesis, irritable bowel syndrome (e.g. diarrhea-predominant irritable bowel syndrome (IBS-d), constipation-predominant irritable bowel syndrome (IBS-c) and/or alternating or mixed irritable bowel syndrome (IBS-m)), post-operative ileus, ulcerative colitis, chronic constipation, constipation, interstitial cystitis, prostatitis, testicular pain, painful bladder syndrome, endometriosis, vulvodynia, rectal pain, diverticular disease or pain, pain associated with GI disorders, and disorders and conditions associated with constipation (e.g. constipation associated with use of opiate pain killers, post-surgical constipation, and constipation associated with neuropathic disorders as well as other conditions and disorders described herein). The patient can be diagnosed with a functional gastrointestinal disorder (e.g. IBS-c) according to the Rome Criteria (e.g. Rome II).

In some embodiments, the DR composition comprises enteric-coated tablets comprising an immediate release tablet core and containing a unit dose of linaclotide that dissolves only under pH conditions of the distal segment of intestine. In some embodiments, the enteric or functional coating is selected from methyl acrylate-methacrylic acid copolymers (e.g. Eudragit®); cellulose acetate succinate (CAS); hydroxy propyl methyl cellulose phthalate (HPMCP); PVA; PVP; PVP-LP, hydroxy propyl methyl cellulose acetate succinate (HPMCAS); polyvinyl acetate phthalate (PVAP); methyl methacrylate-methacrylic acid copolymers; sodium alginate and stearic acid; guar gum; and carbomers. In further embodiments, the enteric coating is selected from Eudragit® FS30D, PlasAcryl®, Eudragit® S100, Eudragit®L100, Eudragit®L100-55, Eudragit® L30D-55, Eudragit® S, Eudragit®RL30D, Eudragit®RS30D, Eudragit® RS, Eudragit® EC, or mixture thereof.

The delayed release compositions may include any effective amount of linaclotide. In some embodiments, for example, the composition comprises from 0.05 µg to 6 mg of linaclotide. In some embodiments, for example, the composition comprises from 1 µg to 2 mg of linaclotide. In some embodiments, the composition comprises from 25 µg to 2 mg of linaclotide, for example, from 50 µg to 1 mg of linaclotide. In some embodiments, for example, the composition comprises from 0.1 µg to 90 µg of linaclotide. In some embodiments, for example, the composition comprises from 0.1 µg to 45 µg of linaclotide. In some embodiments, for example, the composition comprises from 0.1 µg to 25 µg of linaclotide. In some embodiments, for example, the composition comprises from 36 µg to 290 µg of linaclotide. In some embodiments, the composition comprises 0.05 µg, 0.1 µg, 0.15 µg 0.25 µg, 0.5 µg, 0.75 µg, 1 µg, 1.5 µg 2 µg 2.5 µg 3 µg, 3.5 µg 4 µg 4.5 µg, 5 µg, 7.5 µg, 9 µg, 10 µg, 15 µg 20 µg 25 µg, 30 µg, 35 µg, 36 µg, 40 µg 45 µg 50 µg, 60 µg 72 µg 75 µg, 90 µg, 100 µg 145 µg, 150 µg, 200 µg 250 µg, 290 µg 300 µg, 350 µg, 400 µg, 450 µg, 500 µg 550 µg, 579 µg 600 µg, 650 µg, 700 µg, 750 µg 800 µg, 850 µg, 900 µg 950 µg or 1 mg of linaclotide. In some embodiments, the composition comprises from 100 µg to 600 µg of linaclotide. In some embodiments, the composition comprises 30 µg, 50 µg, 75 µg, 100 µg, 150 µg, 290 µg, 300 µg 400 µg 500 µg or 600 µg of linaclotide. In some embodiments, the composition comprises 9 µg, 10 µg, 15 µg, 36 µg, 72 µg, 75 µg, 145 µg, 290 µg, 579 µg, or 600 µg of linaclotide.

In some embodiments, the composition comprises 30 µg of linaclotide. In some embodiments, the composition comprises 50 µg of linaclotide. In some embodiments, the composition comprises 100 µg of linaclotide. In some embodiments, the composition comprises 145 µg of linaclotide. In some embodiments, the composition comprises 300 µg of linaclotide. In the present invention, the amount of linaclotide in the composition is between 30 µg and 300 µg.

It has been found, in some embodiments, that the stability of delayed release compositions of linaclotide can be increased or improved by including in the compositions a suitable amount of a sterically hindered primary amine (e.g., amino acid) component, a cation (e.g., metal cation) component, and/or a polymer component. These components increase or enhance the stability of delayed release compositions of linaclotide, for example, by preventing, lessening, and/or decreasing degradation of linaclotide within the composition (for example, due to moisture-driven degradation reactions, e.g., hydrolysis, deamidation, and/or multimerization reactions). For instance, it has been found in some embodiments that addition or inclusion of a suitable amount of a cation *(e.g.,* Mg²⁺, Ca²⁺, Zn²⁺) in the composition increases the stability of the composition against oxidative degradation of linaclotide. Moreover, it has been found in some embodiments that inclusion of a suitable amount of a sterically hindered primary amine for an example in the form of an amino acid (e.g., histidine) in the composition increases the stability of the composition against, for example, the nucleophilic addition of formaldehyde or a formaldehyde equivalent to the N-terminus of linaclotide, e.g. by acting as a scavenger, and/or by buffering the composition. Moreover, it has been found in some embodiments that inclusion of both a sterically hindered primary amine *(e.g.,* histidine) and a cation *(e.g.,* Ca²⁺) in suitable amounts in the composition increases the stability of the composition against the formation of hydrolysis and formaldehyde (Cys¹-IMD) products of linaclotide. It has also been found in some embodiments that inclusion of a suitable amount of a polymer (e.g., polyvinyl pyrrolidone or polyvinyl alcohol) in the delayed release composition increases the stability of the composition for example by decreasing the mobility and/or reactivity of linaclotide within the composition, e.g., by forming a complex or matrix (for example, a glassy and/or rigid matrix) with linaclotide (e.g., by vitrification reaction), by preventing or lessening hydrogen bond formation between linaclotide and water molecules, and/or by enhancing the three-dimensional structural integrity of linaclotide.

In this regard, it has been found in some embodiments that combining linaclotide in an delayed release pharmaceutical composition with specific concentrations or molar ratios of the cation and sterically hindered primary amine causes a synergistic enhancement or improvement in the stability of linaclotide within the composition, for example as compared to similar compositions not containing the cation and/or sterically hindered primary amine and/or the same concentrations of these components. In some embodiments, composition can comprise any stabilizing amount of a sterically hindered primary amine component. In other embodiments, the composition comprises a molar ratio of sterically hindered primary amine to linaclotide between 400:1 and 1:1. In further, embodiments, the composition comprises a molar ratio of sterically hindered primary amine to linaclotide between 200:1 and 50:1. In other embodiments, the composition can comprise a molar ratio of sterically hindered primary amine *(e.g.,* amino acid) to linaclotide between 100:1 and 1:100. In some embodiments, the composition comprises a molar ratio of sterically hindered primary amine to linaclotide between 100:1 and 1:1. In some embodiments, the composition comprises a molar ratio of sterically hindered primary amine to linaclotide between 90:1 and 2:1. In some embodiments, the composition comprises a molar ratio of sterically hindered primary amine to linaclotide between 80:1 and 5:1. In some embodiments, the composition comprises a molar ratio of sterically hindered primary amine to linaclotide between 70:1 and 10:1. In some embodiments, the composition comprises a molar ratio of sterically hindered primary amine to linaclotide between 60:1 and 20:1. In some embodiments, the composition comprises a molar ratio of sterically hindered primary amine to linaclotide between 50:1 and 30:1. In some embodiments, the composition comprises a molar ratio of sterically hindered primary amine to linaclotide between 40:1 and 20:1. In some embodiments, the composition comprises a molar ratio of sterically hindered primary amine to linaclotide between 100:1 and 20:1. In some embodiments, the composition comprises a molar ratio of sterically hindered primary amine to linaclotide between 100:1 and 25:1. In some embodiments, the composition comprises a molar ratio of sterically hindered primary amine to linaclotide between 100:1 and 30:1. In some embodiments, the composition comprises a molar ratio of sterically hindered primary amine to linaclotide between 100:1 and 40:1. In some embodiments, the composition comprises a molar ratio of sterically hindered primary amine to linaclotide between 100:1 and 50:1. In some embodiments, the composition comprises a molar ratio of sterically hindered primary amine to linaclotide between 100:1 and 60:1. In some embodiments, the composition comprises a molar ratio of sterically hindered primary amine to linaclotide between 100:1 and 70:1. In some embodiments, the composition comprises a molar ratio of sterically hindered primary amine to linaclotide of at least 5:1. In some embodiments, the composition comprises a molar ratio of sterically hindered primary amine to linaclotide of at least 10:1. In some embodiments, the composition comprises a molar ratio of sterically hindered primary amine to linaclotide of at least 20:1. In some embodiments, the composition comprises a molar ratio of sterically hindered primary amine to linaclotide of at least 25:1. In some embodiments, the composition comprises a molar ratio of sterically hindered primary amine to linaclotide of at least 30:1. In some embodiments, the composition comprises a molar ratio of sterically hindered primary amine to linaclotide of at least 40:1.

Suitable sterically hindered primary amines for inclusion in the delayed release composition are, for example, naturally-occurring amino acids (e.g., alanine, arginine, asparagine, aspartic acid, cysteine, glutamic acid, glutamine, glycine, histidine, isoleucine, leucine, lysine, meglumine, methionine, phenylalanine, proline, serine, threonine, tryptophan, tyrosine, valine), synthetic amino acids (e.g., lanthionine, theanine or 1-amino cyclohexane), amino sugars (e.g., chitosan or glucosamine), or combination or mixtures thereof. In some embodiments, the composition comprises an amino acid selected from alanine, arginine, asparagine, aspartic acid, cysteine, glutamic acid, glutamine, histidine, isoleucine, leucine, lysine, methionine, phenylalanine, proline, serine, threonine, tryptophan, tyrosine, valine, or a mixture thereof. In some embodiments, the composition comprises an amino acid selected from leucine, isoleucine, asparagine, glutamine, glutamic acid, histidine, cysteine, alanine, serine, threonine, tyrosine, proline, tryptophan, or a combination or mixture thereof. In some embodiments, the composition comprises an amino acid selected from leucine, isoleucine, methionine, alanine, or a combination or mixture thereof. In some embodiments, the composition comprises an amino acid selected from leucine, isoleucine, alanine, or a combination or mixture thereof. In some embodiments, the composition comprises an amino acid selected from leucine, isoleucine, methionine, or a combination or mixture thereof. In some embodiments, the composition comprises an amino acid selected from leucine, methionine, alanine, or a combination or mixture thereof. In some embodiments, the composition comprises leucine, methionine, or a mixture thereof. In some embodiments, the composition comprises leucine, isoleucine, or a mixture thereof. In some embodiments, the composition comprises leucine, alanine, or a mixture thereof. In some embodiments, the composition comprises leucine. In some embodiments, the composition comprises isoleucine. In some embodiments, the composition comprises methionine. In some embodiments, the composition comprises alanine. In the present invention, the composition comprises histidine.

The delayed release composition can comprise any stabilizing amount of a cation (e.g., metal cation). In some embodiments, the composition comprises a molar ratio of cation to linaclotide between 300:1 and 1:1. In further embodiments, the composition comprises a molar ratio of cation to linaclotide between 250:1 and 30:1. In other embodiments, the composition can comprise a molar ratio of cation to linaclotide between 100:1 and 1:100. In some embodiments, the composition comprises a molar ratio of cation to linaclotide between 100:1 and 1:1. In some embodiments, the composition comprises a molar ratio of cation to linaclotide between 90:1 and 2:1. In some embodiments, the composition comprises a molar ratio of cation to linaclotide between 80:1 and 5:1. In some embodiments, the composition comprises a molar ratio of cation to linaclotide between 70:1 and 10:1. In some embodiments, the composition comprises a molar ratio of cation to linaclotide between 60:1 and 20:1. In some embodiments, the composition comprises a molar ratio of cation to linaclotide between 50:1 and 30:1. In some embodiments, the composition comprises a molar ratio of cation to linaclotide between 40:1 and 20:1. In some embodiments, the composition comprises a molar ratio of cation to linaclotide between 100:1 and 20:1. In some embodiments, the composition comprises a molar ratio of cation to linaclotide between 100:1 and 25:1. In some embodiments, the composition comprises a molar ratio of cation to linaclotide between 100:1 and 30:1. In some embodiments, the composition comprises a molar ratio of cation to linaclotide between 100:1 and 40:1. In some embodiments, the composition comprises a molar ratio of cation to linaclotide between 100:1 and 50:1. In some embodiments, the composition comprises a molar ratio of cation to linaclotide between 100:1 and 60:1. In some embodiments, the composition comprises a molar ratio of cation to linaclotide between 100:1 and 70:1. In some embodiments, the composition comprises a molar ratio of cation to linaclotide of at least 5:1. In some embodiments, the composition comprises a molar ratio of cation to linaclotide of at least 10:1. In some embodiments, the composition comprises a molar ratio of cation to linaclotide of at least 20:1. In some embodiments, the composition comprises a molar ratio of cation to linaclotide of at least 25:1. In some embodiments, the composition comprises a molar ratio of cation to linaclotide of at least 30:1. In some embodiments, the composition comprises a molar ratio of cation to linaclotide of at least 40:1. In some embodiments, the composition comprises a molar ratio of cation to linaclotide of at least 60:1.

Any suitable cation(s) can be included in the composition, for example, any suitable metal cation or organic cation. In some embodiments, the composition comprises a metal cation selected from calcium, potassium, magnesium, zinc, aluminum, iron, tin, manganese, chromium, cobalt, nickel, barium, sodium, or a combination or mixture thereof. In some embodiments, the composition comprises a metal cation selected from calcium, potassium, magnesium, zinc, aluminum, manganese, chromium, cobalt, nickel, barium, sodium, or a combination or mixture thereof. In some embodiments, the composition comprises a metal cation selected from aluminum, calcium, potassium, sodium, magnesium, manganese, zinc, or a combination or mixture thereof. In some embodiments, the composition comprises a metal cation selected from calcium, magnesium, manganese, zinc, or a combination or mixture thereof. In some embodiments, the composition comprises a divalent metal cation. In some embodiments, the composition comprises a divalent metal cation selected from Al^{3+,} Ca²⁺, Mg²⁺, Zn²⁺, Mn²⁺, or a combination or mixture thereof. In some embodiments, the composition comprises Mg²⁺. In some embodiments, the composition comprises Ca²⁺. In some embodiments, the composition comprises Zn²⁺. In some embodiments, the composition comprises aluminum. In the present invention, the composition comprises Ca²⁺.

Moreover, the metal cation can be added to the composition in any suitable form, for example any pharmaceutically acceptable salt with any appropriate counterion. Suitable metal salts include, for example, calcium chloride, calcium carbonate, calcium acetate, magnesium chloride, magnesium acetate, zinc acetate, zinc chloride, or mixtures thereof. In some embodiments, the composition comprises calcium chloride, magnesium chloride, zinc acetate, or any combination or mixture thereof. In some embodiments, the composition comprises calcium chloride. In some embodiments, the composition comprises magnesium chloride. In some embodiments, the composition comprises zinc acetate. Suitable organic cations include, for example, ammonium hydroxide, D-arginine, L-arginine, t-butylamine, calcium acetate hydrate, calcium carbonate, calcium DL-malate, calcium hydroxide, choline, ethanolamine, ethylenediamine, glycine, L-histidine, L-lysine, magnesium hydroxide, N-methyl-D-glucamine, L-ornithine hydrochloride, potassium hydroxide, procaine hydrochloride, L-proline, pyridoxine, L-serine, sodium hydroxide, DL-tryptophan, tromethamine, L-tyrosine, L-valine, carnitine, taurine, creatine malate, arginine alpha ketoglutarate, ornithine alpha ketoglutarate, spermine acetate, spermidine chloride, or combinations or mixtures thereof. In some embodiments, the organic cation is selected from the group consisting of N-methyl D-glucamine, choline, arginine, lysine, procaine, tromethamine (TRIS), spermine, N-methyl-morpholine, glucosamine, N,N-bis(2-hydroxyethyl) glycine, diazabicycloundecene, creatine, arginine ethyl ester, amantadine, rimantadine, ornithine, taurine, citrulline, or a combination or mixture thereof.

The composition can contain any stabilizing amount of a polymer. In some embodiments, the composition comprises between 1 and 25 % by weight of a polymer, relative to the total weight of the composition. In some embodiments, the composition comprises between 1 and 10% by weight of a polymer, relative to the total weight of the composition.

In some embodiments, the composition comprises between 2 and 4 % by weight of a polymer, relative to the total weight of the composition. In some embodiments, the composition comprises between 0.1 and 75 wt.% of a polymer. In some embodiments, the composition comprises between 0.1 and 55 wt.% of a polymer. In some embodiments, the composition comprises between 0.1 and 35 wt.% of a polymer. In some embodiments, the composition comprises between 0.1 and 30 wt.% of a polymer. In some embodiments, the composition comprises between 0.1 and 25 wt.% of a polymer. In some embodiments, the composition comprises between 1 and 25 wt.% of a polymer. In some embodiments, the composition comprises between 5 and 25 wt.% of a polymer. In some embodiments, the composition comprises between 10 and 25 wt.% of a polymer. In some embodiments, the composition comprises between 15 and 25 wt.% of a polymer. In some embodiments, the composition comprises between 0.1 and 22 wt.% of a polymer. In some embodiments, the composition comprises between 1 and 22 wt.% of a polymer. In some embodiments, the composition comprises between 5 and 22 wt.% of a polymer. In some embodiments, the composition comprises between 10 and 22 wt.% of a polymer. In some embodiments, the composition comprises between 0.1 and 20 wt.% of a polymer. In some embodiments, the composition comprises between 1 and 20 wt.% of a polymer. In some embodiments, the composition comprises between 5 and 20 wt.% of a polymer. In some embodiments, the composition comprises between 10 and 20 wt.% of a polymer. In some embodiments, the composition comprises between 0.01 and 15 wt.% of a polymer. In some embodiments, the composition comprises between 0.01 and 10 wt.% of a polymer. In some embodiments, the composition comprises between 0.01 and 5 wt.% of a polymer. In some embodiments, the composition comprises between 0.1 and 95 wt.%, for example, between 5 and 95 wt.%, between 15 and 95 wt.%, between 25 and 95 wt.%, between 35 and 95 wt.%, between 45 and 95 wt.%, between 0.1 and 85 wt.%, between 1 and 85 wt.%, between 5 and 85 wt.%, between 15 and 85 wt.%, between 25 and 85 wt.%, between 35 and 85 wt.%, between 0.1 and 80 wt.%, between 1 and 80 wt.%, between 5 and 80 wt.%, between 15 and 80 wt.%, between 25 and 80 wt.%, between 35 and 80 wt.%, between 0.1 and 75 wt.%, between 1 and 75 wt.%, between 5 and 75 wt.%, between 15 and 75 wt.%, between 25 and 75 wt.%, between 35 and 75 wt.%, between 0.1 and 65 wt.%, between 1 and 65 wt.%, between 5 and 65 wt.%, between 15 and 65 wt.%, between 25 and 65 wt.%, between 35 and 65 wt.%, between 0.1 and 60 wt.%, between 1 and 60 wt.%, between 5 and 60 wt.%, between 15 and 60 wt.%, between 25 and 60 wt.%, or between 35 and 60 wt.% of a polymer.

In some embodiments, the polymer acts as both a stabilizer, protective coating, or as a film forming agent within the delayed release composition. In some embodiments, the delayed release composition comprises a molar ratio of polymer (e.g., PVP or PVA) to linaclotide between 80:1 and 300:1, for example, between 100:200:1, between 110:1 and 190:1, or even between 120:1 and 180:1. In some embodiments, the delayed release composition comprises a molar ratio of polymer (e.g., PVP or PVA) to linaclotide greater than about 80:1, for example, greater than about 100:1, or even greater than about 120:1. In some embodiments, the delayed release composition comprises a weight ration of polymer *(e.g.,* PVP or PVA) to linaclotide between 10:1 and 300:1, for example, between 80:1 and 200:1, between 100:1 and 180:1, or even between 110:1 and 150: 1. In some embodiments, the delayed release composition comprises a weight ration of polymer (e.g., PVP or PVA) to linaclotide between 100:1 and 500:1, for example, between 200:1 and 400:1, between 250:1 and 350:1, or even between 300:1 and 350:1.

Suitable polymers for inclusion in the delayed release compositions are, for example, polyvinyl pyrrolidone (PVP), polyvinyl alcohol (PVA), polyvinyl alcohol low peroxide (PVA-LP), hydroxylpropyl methyl cellulose (HPMC), hydroxylpropyl cellulose (HPC), methyl cellulose, methacrylate polymers, cyclodextrin, dextrin, dextran, polyacrylic acid, chitosan, guar gum, xanthan gum, polyethylene oxide (e.g., polyethylene polypropylene oxide), poly (sodium vinylsulfonate), polyethylene glycol, poly(arginine), poly carbophil, polyvinyl pyrrolidone-co-vinyl acetate, a poloxamer *(e.g.,* Pluronic® products available from BASF), alginate, trehalose, sucrose, inulin, or a combination or mixture thereof. In some embodiments, the composition comprises a polymer selected from PVP, PVA, methacrylate polymers, cyclodextrin, dextran, polyacrylic acid, chitosan, guar gum, xanthan gum, polyethylene oxide, polyethylene glycol, poly(arginine), poly carbophil, polyvinyl pyrrolidone-co-vinyl acetate, a poloxamer, or a combination or mixture thereof. In some embodiments, the composition comprises PVP, PVA, polyethylene oxide, or a mixture thereof. In some embodiments, the composition comprises PVP, PVA, or a mixture thereof. In some embodiments, the composition comprises PVP. In the present invention, the composition comprises PVA.

In some embodiments, the delayed release composition comprises two or more stabilizing agents. For example, the composition can include a stabilizing amount of a polymer and a stabilizing amount of a sterically hindered primary amine. Moreover, the composition can include a stabilizing amount of a polymer and a stabilizing amount of a cation (e.g., metal cation). In addition, the composition can include a stabilizing amount of a sterically hindered primary amine and a stabilizing amount of a cation (e.g., metal cation). In some embodiments, the composition comprises a stabilizing amount of a polymer, a stabilizing amount of a sterically hindered primary amine, and a stabilizing amount of a cation (e.g., metal cation).

In some embodiments, the delayed release composition comprises a stabilizing amount of PVP and a stabilizing amount of an amino acid selected from histidine, alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, or a mixture thereof. In some embodiments, the composition comprises a stabilizing amount of PVP and a stabilizing amount of an amino acid selected from alanine, arginine, asparagine, aspartic acid, cysteine, glutamic acid, glutamine, histidine, isoleucine, leucine, lysine, methionine, phenylalanine, proline, serine, threonine, tryptophan, tyrosine, valine, or a mixture thereof. In some embodiments, the composition comprises a stabilizing amount of PVP and a stabilizing amount of leucine, isoleucine, methionine, alanine, or a combination or mixture thereof. In some embodiments, the composition comprises a stabilizing amount of PVP and a stabilizing amount of histidine.

In some embodiments, the delayed release composition comprises a stabilizing amount of PVA and a stabilizing amount of an amino acid selected from alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, or a mixture thereof. In some embodiments, the composition comprises a stabilizing amount of PVA and a stabilizing amount of an amino acid selected from alanine, arginine, asparagine, aspartic acid, cysteine, glutamic acid, glutamine, histidine, isoleucine, leucine, lysine, methionine, phenylalanine, proline, serine, threonine, tryptophan, tyrosine, valine, or a mixture thereof. In some embodiments, the composition comprises a stabilizing amount of PVA and a stabilizing amount of leucine, isoleucine, methionine, alanine, or a combination or mixture thereof. In some embodiments, the composition comprises a stabilizing amount of PVA and a stabilizing amount of leucine. In the present invention, the composition comprises PVA and histidine.

In some embodiments, the delayed release composition comprises a stabilizing amount of PVP and a stabilizing amount of a cation (e.g., metal cation). In some embodiments, the composition comprises a stabilizing amount of PVP and a stabilizing amount of a divalent metal cation. In some embodiments, the composition comprises a stabilizing amount of PVP and a stabilizing amount of Mg²⁺, Ca²⁺, Zn²⁺ or a salt thereof or a combination or mixture thereof. In some embodiments, the composition comprises a stabilizing amount of PVP and a stabilizing amount of Ca²⁺ or a salt thereof. In some embodiments, the composition comprises a stabilizing amount of PVP and a stabilizing and a stabilizing amount of Zn²⁺ or a salt thereof.

In some embodiments, the delayed release composition comprises a stabilizing amount of PVA and a stabilizing amount of a cation (e.g., metal cation). In some embodiments, the composition comprises a stabilizing amount of PVA and a stabilizing amount of a divalent metal cation. In some embodiments, the composition comprises a stabilizing amount of PVA and a stabilizing amount of Mg²⁺, Ca²⁺, Zn²⁺ or a salt thereof or a combination or mixture thereof. In some embodiments, the composition comprises a stabilizing amount of PVA and a stabilizing amount of Ca²⁺ or a salt thereof. In some embodiments, the composition comprises a stabilizing amount of PVA and a stabilizing amount of Mg²⁺ or a salt thereof. In some embodiments, the composition comprises a stabilizing amount of PVA and a stabilizing amount of Zn²⁺ or a salt thereof. In the present invention, the composition comprises PVA and Ca²⁺ or a salt thereof.

In some embodiments, the delayed release composition comprises a stabilizing amount of an amino acid selected from leucine, isoleucine, methionine, alanine; and a stabilizing amount of a divalent metal cation selected from Mg²⁺, Ca²⁺, Zn²⁺ or a salt thereof or a combination or mixture thereof. In some embodiments, the composition comprises a stabilizing amount of an amino acid selected from leucine, and isoleucine; and a stabilizing amount of a divalent metal cation selected from Mg²⁺, Ca²⁺ or a salt thereof or a combination or mixture thereof. In some embodiments, the composition comprises a stabilizing amount of an amino acid selected from leucine or methionine; and a stabilizing amount of a divalent metal cation selected from Ca²⁺, Zn²⁺ or a salt thereof or a combination or mixture thereof. In some embodiments, the composition comprises a stabilizing amount of leucine and a stabilizing amount of Ca²⁺ or a salt thereof. In some embodiments, the composition comprises a stabilizing amount of a cation and a stabilizing amount of a sterically hindered primary amine. In the present invention, the composition comprises histidine and Ca²⁺ or a salt thereof. In some embodiments, the composition comprises a cation and a sterically hindered primary amine in a molar ratio of cation:sterically hindered primary amine (e.g., Ca²⁺:leucine) of at least 1.5:1, *e.g.,* at least 2:1, at least 2.5:1, at least 3:1, at least 4:1, or even at least 5:1 (for example, a molar ratio between 1.5:1 and 5:1, e.g., between 2:1 and 4:1).

In some embodiments, the delayed release composition comprises (i) a stabilizing amount of PVP or PVA, (ii) a stabilizing amount of leucine, isoleucine, methionine, alanine, and (iii) a stabilizing amount of Mg²⁺, Ca²⁺, Zn²⁺ or a salt thereof or a combination or mixture thereof. In some embodiments, the composition comprises a stabilizing amount of PVP, a stabilizing amount of leucine, and a stabilizing amount of a metal cation. In some embodiments, the composition comprises a stabilizing amount of PVA, a stabilizing amount of histidine, and a stabilizing amount of Ca²⁺ or a salt thereof. In some embodiments, the composition comprises a stabilizing amount of PVP, a stabilizing amount of leucine, and a stabilizing amount of Mg²⁺ or a salt thereof. In some embodiments, the composition comprises a stabilizing amount of PVP, a stabilizing amount of leucine, and a stabilizing amount of Zn²⁺ or a salt thereof. In some embodiments, the composition comprises a stabilizing amount of PVA, a stabilizing amount of leucine, and a stabilizing amount of Ca²⁺ or a salt thereof. In some embodiments, the composition comprises a stabilizing amount of PVA, a stabilizing amount of leucine, and a stabilizing amount of Mg²⁺ or a salt thereof. In some embodiments, the composition comprises a stabilizing amount of PVA, a stabilizing amount of leucine, and a stabilizing amount of Zn²⁺ or a salt thereof. In the present invention, the composition comprises PVA, histidine, and Ca²⁺ or a salt thereof.

In some embodiments, the composition comprises (i) between 0.1 and 30 wt.% of a polymer, (ii) a sterically hindered primary amine *(e.g.,* an amino acid) in a molar ratio of primary amine to linaclotide between 100:1 and 10:1, and (iii) a cation *(e.g.,* a metal cation) in a molar ratio of cation to linaclotide between 100:1 and 40:1. In some embodiments, the composition comprises (i) between 5 and 25 wt.% of a polymer, (ii) a sterically hindered primary amine *(e.g.,* an amino acid) in a molar ratio of primary amine to linaclotide 100:1 and 30:1 *(e.g.,* between 60:1 and 30:1 or even between 50:1 and 30:1), and (iii) a cation *(e.g.,* a metal cation) in a molar ratio of cation to linaclotide between 100:1 and 60:1. In some embodiments, the composition comprises (i) between 0.1 and 30 wt.% of a polymer selected from PVP and PVA, (ii) an amino acid selected from leucine, isoleucine, alanine, and methionine in a molar ratio of amino acid to linaclotide between 100:1 and 10:1, and (iii) a metal cation selected from Ca²⁺, Mg²⁺, and Zn²⁺ in a molar ratio of cation to linaclotide between 100:1 and 40:1. In some embodiments, the composition comprises (i) between 5 and 25 wt.% of a polymer selected from PVP and PVA, (ii) an amino acid selected from leucine, isoleucine, alanine, and methionine in a molar ratio of amino acid to linaclotide 100:1 and 30:1 (e.g., between 60:1 and 30:1), and (iii) a metal cation selected from Ca²⁺, Mg²⁺, and Zn²⁺ in a molar ratio of cation to linaclotide between 100:1 and 60:1. In some embodiments, the composition comprises (i) between 0.1 and 30 wt.% (e.g., between 5 and 25 wt.%) of PVP or PVA, (ii) leucine in a molar ratio of leucine to linaclotide between 100:1 and 30:1 *(e.g.,* between 60:1 and 30:1 or even between 50:1 and 30:1), and (iii) Ca²⁺ in a molar ratio of Ca²⁺ to linaclotide between 100:1 and 60:1.

In some embodiments, the composition comprises (i) between 45 and 99 wt.% of a polymer, (ii) a sterically hindered primary amine (e.g., an amino acid) in a molar ratio of primary amine to linaclotide between 100:1 and 10:1, and (iii) a cation (e.g., a metal cation) in a molar ratio of cation to linaclotide between 100:1 and 40:1. In some embodiments, the composition comprises (i) between 45 and 70 wt.% of a polymer, (ii) a sterically hindered primary amine (*e.g*., an amino acid) in a molar ratio of primary amine to linaclotide 100:1 and 30:1 (*e.g.,* between 60:1 and 30:1 or even between 50:1 and 30:1), and (iii) a cation (*e.g.,* a metal cation) in a molar ratio of cation to linaclotide between 100:1 and 60:1. In some embodiments, the composition comprises (i) between 45 and 99 wt.% of a polymer selected from PVP and PVA, (ii) an amino acid selected from leucine, isoleucine, alanine, and methionine in a molar ratio of amino acid to linaclotide between 100:1 and 10:1, and (iii) a metal cation selected from Ca²⁺, Mg²⁺, and Zn²⁺ in a molar ratio of cation to linaclotide between 100:1 and 40:1. In some embodiments, the composition comprises (i) between 45 and 70 wt.% of a polymer selected from PVP and PVA, (ii) an amino acid selected from leucine, isoleucine, alanine, and methionine in a molar ratio of amino acid to linaclotide 100:1 and 30:1 (*e.g*., between 60:1 and 30:1), and (iii) a metal cation selected from Ca²⁺, Mg²⁺, and Zn²⁺ in a molar ratio of cation to linaclotide between 100:1 and 60:1. In some embodiments, the composition comprises (i) between 45 and 99 wt.% (*e.g*., between 45 and 70 wt.%) of PVP or PVA, (ii) leucine in a molar ratio of leucine to linaclotide between 100:1 and 30:1 (*e.g*., between 60:1 and 30:1 or even between 50:1 and 30:1), and (iii) Ca²⁺ in a molar ratio of Ca²⁺ to linaclotide between 100:1 and 60:1.

The delayed release composition (*e.g*., delayed release tablet) may also comprise any one or more filling agents. Suitable filling agents include, but are not limited to, starch, calcium carbonate, calcium sulfate, hydroxylpropylmethyl cellulose, fructose, methyl cellulose, dextrates, dextrose, dextran, lactitol, maltose, sucrose, sorbitol, isomalt, pregelatinized starch, dicalcium phosphate, microcrystalline cellulose, mannitol, gelatin, trehalose, erythritol, maltitol, lactose, glucose, or a combination thereof, or a mixture thereof. In some embodiments, the filling agent is isomalt. In some embodiments, the filling agent is gelatin. In some embodiments, the filling agent is mannitol. In some embodiments, the filling agent is pregelatinized starch. In some embodiments, the filling agent is microcrystalline cellulose.

The delayed release composition (*e.g*., delayed release tablet) can comprise any suitable concentration of filling agent. In some embodiments, for example, the composition comprises one or more filling agents in a concentration of 0.1-99 % by weight, relative to the total weight of the composition. In some embodiments, for example, the composition comprises one or more filling agents in a concentration of 1-95 wt.% of filling agent(s), relative to the total weight of the composition. In some embodiments, for example, the composition comprises one or more filling agents in a concentration of 10-90 wt.% of filling agent(s), relative to the total weight of the composition. In some embodiments, for example, the composition comprises one or more filling agents in a concentration of 20-90 wt.% of filling agent(s), relative to the total weight of the composition. In some embodiments, for example, the composition comprises one or more filling agents in a concentration of 25-85 wt.% of filling agent(s), relative to the total weight of the composition. In some embodiments, for example, the composition comprises one or more filling agents in a concentration of 30-80 wt.% of filling agent(s), relative to the total weight of the composition. In some embodiments, for example, the composition comprises one or more filling agents in a concentration of 40-70 wt.% of filling agent(s), relative to the total weight of the composition. In some embodiments, for example, the composition comprises one or more filling agents in a concentration of 10-60 wt.% of filling agent(s), relative to the total weight of the composition. In some embodiments, for example, the composition comprises one or more filling agents in a concentration of 20-50 wt.% of filling agent(s), relative to the total weight of the composition. In some embodiments, the composition comprises one or more filling agents in a concentration of at least 20 wt.%, for example, at least 40 wt.%, at least 60 wt.%, at least 70 wt.%, at least 80 wt.%, or at least 90 wt.%, relative to the total weight of the composition.

In some embodiments, the delayed release composition (e.g., delayed release film) comprises one or more plasticizers. Suitable plasticizers include, but are not limited to, polyethylene glycol, propylene glycol, glycerin, glycerol, monoacetin, diacetin, triacetin, dimethyl phthalate, diethyl phthalate, dibutyl phthalate, dibutyl sebacate, triethyl titrate, tributyl citrate, triethyl citrate, triethyl acetyl citrate, castor oil, acetylated monoglycerides, sorbitol or combinations thereof. In exemplary embodiments, the concentration of the plasticizer in the formulation may be about 0 to about 30 wt.%, for example, about 1 to about 20 wt.%, about 0 to about 10 wt.%, about 1 to about 5 wt.%, or even 0 to about 4 wt.%.

In some embodiments, the delayed release composition comprises a film forming agent, a water-soluble polymer, a pH sensitive polymer, biodegradable polymer, or combination thereof. Water soluble, pH sensitive, or biodegradable polymers that may be used in the orally dissolving formulations of the present invention include, but are not limited to, cellulose derivatives, synthetic polymers polyacrylates and natural gums. For example, the water soluble polymers used in the orally dissolving formulations of the present invention may include, but are not limited to, methyl cellulose, hydroxypropyl cellulose, hydroxypropylmethyl cellulose, ethyl cellulose, hydroxyethyl cellulose, hydroxypropyl cellulose, carboxymethyl cellulose, cellulose acetate phthalate, cellulose acetate butyrate, amylose, dextran, casein, pullulan, gelatin, pectin, agar, carrageenan, xanthan gum, tragacanth, guar gum, acacia gum, arabic gum, polyethylene glycol, polyethylene oxide, polyvinyl pyrrolidone, polyvinyl alcohol, cyclodextrin, carboxyvinyl polymers, sodium alginate, polyacrylic acid, methylmethacrylate or mixtures thereof. In exemplary embodiments, the concentration of the water-soluble polymer in the formulation may be about 20% to about 90% (by weight), preferably between about 40% to about 80% (by weight).

In some embodiments, the pH sensitive polymer is Eudragit® L100 that has a threshold pH of 6.0. In some embodiments, the pH sensitive polymer is Eudragit® S100 that has a threshold pH of 7.0. In some embodiments, the pH sensitive polymer is Eudragit® L-30D that has a threshold pH of 5.6. In some embodiments, the pH sensitive polymer is Eudragit® FS 30D that has a threshold pH of 6.8. In some embodiments, the pH sensitive polymer is Eudragit® L100-55 that has a threshold pH of 5.5. In some embodiments, the pH sensitive polymer is Polyvinyl acetate phthalate that has a threshold pH of 5.0. In some embodiments, the pH sensitive polymer is Hydroxypropylmethylcellulose phthalate that has a threshold pH of 4.5-4.8. In some embodiments, the pH sensitive polymer is Hydroxypropylmethylcellulose phthalate 50 that has a threshold pH of 5.2. In some embodiments, the pH sensitive polymer is Hydroxypropylmethylcellulose phthalate 55 that has a threshold pH of 5.4. In some embodiments, the pH sensitive polymer is Cellulose acetate trimelliate that has a threshold pH of 4.8. In some embodiments, the pH sensitive polymer is Cellulose acetate phthalate that has a threshold pH of 5.0. In some embodiments the delayed release composition comprises a combination of the pH sensitive polymers mentioned above.

One skilled in the art, with the benefit of this disclosure, will understand that other components may be included to enhance one or more properties of the delayed release composition. In some embodiments, for example, the delayed release compositions may include one or more disintegrants, lubricants, anti-caking additives, anti-microbial agents, antifoaming agents, emulsifiers, surfactants, buffering agents, and/or coloring agents.

Suitable disintegrants include, for example, agar-agar, calcium carbonate, microcrystalline cellulose, croscarmellose sodium, crospovidone, povidone, polacrilin potassium, sodium starch glycolate, potato or tapioca starch, other starches, pre-gelatinized starch, clays, other algins, other celluloses, gums, and mixtures thereof. In some embodiments, the disintegrant is crospovidone. In some embodiments, the disintegrant is croscarmellose sodium.

Suitable lubricants include, for example, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil and soybean oil), zinc stearate, ethyl oleate, ethyl laurate, agar, syloid silica gel (AEROSIL® 200, W.R. Grace Co., Baltimore, MD USA), a coagulated aerosol of synthetic silica (Evonik Degussa Co., Piano, TX USA), a pyrogenic silicon dioxide (CAB-O-SIL, Cabot Co., Boston, MA USA), and mixtures thereof.

Suitable anti-caking additives include, for example, calcium silicate, magnesium silicate, silicon dioxide, colloidal silicon dioxide, talc, glyceryl, and mixtures thereof.

Suitable anti-microbial additives that may be used, e.g., as a preservative for the linaclotide compositions, include, for example, benzalkonium chloride, benzethonium chloride, benzoic acid, benzyl alcohol, butyl paraben, cetylpyridinium chloride, cresol, chlorobutanol, dehydroacetic acid, ethylparaben, methylparaben, phenol, phenylethyl alcohol, phenoxyethanol, phenylmercuric acetate, phenylmercuric nitrate, potassium sorbate, propylparaben, sodium benzoate, sodium dehydroacetate, sodium propionate, sorbic acid, thimersol, thymol, and mixtures thereof.

The composition may also comprise any suitable pharmaceutically acceptable carrier or medium. Suitable pharmaceutically acceptable carriers include, for example, any solvents, dispersants, pH buffering agents, coatings, absorption promoting agents, controlled release agents, and one or more inert excipients (e.g., filling agents, starches, polyols, granulating agents, microcrystalline cellulose, diluents, lubricants, binders, disintegrating agents), or the like. In addition, the compositions can contain any desired additional components, additives, and/or species, for example, surface active additives, dispersing additives, humectants, suspending agents, solubilizers, buffering agents, disintegrants, preservatives, colorants, flavorants, and the like. In some embodiments, the composition comprises one or more ion species that interact with linaclotide.

The composition can also comprise any suitable pH buffering agent. In some embodiments, the pH buffering agent is present in the composition in an amount sufficient to achieve the isoelectric point of linaclotide. In the regard, the composition can have any desired pH. In some embodiments, the composition has a pH of 2 to 5 (for example, a pH of 2 to 4.5, a pH of 2 to 4, a pH of 2.5 to 4, a pH of 2.5 to 3.5, a pH of 2.5 to 3, or even a pH of 3).

In some embodiments, the composition comprises linaclotide and a hydrolysis product, e.g., a hydrolysis product comprising or having a structure of: The composition can contain any desired concentration of the hydrolysis product. In some embodiments, the composition comprises less than 10 wt.% of the hydrolysis product. In some embodiments, the composition comprises less than 7 wt.% of the hydrolysis product. In some embodiments, the composition comprises less than 6 wt.% of the hydrolysis product. In some embodiments, the composition comprises less than 5 wt.% of the hydrolysis product. In some embodiments, the composition comprises less than 4 wt.% of the hydrolysis product. In some embodiments, the composition comprises less than 3 wt.% of the hydrolysis product. In some embodiments, the composition comprises less than 2 wt.% of the hydrolysis product. In some embodiments, the composition comprises less than 1 wt.% of the hydrolysis product. In some embodiments, the composition comprises between 0.01 and 10 wt.% of the hydrolysis product. In some embodiments, the composition comprises between 0. 1 and 7 wt.% of the hydrolysis product. In some embodiments, the composition comprises between 0. 1 and 5 wt.% of the hydrolysis product. In some embodiments, the composition comprises between 0.5 and 5 wt.% of the hydrolysis product. In some embodiments, the composition comprises between 1 and 5 wt.% of the hydrolysis product. In some embodiments, the composition comprises between 0. 1 and 4 wt.% of the hydrolysis product. In some embodiments, the composition comprises between 0.5 and 4 wt.% of the hydrolysis product. In some embodiments, the composition comprises between 1 and 4 wt.% of the hydrolysis product. In some embodiments, the composition comprises between 0. 1 and 3 wt.% of the hydrolysis product. In some embodiments, the composition comprises between 0.5 and 3 wt.% of the hydrolysis product. In some embodiments, the composition comprises between 1 and 3 wt.% of the hydrolysis product. In some embodiments, the composition comprises between 0.1 and 2.5 wt.% of the hydrolysis product. In some embodiments, the composition comprises between 0.5 and 2.5 wt.% of the hydrolysis product. In some embodiments, the composition comprises between 1 and 2.5 wt.% of the hydrolysis product. In some embodiments, the composition comprises between 0.1 and 2 wt.% of the hydrolysis product. In some embodiments, the composition comprises between 0.5 and 2 wt.% of the hydrolysis product. In some embodiments, the composition comprises between 1 and 2 wt.% of the hydrolysis product. In some embodiments, the composition comprises between 0.1 and 1.5 wt.% of the hydrolysis product. In some embodiments, the composition comprises between 0.5 and 1.5 wt.% of the hydrolysis product. In some embodiments, the composition comprises between 0.1 and 1 wt.% of the hydrolysis product. In some embodiments, the composition comprises between 0.5 and 1 wt.% of the hydrolysis product.

In some embodiments, the composition comprises linaclotide and an oxidation product, *e.g.,* an oxidation product comprising or having a structure of: Alternatively, or in addition, the composition comprises linaclotide and an oxidation product having the depicted structure but wherein oxidation occurs at any one or more of the six depicted cysteinyl sulfurs. The composition can contain any desired concentration of the oxidation product. In some embodiments, the composition comprises less than 10 wt.% of the oxidation product. In some embodiments, the composition comprises less than 7 wt.% of the oxidation product. In some embodiments, the composition comprises less than 6 wt.% of the oxidation product. In some embodiments, the composition comprises less than 5 wt.% of the oxidation product. In some embodiments, the composition comprises less than 4 wt.% of the oxidation product. In some embodiments, the composition comprises less than 3 wt.% of the oxidation product. In some embodiments, the composition comprises less than 2 wt.% of the oxidation product. In some embodiments, the composition comprises less than 1 wt.% of the oxidation product. In some embodiments, the composition comprises between 0.01 and 10 wt.% of the oxidation product. In some embodiments, the composition comprises between 0. 1 and 7 wt.% of the oxidation product. In some embodiments, the composition comprises between 0. 1 and 5 wt.% of the oxidation product. In some embodiments, the composition comprises between 0.5 and 5 wt.% of the oxidation product. In some embodiments, the composition comprises between 1 and 5 wt.% of the oxidation product. In some embodiments, the composition comprises between 0. 1 and 4 wt.% of the oxidation product. In some embodiments, the composition comprises between 0.5 and 4 wt.% of the oxidation product. In some embodiments, the composition comprises between 1 and 4 wt.% of the oxidation product. In some embodiments, the composition comprises between 0. 1 and 3 wt.% of the oxidation product. In some embodiments, the composition comprises between 0.5 and 3 wt.% of the oxidation product. In some embodiments, the composition comprises between 1 and 3 wt.% of the oxidation product. In some embodiments, the composition comprises between 0.1 and 2.5 wt.% of the oxidation product. In some embodiments, the composition comprises between 0.5 and 2.5 wt.% of the oxidation product. In some embodiments, the composition comprises between 1 and 2.5 wt.% of the oxidation product. In some embodiments, the composition comprises between 0.1 and 2 wt.% of the oxidation product. In some embodiments, the composition comprises between 0.5 and 2 wt.% of the oxidation product. In some embodiments, the composition comprises between 1 and 2 wt.% of the oxidation product. In some embodiments, the composition comprises between 0.1 and 1.5 wt.% of the oxidation product. In some embodiments, the composition comprises between 0.5 and 1.5 wt.% of the oxidation product. In some embodiments, the composition comprises between 0.1 and 1 wt.% of the oxidation product. In some embodiments, the composition comprises between 0.5 and 1 wt.% of the oxidation product.

In some embodiments, the composition comprises linaclotide and an acetylation product, e.g., an acetylation product comprising or having: The composition can contain any desired concentration of the acetylation product. In some embodiments, the composition comprises less than 10 wt.% of the acetylation product. In some embodiments, the composition comprises less than 7 wt.% of the acetylation product. In some embodiments, the composition comprises less than 6 wt.% of the acetylation product. In some embodiments, the composition comprises less than 5 wt.% of the acetylation product. In some embodiments, the composition comprises less than 4 wt.% of the acetylation product. In some embodiments, the composition comprises less than 3 wt.% of the acetylation product. In some embodiments, the composition comprises less than 2 wt.% of the acetylation product. In some embodiments, the composition comprises less than 1 wt.% of the acetylation product. In some embodiments, the composition comprises between 0.01 and 10 wt.% of the acetylation product. In some embodiments, the composition comprises between 0. 1 and 7 wt.% of the acetylation product. In some embodiments, the composition comprises between 0. 1 and 5 wt.% of the acetylation product. In some embodiments, the composition comprises between 0.5 and 5 wt.% of the acetylation product. In some embodiments, the composition comprises between 1 and 5 wt.% of the acetylation product. In some embodiments, the composition comprises between 0. 1 and 4 wt.% of the acetylation product. In some embodiments, the composition comprises between 0.5 and 4 wt.% of the acetylation product. In some embodiments, the composition comprises between 1 and 4 wt.% of the acetylation product. In some embodiments, the composition comprises between 0. 1 and 3 wt.% of the acetylation product. In some embodiments, the composition comprises between 0.5 and 3 wt.% of the acetylation product. In some embodiments, the composition comprises between 1 and 3 wt.% of the acetylation product. In some embodiments, the composition comprises between 0.1 and 2.5 wt.% of the acetylation product. In some embodiments, the composition comprises between 0.5 and 2.5 wt.% of the acetylation product. In some embodiments, the composition comprises between 1 and 2.5 wt.% of the acetylation product. In some embodiments, the composition comprises between 0.1 and 2 wt.% of the acetylation product. In some embodiments, the composition comprises between 0.5 and 2 wt.% of the acetylation product. In some embodiments, the composition comprises between 1 and 2 wt.% of the acetylation product. In some embodiments, the composition comprises between 0.1 and 1.5 wt.% of the acetylation product. In some embodiments, the composition comprises between 0.5 and 1.5 wt.% of the acetylation product. In some embodiments, the composition comprises between 0.1 and 1 wt.% of the acetylation product. In some embodiments, the composition comprises between 0.5 and 1 wt.% of the acetylation product.

In some embodiments, there is provided a pharmaceutical composition comprising linaclotide, and one or more peptides selected from:
i. a peptide ("Cys¹-IMD") or a pharmaceutically acceptable salt thereof, wherein the peptide comprises the amino acid structure of:
ii. a hydrolysis peptide ("Asp⁷") or a pharmaceutically acceptable salt thereof, wherein the peptide comprises the amino acid structure of:
iii. an acetylation peptide ("Cys¹-N-Acetyl") or a pharmaceutically acceptable salt thereof, wherein the peptide comprises the amino acid structure of:
iv. a linaclotide trisulfide peptide or a pharmaceutically acceptable salt thereof, wherein the peptide comprises the amino acid sequence of Cys Cys Glu Tyr Cys Cys Asn Pro Ala Cys Thr Gly Cys Tyr wherein an additional sulfur atom may be attached to any one of the six cysteinyl sulfurs;
v. a peptide ("Des-Tyr¹⁴") or a pharmaceutically acceptable salt thereof, wherein the peptide comprises the amino acid structure of: or
vi. a peptide (Cys¹-α-Ketone) or a pharmaceutically acceptable salt thereof, wherein the peptide comprises the amino acid structure of:

In some embodiments, thea Cys¹-α-Ketone peptide may be present in its hydrated form or a pharmaceutically acceptable salt thereof, wherein the peptide comprises an amino acid structure of: . One skilled in the art would recognize that the Cys¹-α-Ketone peptide would readily convert between its hydrate and ketone form.

In some embodiments, the Cys¹-α-Ketone peptide comprises less than about 15% by weight of the composition, less than about 10% by weight of the composition, less than about 7% by weight of the composition, less than about 5% by weight of the composition, less than about 4% by weight of the composition, less than about 3% by weight of the composition, less than about 2% by weight of the composition, less than about 1.5% by weight of the composition, or less than about 1% by weight of the composition. In other exemplary embodiments, the Cys¹-α-Ketone peptide comprises from about 0.01% to about 15% by weight of the composition, about 0.05% to about 10% by weight of the composition, about 0.05% to about 7% by weight of the composition or about 0.05% to about 5% by weight of the composition.

In some embodiments, the Cys¹-IMD peptide comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary embodiments, the Cys'-IMD peptide comprises from about 0.01% to about 15% by weight of the composition, about 0.05% to about 10% by weight of the composition, about 0.05% to about 7% by weight of the composition or about 0.05% to about 5% by weight of the composition.

In some embodiments, the hydrolysis peptide ("Asp⁷") comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary embodiments, the hydrolysis peptide ("Asp⁷") comprises from about 0.01% to about 15% by weight of the composition, about 0.05% to about 10% by weight of the composition, about 0.05% to about 7% by weight of the composition or about 0.05% to about 5% by weight of the composition.

In some embodiments, the acetylation peptide ("Cys¹-N-Acetyl") comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary embodiments, the acetylation peptide ("Cys¹-N-Acetyl") comprises from about 0.01% to about 15% by weight of the composition, about 0.05% to about 10% by weight of the composition, about 0.05% to about 7% by weight of the composition or about 0.05% to about 5% by weight of the composition.

In some embodiments, the linaclotide trisulfide peptide comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary embodiments, the linaclotide trisulfide peptide comprises from about 0.01% to about 15% by weight of the composition, about 0.05% to about 10% by weight of the composition, about 0.05% to about 7% by weight of the composition or about 0.05% to about 5% by weight of the composition.

In some embodiments, the Des-Tyr¹⁴ peptide comprises less than about 15% by weight of the composition, less than about 10% by weight of the composition, less than about 7% by weight of the composition, less than about 5% by weight of the composition, less than about 4% by weight of the composition, less than about 3.5% by weight of the composition, less than about 3% by weight of the composition, less than about 2% by weight of the composition, or less than about 1% by weight of the composition. In other exemplary embodiments, the Des-Tyr¹⁴ peptide comprises from about 0.01% to about 15% by weight of the composition, about 0.05% to about 10% by weight of the composition, about 0.05% to about 7% by weight of the composition or about 0.05% to about 5% by weight of the composition.

In some embodiments, the composition comprises linaclotide and any desired concentration of multimers. In some embodiments, the composition comprises less than 10 wt.% of multimer(s). In some embodiments, the composition comprises between 0.5 and 1 wt.% of multimer(s).

In some embodiments, the composition comprises an effective amount of linaclotide and any desired amount of reduced form linaclotide. As used herein, the term "reduced form linaclotide" refers to linaclotide having no disulfide bonds between cysteine amino acids. In some embodiments, the composition comprises less than 10 wt.% of reduced form linaclotide. In some embodiments, the composition comprises between 0.5 and 1 wt.% of reduced form linaclotide.

In some embodiments, the composition comprises an effective amount of linaclotide and any desired amount of scrambled form linaclotide. As used herein, the term "scrambled form linaclotide" refers to linaclotide having disulfide bonds between Cys₁ and Cys₁₀, between Cys₁ and Cys₁₃, between Cys₁ and Cys₅, between Cys₁ and Cys₂, between Cys₂ and Cys₆, between Cys₂ and Cys₁₃, between Cys₂ and Cys₅, between Cys₅ and Cys₆, and/or between Cys₅ and Cys₁₀. In some embodiments, the composition comprises between 0.5 and 1 wt.% of scrambled form linaclotide. In some embodiments, the composition comprises less than 10 wt.% of scrambled form linaclotide.

In some embodiments, the composition comprises a total degradant concentration of less than about 10 wt.%. In some embodiments, the composition comprises a total degradant concentration of less than about 8 wt.%. In some embodiments, the composition comprises a total degradant concentration of less than about 7 wt.%. In some embodiments, the composition comprises a total degradant concentration of less than about 6.5 wt.%. In some embodiments, the composition comprises a total degradant concentration of less than about 6 wt.%. In some embodiments, the composition comprises a total degradant concentration of less than about 5.5 wt.%. In some embodiments, the composition comprises a total degradant concentration of less than about 5 wt.%. In some embodiments, the composition comprises a total degradant concentration of less than about 4 wt.%. In some embodiments, the composition comprises a total degradant concentration of less than about 3 wt.%. In some embodiments, the composition comprises a total degradant concentration of less than about 2.5 wt.%. In some embodiments, the composition comprises a total degradant concentration of less than about 2 wt.%. In some embodiments, the composition comprises a total degradant concentration of less than about 1 wt.%.

In some embodiments, the compositions can be prepared by spray drying, which is a technique used to prepare microparticles (e.g., microcapsules or microspheres) of drugs. Spray-dried peptides generally retain their biological activity upon dissolution and may have useful physical characteristics, including a uniform particle size and a spherical shape. In addition, the microparticles prepared by spray drying are often free flowing, which is helpful for pharmaceutical manufacturing processes such as forming tablets and filling capsules. Spray drying processes are also useful because they may be readily scaled up for clinical and commercial manufacturing. In one embodiment, the spray buffer comprises HCl, histidine, 1.5% PVA and 0.6% talc. This formulation can be used to produce lower dosing ranges between 36-290µg.

The composition, when administered, will dissolve to release linaclotide in targeted areas of the gastrointestinal tract. The formulation may release the linaclotide over a period of time that is determined by a number of different factors. These factors include the dimensions of the formulation, the concentration of the linaclotide, and how the linaclotide is dispersed throughout the formulation. For example, by varying the thickness and surface area of the formulations the rate of dissolution may be adjusted. A thick formulation will dissolve more slowly than an otherwise similar thin formulation and may be desirable to administer high dosages of linaclotide.

In some embodiments, the delayed release composition has a disintegration rate of less than about 60 minutes in the targeted pH conditions. In some embodiments, the delayed release composition has a disintegration rate of less than about 30 minutes in the targeted pH conditions. In some embodiments, the delayed release composition has a disintegration rate of less than about 25 minutes. In some embodiments, the delayed release composition has a disintegration rate of less than about 20 minutes. In some embodiments, the delayed release composition has a disintegration rate of less than about 15 minutes. In some embodiments, the delayed release composition has a disintegration rate of less than about 10 minutes. In some embodiments, the delayed release composition disintegrates in less than about 30 minutes after entering a targeted environment. In some embodiments, the delayed release composition disintegrates in less than about 25 minutes after entering a targeted environment. In some embodiments, the delayed release composition disintegrates in less than about 20 minutes after entering a targeted environment. In some embodiments, the delayed release composition disintegrates in less than about 15 minutes after entering a targeted environment.

In some embodiments, the delayed release composition releases at least about 75% of the linaclotide contained therein within 60 minutes of entering a targeted environment. In some embodiments, the delayed release composition releases at least about 75% of the linaclotide contained therein within 30 minutes of entering a targeted environment. In some embodiments, the delayed release composition releases at least about 80% of the linaclotide contained therein within 30 minutes of entering a targeted environment. In some embodiments, the delayed release composition releases at least about 85% of the linaclotide contained therein within 30 minutes of entering a targeted environment. In some embodiments, the delayed release composition releases at least about 90% of the linaclotide contained therein within 30 minutes of entering a targeted environment. In some embodiments, the delayed release composition releases at least about 95% of the linaclotide contained therein within 30 minutes of entering a targeted environment. In some embodiments, the delayed release composition releases at least about 99% of the linaclotide contained therein within 30 minutes of entering a targeted environment.

In some embodiments, the delayed release composition releases at least about 40% of the linaclotide contained therein within 15 minutes of entering a targeted environment. In some embodiments, the delayed release composition releases at least about 50% of the linaclotide contained therein within 15 minutes of entering a targeted environment. In some embodiments, the delayed release composition releases at least about 60% of the linaclotide contained therein within 15 minutes of entering a targeted environment. In some embodiments, the delayed release composition releases at least about 70% of the linaclotide contained therein within 15 minutes of entering a targeted environment. In some embodiments, the delayed release composition releases at least about 80% of the linaclotide contained therein within 15 minutes of entering a targeted environment. In some embodiments, the delayed release composition releases at least about 85% of the linaclotide contained therein within 15 minutes of entering a targeted environment. In some embodiments, the delayed release composition releases at least about 90% of the linaclotide contained therein within 15 minutes of entering a targeted environment. In some embodiments, the delayed release composition releases at least about 95% of the linaclotide contained therein within 15 minutes of entering a targeted environment.

In some embodiments, the delayed release composition releases at least about 80% of the linaclotide contained therein between about 2 to about 2 hours of entering a targeted environment.

In some embodiments, the delayed release composition releases at least about 75% of the linaclotide contained therein within 30 minutes of contacting a pH greater than 5. In some embodiments, the delayed release composition releases at least about 80% of the linaclotide contained therein within 30 minutes of contacting a pH greater than 5. In some embodiments, the delayed release composition releases at least about 85% of the linaclotide contained therein within 30 minutes of contacting a pH greater than 5. In some embodiments, the delayed release composition releases at least about 90% of the linaclotide contained therein within 30 minutes of contacting a pH greater than 5. In some embodiments, the delayed release composition releases at least about 95% of the linaclotide contained therein within 30 minutes of contacting a pH greater than 5. In some embodiments, the delayed release composition releases at least about 99% of the linaclotide contained therein within 30 minutes of contacting a pH greater than 5.

In some embodiments, the delayed release composition releases at least about 75% of the linaclotide contained therein within 30 minutes of contacting a pH greater than 7. In some embodiments, the delayed release composition releases at least about 80% of the linaclotide contained therein within 30 minutes of contacting a pH greater than 7. In some embodiments, the delayed release composition releases at least about 85% of the linaclotide contained therein within 30 minutes of contacting a pH greater than 7. In some embodiments, the delayed release composition releases at least about 90% of the linaclotide contained therein within 30 minutes of contacting a pH greater than 7. In some embodiments, the delayed release composition releases at least about 95% of the linaclotide contained therein within 30 minutes of contacting a pH greater than 7. In some embodiments, the delayed release composition releases at least about 99% of the linaclotide contained therein within 30 minutes of contacting a pH greater than 7.

In some embodiments, the linaclotide DR compositions are formulated for delivery of linaclotide to the ileum, terminal ileum, or colon.

In some embodiments, the linaclotide DR compositions are formulated for delivery of linaclotide to the colon.

The composition can be used to treat other diseases, disorders, or conditions that are responsive to treatment with agonists of the GC-C receptor. The composition can be used to treat any gastrointestinal disorders and/or conditions in a patient (e.g., mammal or human) or inflammation or pain associated therewith. The delayed release compositions of the present invention may be used to treat various conditions, but is particularly suited to treat gastrointestinal disorders, such as irritable bowel syndrome ("IBS") (for example, IBS with constipation "IBS-c", IBS with diarrhea "IBS-d", or mixed IBS with constipation and diarrhea "IBS-m"), constipation (for example, chronic idiopathic constipation), colon cancer, diverticulitis, interstitial cystitis, and abdominal or visceral pain.

Suitable such gastrointestinal disorders and conditions, also include, but are not limited to, irritable bowel syndrome, irritable bowel syndrome with constipation, colon cancer, dyspepsia (including functional dyspepsia or non-ulcer dyspepsia), gastrointestinal motility disorders, functional gastrointestinal disorders, gastroesophageal reflux disease (GERD), Crohn's disease, ulcerative colitis, inflammatory bowel disease, functional heartburn, gastroparesis, chronic intestinal pseudo-obstruction (or colonic pseudo-obstruction), and disorders and conditions associated with constipation, for example, chronic constipation, opioid induced constipation, post-surgical constipation (post-operative ileus), and constipation associated with neuropathic disorders or a combination of symptoms thereof (such as a combination of irritable bowel syndrome and chronic constipation), constipation associated with neuropathic disorders (e.g., constipation associated with Parkinson's Disease), constipation associated with cystic fibrosis or thyroid disease. In some embodiments, a method is provided for treating gastrointestinal disorders in a patient (e.g., mammal or human) diagnosed with one or more gastrointestinal disorders or conditions, wherein the method comprises administering an effective amount of the composition to the patient.

In some embodiments, a method of treating a gastrointestinal disorders comprising administering to a patient in need thereof, a therapeutically effective amount of compositions described herein. The gastrointestinal disorder is selected from the group consisting of: irritable bowel syndrome (IBS), constipation, a functional gastrointestinal disorder, gastroesophageal reflux disease, functional heartburn, inflammatory bowel disease, dyspepsia, diverticulitis pain, visceral pain, or abdominal pain, prostatitis, testicular pain, abdominal or visceral inflammation, painful bladder syndrome, endometriosis, vulvodynia, or rectal pain. In some embodiments, the constipation is chronic constipation, idiopathic constipation, chronic idiopathic constipation, constipation due to post-operative ileus, or constipation caused by opiate use. In other embodiments, the irritable bowel syndrome is irritable bowel syndrome with constipation (IBS-c), irritable bowel syndrome with diarrhea (IBS-d) or mixed irritable bowel syndrome (IBS-m).

In some embodiments, the compositions can be used to treat constipation, irritable bowel syndrome with constipation, and visceral pain. In other embodiments, the compositions can be used to predominantly treat visceral pain or abdominal pain over other GI disorder symptoms.

As described herein above, linaclotide has been previously shown to reduce abdominal pain which is thought to be mediated by of cGMP into the submucosal/basolateral space. However, due to the reducing environment of the intestinal tract, it is believed that much of the oral linaclotide dose is degraded prior to reaching the distal colon. In human volunteers treated with linaclotide, only about 5% of the oral dose is found in feces. The compositions described herein are delayed release ("DR") compositions of linaclotide that target the lower GI may improve linaclotide's efficacy towards relieving pain associated with various GI disorders by allowing for delivery of a higher dose of linaclotide to the colon. As such, in one embodiment, the compositions described herein are useful for the treatment of the lower GI.

Such DR compositions of linaclotide would have the potential to release linaclotide predominantly (or fully) in the lower GI. As a result, for example, the DR formulation or composition would have an increased capacity to treat lower GI associated disorders. Moreover, it may have a capacity to cause lower incidences of adverse events (such as diarrhea) than the immediate-release dosage form, e.g., because it would cause lower overall intestinal fluid secretion by not activating GC-C receptors in the upper GI. This would occur while maintaining or even improving linaclotide efficacy for treating symptoms of GI disorders, such as pain.

In some embodiments, the compositions described herein are useful for the treatment of diseases or symptoms associated with visceral pain selected from the group consisting of general abdominal pain, diverticular disease, pain associated with irritable bowel syndrome (IBS), chronic or acute radiation proctopathy (also referred to as radiation proctitis), rectal pain, chronic proctalgia, proctalgia fugax, anal pain, chronic anal fissure, post-operative anal pain, overactive bladder syndrome, stress incontinence, interstitial cystitis, bladder pain syndrome, pain associated with cancer, pain associated with gastrointestinal tract neoplasms, general pelvic pain, endometriosis, orchialgia, chronic prostatitis, prostatodynia, vulvodynia, urethral syndrome, penile pain, perianal pain, ulcerative colitis, ulcerative proctitis, and Crohn's disease. In one particular embodiment, the compositions described herein are useful for the treatment of bladder pain syndrome. In another particular embodiment, the compositions described herein are useful for the treatment of overactive bladder syndrome (including for example bladder hypersensitivity or colitis induced bladder afferent hyperactivity).

In still another embodiment, the compositions described herein are useful for the treatment of interstitial cystitis. In still another embodiment, the compositions described herein are useful for the treatment of endometriosis. In another embodiment, the compositions described herein are useful for the treatment of anal pain.

In some embodiments, a method of treating a disorder is provided comprising administering to a patient in need thereof, a therapeutically effective amount of the composition described herein. In some embodiments, the disorder is cancer selected from colorectal/local metastasized colorectal cancer, intestinal polyps, Barrett's esophagus, gastrointestinal tract cancer, lung cancer, cancer or pre-cancerous growths or metastatic growths of epithelial cells, polyps, breast, colorectal, lung, ovarian, pancreatic, prostatic, renal, stomach, bladder, liver, esophageal and testicular carcinoma.

In some embodiments, methods are provided for preventing a cancer or hyperplasia of the gastrointestinal tract or preventing reoccurrence of cancer or hyperplasia of the gastrointestinal tract in a patient in need thereof comprising administering an effective amount of the composition or the oral dosage form to the patient. In some embodiments, the cancer or hyperplasia is colorectal cancer, intestinal polyps or pre-cancerous growths or metastatic growths of gastrointestinal epithelial cells. In some embodiments, the composition or oral dosage form is administered simultaneously or sequentially with an effective amount of a COX-2 inhibitor. Examples of highly selective and selective COX-2 inhibitors include etoricoxib, rofecoxib, lumiracoxib, valdecoxib, celecoxib (Celebrex®), sulindac, diclofenac, meloxicam and etodolac. Non-selective NSAIDs that inhibit COX-2 include naproxen, ibuprofen, sodium salicylate and diflunisal. As used herein, the term "prevent" or "preventing" means to arrest, delay the onset (i.e., the period prior to clinical manifestation of a disease) or reoccurrence of cancer or hyperplasia, and/or reduce the risk of developing cancer or hyperplasia relative to a patient that has not been treated with a composition described herein.

In some embodiments, methods are provided for treating gastrointestinal disorders in pediatric patients with the compositions and oral dosage forms described herein. In some embodiments, methods are provided for treating gastrointestinal disorders in a pediatric patient diagnosed with one or more gastrointestinal disorders or conditions, wherein the method comprises administering an effective amount of the composition or the oral dosage form to the patient. In some embodiments, methods are provided to use the compositions and oral dosage forms for treating gastrointestinal disorders including, but not limited to, GI motility disorders, irritable bowel syndrome, constipation-predominant irritable bowel syndrome (IBS-c), mixed-type irritable bowel syndrome (IBS-m), chronic constipation, chronic idiopathic constipation, opioid induced constipation, post-surgical constipation (post-operative ileus), constipation associated with neuropathic disorders, constipation associated with cystic fibrosis or thyroid disease, dyspepsia (including functional dyspepsia or non-ulcer dyspepsia), gastroparesis, gastrointestinal motility disorders, functional gastrointestinal disorders, gastroesophageal reflux disease (GERD), inflammatory bowel disease, Crohn's disease, ulcerative colitis, functional heartburn, chronic intestinal pseudo-obstruction (or colonic pseudo-obstruction), visceral pain, abdominal pain, pelvic pain, anal fissure pain, pain associated with vulvodynia, pain associated with endometriosis, pain associated with fibromyalgia, functional abdominal pain, interstitial cystitis pain, diverticulitis, pain associated with diverticulitis, and pain associated with celiac sprue. In some embodiments, methods are provided to treat IBS-c, IBS-m or chronic constipation (e.g., chronic idiopathic constipation) in pediatric patients with the compositions and oral dosage forms described herein. In some embodiments, methods are provided to treat IBS-c in a pediatric patient in need thereof. In some embodiments, methods are provided to treat chronic idiopathic constipation in a pediatric patient in need thereof.

In some embodiments, a method is for treating or relieving pain comprising administering to a patient in need thereof, a therapeutically effective amount of the composition described herein. In some embodiments, the pain is selected from visceral pain; abdominal pain; pelvic pain; or pain associated with gastrointestinal disorders, venereal diseases, bladder pain syndrome, diverticulitis pain, prostatitis, testicular pain, endometriosis, vulvodynia, rectal pain.or interstitial cystitis. In some embodiments, the pain is selected from pelvic pain, pain associated with proctitis, anal fissure pain, pain associated with vulvodynia, pain associated with endometriosis, pain associated with fibromyalgia, functional abdominal pain, interstitial cystitis pain, pain associated with venereal disease, diverticulitis, pain associated with diverticulitis, and pain associated with celiac sprue.

In another embodiment, the method is for increasing intestinal motility in a patient in need thereof, comprising administering an effective amount of the composition to the patient. Intestinal motility involves spontaneous coordinated dissentions and contractions of the stomach, intestines, colon and rectum to move food through the gastrointestinal tract during the digestive process. In some embodiments, the disorder is post-operative ileus, or constipation caused by opiate use.

In exemplary embodiments, the methods may comprise administering a therapeutically effective amount of the pharmaceutical composition to a patient in need thereof. An effective amount of a composition comprising linaclotide or a pharmaceutically acceptable salt thereof required to achieve desired results (such as desired treatment and/or symptom relief) of a subject is dependent on several understood factors, such as the identity and severity of the disorder being treated, as well as the age, weight, etc., of the patient being treated.

In some embodiments, the composition or oral dosage form is administered to a pediatric patient in need thereof as a tablet, capsule or sachet. In some embodiments, a sachet comprising the composition is opened and the contents are sprinkled on or stirred into food, such as applesauce, or into a beverage, such as water. In some embodiments, a capsule is swallowed whole with fluid, such as water, or is opened and sprinkled on or stirred into food or a beverage. Tablets may be swallowed whole, may be crushed and stirred into food or a beverage, or may be formulated as a chewable tablet. The composition of the present invention is an enteric coated tablet.

A subject or patient in whom administration of the pharmaceutical composition is an effective therapeutic regimen for a disease or disorder is preferably a human, but can be any animal, including a laboratory animal in the context of a clinical trial or screening or activity experiment. Thus, as can be readily appreciated by one of ordinary skill in the art, the methods, compounds and compositions described herein are particularly suited for administration to any animal, particularly a mammal, and including, but by no means limited to, humans, rodents and non-rodents, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., *e.g.,* for veterinary medical use.

**In** some embodiments, the linaclotide composition may be formulated as a rectal dosage form for rectal administration. Rectal dosage forms include, without limitation, rectal suppositories, rectal foams or aerosols, enemas, rectal gels and rectal ointments. In some embodiments, the rectal dosage form may be administered to a patient in need thereof. In some embodiments, the rectal dosage form may be administered to a patient to treat abdominal or rectal pain, pain from anal fissures, ulcerative colitis, Crohn's disease or inflammatory bowel disease. In some embodiments, the rectal dosage form may be administered to a pediatric or geriatric patient.

In some embodiments, the effective dose range of linaclotide for adult humans is from 25 µg to 6 mg per day orally. In some embodiments, the dose range is 15 µg to 2 mg per day orally. In some embodiments, the dose range for adult humans is 50 µg to 1 mg per day orally (*e.g.,* 15 µg, 30 µg, 50 µg 72 µg, 100 µg 145 µg 150 µg, 200 µg, 250 µg, 290 µg 300 µg, 350 µg 400 µg, 450 µg, 500 µg 550 µg, 579 µg, 600 µg, 650 µg, 700 µg, 750 µg 800 µg, 850 µg, 900 µg, 950 µg or 1 mg). In some embodiments, the dose range for adult humans is 30 µg to 300 µg per day orally In some embodiments, the dose range is 100 µg to 600 µg per day orally. In some embodiments, the dose is 30 µg, 100 µg, 150 µg, 200 µg, 300 µg, 400 µg, 500 µg or 600 µg linaclotide per day orally. In some embodiments, the dose is 50 µg linaclotide per day orally. In some embodiments, the dose is 100 µg linaclotide per day orally. In some embodiments, the dose is 145 µg linaclotide per day orally. In some embodiments, the dose is 200 µg linaclotide per day orally. In some embodiments, the dose is 290 µg linaclotide per day orally. In some embodiments, the dose is 300 µg linaclotide per day orally. In some embodiments, the dose is 500 µg linaclotide per day orally. In some embodiments, the dose is 600 µg linaclotide per day orally.

In some embodiments, the effective pediatric dose range of linaclotide is from 0.05 µg to 2 mg per day orally. In some embodiments, the effective pediatric dose range of linaclotide is from 0.05 µg to 100 µg per day orally. In some embodiments, the effective pediatric dose range of linaclotide is from 0.1 µg to 90 µg per day orally. In some embodiments, the effective pediatric dose range of linaclotide is from 0.1 µg to 50 µg per day orally. In some embodiments, the effective pediatric dose range of linaclotide is from 0.1 µg to 25 µg per day orally. In some embodiments, the effective pediatric dose range of linaclotide is from 0.1 µg to 10 µg per day orally. In some embodiments, the effective pediatric dose range of linaclotide is from 0.1 µg to 5 µg per day orally. In some embodiments, the effective pediatric dose range of linaclotide is from 0.1 µg to 1 µg per day orally. In some embodiments, the effective pediatric dose range of linaclotide is from 0.1 µg to 0.5 µg per day orally. In some embodiments, the effective pediatric dose range of linaclotide is 0.1 µg per day orally. In some embodiments, the effective pediatric dose range of linaclotide is 0.15 µg per day orally. In some embodiments, the effective pediatric dose range of linaclotide is 0.25 µg per day orally. In some embodiments, the effective pediatric dose range of linaclotide is 0.5 µg per day orally. In some embodiments, the effective pediatric dose range of linaclotide is 3.5 µg per day orally. In some embodiments, the effective pediatric dose range of linaclotide is 15 µg per day orally. In some embodiments, the effective pediatric dose range of linaclotide is 36 µg per day orally. In some embodiments, the effective pediatric dose range of linaclotide is 45 µg per day orally. In some embodiments, the effective pediatric dose range of linaclotide is 60 µg per day orally. In some embodiments, the effective pediatric dose range of linaclotide is 90 µg per day orally. In some embodiments, the unit dosage form and daily dose are equivalent.

In some embodiments, the unit dosage form is administered with food at any time of the day, without food at any time of the day, with food after an overnight fast (*e.g.,* with breakfast). In some embodiments, the unit dosage form is administered once a day, twice a day or three times a day. In some embodiments, one, two or three unit dosage forms will contain the daily oral dose of linaclotide. The precise amount of compound administered to a patient will be the responsibility of the attendant physician. However, the dose employed will depend on a number of factors, including the age and sex of the patient, the precise disorder being treated, and its severity.

In some embodiments, the compositions are administered as a monotherapy. In some embodiments, the composition consists essentially of an effective amount of linaclotide. In some embodiments, the composition consists of an effective amount of linaclotide.

In some embodiments, the compositions are directly administered to a patient, for example, in the form of delayed release tablet or delayed release capsule. In some embodiments, the compositions are dissolved, disintegrated and/or mixed on or within food or beverage prior to administration to patients (*e.g.,* elderly or pediatric patients). In some embodiments, the composition is dissolved or disintegrated in a liquid, solution, or fluid optionally containing stabilizing agent(s), preservative(s), sweetener(s), or the like, etc. prior to administration to a patient (*e.g.,* elderly or pediatric patient). In some embodiments, the composition is a multiple dose composition, *i.e.,* containing two, three, five, seven, ten, fifteen, twenty, twenty-five, thirty, forty, fifty, sixty, seventy, eighty, ninety or more daily doses of linaclotide.

In other embodiments, the compositions are administered as part of a combination therapy. For example, a composition may be used in combination with other drugs or therapies that are used in the treatment, prevention, suppression, and/or amelioration of the diseases or conditions for which compounds of the invention are useful. The linaclotide can be co-administered or co-formulated with other medications. In one embodiment, the linaclotide composition can be co-administered with other medications used to treat gastrointestinal disorders including but not limited to acid suppressing agents such as Histamine-2 receptor agonists (H2As) and/or proton pump inhibitors (PPIs). In one embodiment, the linaclotide composition can be co-administered with other medications used to treat gastrointestinal disorders including 5-ASAs such as mesalamine.

Such other drug(s) may be administered, by a route and in an amount commonly used therefore, contemporaneously or sequentially with a compound of the invention. When a compound of the present invention is used contemporaneously with one or more other drugs, a pharmaceutical unit dosage form containing such other drugs in addition to the compound of the invention may be employed. Accordingly, the pharmaceutical compositions of the present invention include those that also contain one or more other active components, in addition to a compound of invention.

Several methods can be used for evaluating the bioactivity of the linaclotide composition, including, but not limited to, immunoassays (*e.g.,* enzyme-linked immunosorbent assay), radioimmuno assays, immunoradiometric assays, gel electrophoresis (*e.g.,* SDS-PAGE), high performance liquid chromatography (HPLC), and/or high performance capillary electrophoresis (HPCE). In some embodiments, the bioactivity of the composition is assessed by a method comprising fixing linaclotide, incubating linaclotide with guanylate cyclase C (GCC), incubating GCC bound linaclotide with antibodies against GCC, incubating GCC antibody-bound linaclotide with fluorescently labeled antibodies against GCC antibodies, and detecting the linaclotide bound to the GCC antibodies by measuring the fluorescence intensity using a plate reader. The drug concentration can then be calculated based on the fluorescence reading of the solution.

For example, the bioactivity of the linaclotide compositions can be assessed and quantified using the following method, though other methods are available. The composition is added to a volumetric flask containing 60 ml of phosphate buffer having a pH of 4.5, and the flask is shaken for 60 minutes. 0.2 ml of the supernatant is then removed, and is added into one or more wells of a 96-well plate that is coated with GC-C receptors. The plate is sealed and incubated at 37°C for 2 hr. At the end of incubation, the sample is removed and the plate is washed with phosphate buffered saline (PBS). The bound linaclotide is then incubated for 1 hour, at room temperature, with GC-C (such as is available from Sigma-Aldrich Inc.) labeled with fluorescein isocyanate (FITC) in blocking buffer. After incubation, the well is washed with PBS. The fluorescence intensity of the end product is detected, for example, by using a plate reader. The linaclotide concentration is then calculated based on the fluorescence reading of the solution.

### Definitions

As used herein, unless otherwise indicated, the term "delayed release" mean that the composition dissolves, melts, disintegrates, liquefies, etc. in a targeted area of the gastrointestinal tract such that substantially all of the linaclotide no longer remains in a formulation, composition, or dosage form. Delayed release compositions include sustained release compositions, gastro-retentive compositions, targeted release compositions (e.g. colonic-release compositions, or compositions that target the ileosecal valve, etc.), extended release compositions and/or combinations thereof.

As used herein, unless otherwise indicated, the term "delayed release composition" ("DR") means that the composition is a dosage form that releases linaclotide at a time other than immediately following oral administration.

As used herein, unless otherwise indicated, the term "extended release composition" means that the composition is a dosage form that releases linaclotide over an extended period of time after administration. This allows a reduction in dosing frequency compared to immediate release compositions.

As used herein, unless otherwise indicated, the "disintegration" and "release" is used herein to mean that the capsule, film, bead, or tablet comprising linaclotide dissolves, melts, disintegrates, liquefies, etc. in the environment of an oral cavity such that substantially all of the linaclotide no longer remains in a formulation form, e.g., a pH greater than 5 or 7, or in a phosphate buffer solution and maintained at 37 ± 1°C.

The term "released from", when referring to the release of linaclotide from the composition, unless otherwise indicated, is used herein to mean that the linaclotide no longer remains in a composition form.

As used herein, unless otherwise indicated, the term "entry into a targeted environment" means contact of the composition within a patient at a targeted organ or segment thereof, or within a segment of the GI intended for linaclotide release, *e.g.,* having a pH greater than 5 or 7.

As used herein, unless otherwise indicated, the term "lower gastrointestinal (GI)" means the distal segment of the gastrointestinal tract, for example, the ileum, terminal ileum, ileocecal valve, or colon.

As used herein, unless otherwise indicated, the term "upper gastrointestinal (GI)" means the proximate segment of the gastrointestinal tract, for example, the stomach, duodenum and/or jejunum.

As used herein, unless otherwise indicated, "stabilizing agent" refers to a polymer, sterically hindered primary amine (*e.g.,* amino acid), or cation (*e.g.,* metal cation) component of the composition which is included in the composition in a stabilizing amount. For example, a polymeric stabilizing agent is a polymer that is included in the composition in a stabilizing amount. Similarly, a sterically hindered primary amine stabilizing agent is a sterically hindered primary amine that is included in the composition in a stabilizing amount. Moreover, a cationic stabilizing agent is a cation that is included in the composition in a stabilizing amount.

As used herein, unless otherwise indicated, "stabilizing amount" refers to a concentration, within the composition, of a polymer, sterically hindered primary amine (e.g., amino acid), or metal cation component at which the component increases the stability of linaclotide in the composition, as compared to a similar composition not having a stabilizing amount of the same component.

As used herein, unless otherwise indicated, the term "substantially all" means at least about 90%, for example, at least about 95% or even at least about 99%.

As used herein, unless otherwise indicated, the term "isolated and purified" means at least 95 percent pure (for example, at least 96% pure, at least 97% pure, at least 98% pure, or even at least 99% pure), as measured, for example, by chromatographic purity using HPLC.

As used herein, unless otherwise indicated, "therapeutically effective amount" means the amount of a linaclotide or a pharmaceutically acceptable salt thereof that, when administered to a mammal for treating a state, disorder or condition, is sufficient to effect a treatment (as defined below). The "therapeutically effective amount" will vary depending on the compound, the disease and its severity and the age, sex, weight, physical condition and responsiveness of the mammal to be treated. For example, a therapeutically effective amount of linaclotide, or its pharmaceutically acceptable salt or hydrate, can be an amount effective to treat gastrointestinal disorders, including irritable bowel syndrome, constipation-predominant irritable bowel syndrome, chronic constipation, opioid induced constipation and/or dyspepsia.

As used herein, unless other indicated, "pharmaceutically acceptable" means biologically or pharmacologically compatible for *in vivo* use in animals or humans, and preferably means, approved by a regulatory agency of the Federal or a state government or listed in the U.S. Pharmacopeia or other generally recognized pharmacopeia for use in animals, and more particularly in humans.

As used herein, unless otherwise indicated, the term "treat", in all its verb forms, is used herein to mean to relieve, alleviate, prevent, and/or manage at least one symptom of a disorder in a subject, the disorder including, for example, a gastrointestinal disorder, such as, irritable bowel syndrome, constipation-predominant irritable bowel syndrome, chronic constipation, opioid induced constipation, dyspepsia, or a combination of symptoms thereof. Within the meaning of the present invention, the term "treat" also denotes, to arrest, delay the onset (*i.e.,* the period prior to clinical manifestation of a disease) and/or reduce the risk of developing or worsening a disease. The term "treatment" means the act of "treating" as defined above.

As used herein, unless otherwise indicated, the term "additives" refers to a pharmaceutically acceptable additive. Pharmaceutically acceptable additives include, without limitation, binders, disintegrants, dispersing additives, lubricants, glidants, antioxidants, coating additives, diluents, surfactants, flavoring additives, humectants, absorption promoting additives, controlled release additives, anti-caking additives, anti-microbial agents (e.g., preservatives), colorants, desiccants, plasticizers and dyes.

As used herein, unless otherwise indicated, an "excipient" is any pharmaceutically acceptable additive, filler, binder or agent.

As used herein, unless otherwise indication, "stressed conditions" refer to 40°C and 75% relative humidity (RH).

As used here, unless otherwise indicated, the terms "about" and "approximately" mean within an acceptable error range for the particular value as determined by one of ordinary skill in the art, which will depend, in part, on how the value is measured or determined, *i.e.,* the limitations of the measurement system. For example, "about" can mean within 1 or more than 1 standard deviation, per practice in the art. Alternatively, "about" with respect to the compositions can mean plus or minus a range of up to 20%, preferably up to 10%. Alternatively, particularly with respect to biological systems or processes, the term can mean within an order of magnitude, preferably within 5-fold, and more preferably within 2-fold, of a value. Particular values are described in the application and claims, unless otherwise stated the term "about" means within an acceptable error range for the particular value.

All weight percentages (*i.e.,* "% by weight" and "wt.%" and w/w) referenced herein, unless otherwise indicated, are measured relative to the total weight of the pharmaceutical composition.

The term "consisting essentially of", and variants thereof, when used to refer to the composition, are used herein to mean that the composition includes linaclotide and other desired pharmaceutically inactive additives, excipients, and/or components (*e.g.,* polymers, sterically hindered primary amines, cations, filling agents, binders, carriers, excipients, diluents, disintegrating additives, lubricants, solvents, dispersants, coating additives, absorption promoting additives, hydrolysis products, formaldehyde imine products, oxidation products, acetylation products, deamidation products, multimers, controlled release additives, anti-caking additives, anti-microbial additives, preservatives, sweetening additives, colorants, flavors, desiccants, plasticizers, dyes, or the like), and no other active pharmaceutical ingredient(s).

### EXAMPLES

The following examples are merely illustrative of the present invention and should not be construed as limiting the scope of the invention in any way as many variations and equivalents that are encompassed by the present invention will become apparent to those skilled in the art upon reading the present disclosure.

Enteric-coated tablets comprising a core immediate release tablet containing a unit dose of linaclotide can be coated with coatings that dissolve only under pH conditions of the distal segment of intestine, so that linaclotide will be released in lower GI tract. Only the enteric coated tablets as claimed are according to the invention. The other formulations are disclosed for reference purposes.

Linaclotide or a pharmaceutically acceptable salt thereof may be produced and purified using standard techniques known in the art, e.g., chemical synthesis or recombinant expression followed by purification using standard techniques.

Preparation of the linaclotide coating solution for beads: Approximately 32 g to 42 g of purified water is mixed with hydrochloric acid to create a solution with a pH between 1.5 and 2.0. The cation, if used, is added to the solution in a quantity to provide the desired concentration, and the solution is mixed for sufficient time to produce a clear solution. The sterically hindered primary amine, if used, is added to the solution in a quantity to provide the desired concentration, and the solution is mixed for sufficient time to produce a clear solution. Other additives, such as antioxidants, are then added, if desired. The pH of the solution is tested, and hydrochloric acid is added, if necessary, to produce a solution having a pH between 1.5 and 2.0. The binder is then added to the solution and the mixture is then stirred for sufficient time to achieve a clear solution. The desired amount of linaclotide is added to the solution and mixed for 30-100 minutes to provide the coating solution. PVA and 0.6% talc. This formulation can be used to produce dosing ranges between 30-300µg.

Preparation of the Active Beads: Approximately 30-36 g of dried microcrystalline cellulose beads are added to a Mini Column Fluid Bed Coater. The microcrystalline cellulose beads are fluidized and heated prior to layering. Next, the coating solution is layered to the beads. The spraying temperature is controlled between 24°C and 55°C by controlling inlet temperature, spray rate, atomization pressure, and air volume. After the entire coating solution is layered to the beads, the beads are dried. The product of this process is referred to as active beads.

### Example 1

### Delayed release Linaclotide Tablet

Linaclotide can be formulated into a tablet for delayed drug release. Compared to an equal volume of beads, tablets have much smaller specific surface area, which makes them potentially less prone to degradation induced by environmental factors such as humidity, oxidation, deamidation, etc. In addition, the smaller surface area of the tablet can become advantageous when an enteric coating is needed since much less coating material is required to cover the surface of the dosage form.

Enteric coatings may be applied in a tablet coating pan, and coatings that are used for delayed release beads can be used for tablets to form delayed release tablets. The amount of coating polymer on the tablet can vary from 5 to 60% (weight gain) depending on the size, shape and surface properties of the tablet. A sub-coat can be applied to the tablets to separate linaclotide from the enteric coat.

### Example 2 (Reference Example) Enteric coated tablet

**Table 1 Eudragit® FS30D Coated linaclotide delayed release (DR) tablet composition**

| | **Ingredients** | **Wt.%** | **Wt. in kg** |
|---|---|---|---|
| **Fluid bed Granulation** | | | |
| 1. | Linaclotide | 0.3 | 2.94 |
| 2 | Isomalt | 93.7 | 937 |
| 3 | Histidine | 0.46 | 4.6 |
| 4 | Calcium chloride dihydrate | 2.57 | 25.7 |
| 5 | Polyvinyl pyrrolidone (PVP) | 3 | 30 |
| 6 | 0.01N HCl | Q.S. | Q.S. |
| 7 | Purified Water | Q.S. | Q.S. |

| **Blending and compression** | | | |
|---|---|---|---|
| i | Linaclotide granules | 27.85 | 139.25 |
| ii | Isomalt | 60.9 | 304.5 |
| iii | Crospovidone | 10 | 50 |
| iv | Magnesium stearate | 0.75 | 3.75 |
| vi | Talc | 0.5 | 2.5 |

| **Enteric coating** | | | |
|---|---|---|---|
| | Linaclotide tablet | 75.19 | 1000 |
| | Eudragit® FS 30D | 22.56 | 1000 |
| | PlasACR YL™ | 2.25 | 150 |
| | Purified Water* | - | 500 |
| | **Total Dry weight** | **100** | **1330** |

### Manufacturing process:

### A. Tablet

The granulation solution may be prepared by dissolving PVP, histidine and calcium chloride in water, adjusting solution pH to 2, and dissolving linaclotide. Granulation is performed in a fluid bed by spraying the granulation solution onto filler isomalt. At the end of granulation, dry the granules for 30 min. The granules are then blended with tablet components including isomalt, crospovidone, Mg stearate and talc until uniform, and compressed into tablets.

### B. Enteric coating

For tablet coating, linaclotide core tablets are placed into a pan coater and warmed up to 35°C. Start tablets coating with Eudragit® FS 30 D suspension, keep the product temperature at 28° to 32°C, and atomization air pressure at 3 bar. At the end of coating, discharge the tablets and place them into a circulated air oven and dry for 2 h at 40°C.

Similarly, other enteric coatings such as Eudragit® L, S, ethyl cellulose, HPMCAS, PVAP, CAP, CAS, etc. may also be applied to form delayed release tablets at various weight gains.

### Example 3 (Only the enteric coated tablets as claimed are according to the invention; the other formulations are disclosed for illustrative purposes only)

### Delayed release compositions comprising linaclotide

Delayed release capsules comprising linaclotide may be formulated to target the ileum or colon. The composition is formulated to include a pH triggered release based on enteric coating of a linaclotide tablet, capsule or linaclotide coated beads contained in a hard gelatin capsule. The composition may be formulated to further comprise stabilizing additives such as a divalent cation and an amino acid. PVA can be used as binder as well as protective layer in between linaclotide and enteric coating. Linaclotide or linaclotide with PVA overcoat (as beads, capsule or tablet) may be coated with an additional enteric coating (e.g. Eudragit® FS30D, Eudragit® S100, Eudragit® L100, Eudragit®L100-55, Eudragit® L 30D-55) that dissolves in a pH dependent manner to release at the appropriate pH of 7 in the ileum of the GI tract. The enteric coatings may consist of blends combining different types of Eudragit® - Eudragit® S100 / Eudragit® L100 in different ratios (e.g. 50/50 ratio); Eudragit® S100 / Eudragit® L100-55 in various ratios; Eudragit ® FS30D/Eudragit® L 30D-55, Eudragit® FS30DEudragit® S / Eudragit® RS or EC in various ratios. The compositions may further comprise other excipients including plasticizing agents such as triethylcitrate. The coatings may further comprise disintegrants as suspended solid to expedite the relevant pH triggered release - resulting in mixed systems as croscarmellose sodium / Eudragit® S. For ease of processing, anti-tacking agent (e.g., talc, Aerosil® 200 or PlasAcryl™) may be used to prevent the beads from sticking.

Additionally, two Eudragit® coatings may be applied to ensure swift release once the desired pH region in the GI tract is reached - including partially neutralized coating systems. Buffering agents such as potassium hydrogen phosphate can be included into one of the two Eudragit® films. Alternative non-Eudragit® pH dependent film coatings include hydroxypropylmethylcellulose acetate succinate (HPMCAS, e.g. Aqoat® AS-HF), cellulose acetate phthalate (CAP, e.g. Aquateric®) or shellac.

### Example 4

### Measurement of content and purity of exemplary peptides

Linaclotide, immidazolidinone degradant product ("Cys¹-IMD"), and α-ketone degradant product ("Cys¹-α-Ketone") can be measured and purified as described in US 2010/0048489, US 2013/0190238, and US 2015/0094272. Generally, content and purity of linaclotide may be determined by reverse phase gradient liquid chromatography using an Agilent Series 1100 LC System with Chemstation Rev A.09.03 software or equivalent. A YMC Pro™ C18 column (dimensions: 3.0 x 150 mm, 3.5 um, 120 Å; Waters Corp., Milford, MA) or equivalent is used and is maintained at 40°C. Mobile phase A (MPA) consists of water with 0.1% trifluoroacetic acid while mobile phase B (MPB) consists of 95% acetonitrile:5% water with 0.1% trifluoroacetic acid. Elution of the linaclotide is accomplished with a gradient from 0% to 47% MPB in 28 minutes followed by a ramp to 100% MPB in 4 minutes with a 5 minute hold at 100% MPB to wash the column. Re-equilibration of the column is performed by returning to 0% MPB in 1 minute followed by a 10 minute hold at 100% MPA. The flow rate is 0.6 mL/min and detection is accomplished by UV at 220 nm.

### Example 5

### In vitro drug release testing

Linaclotide release from the coated tablets can be assessed by dissolution testing using a USP XXIV type II paddle dissolution apparatus (model PTWS, Pharma Test, Hainburg, Germany). The tests are conducted in triplicates, at a paddle speed of 100 rpm in 900 ml dissolution medium maintained at 37.0 ± 0.5°C. Tablets are tested first in 0.1 N hydrochloric acid (pH 1.2) for 30 minutes or 2 hours to simulate gastric residence and then for 6 hours in buffer media of varying pH and ionic composition, akin to small intestinal pH conditions. Linaclotide release may be assessed at pH 6.8-7.4 in two compendial buffer media: 0.067 M mixed sodium and potassium phosphate (Sorensen's) buffer and 0.05 M potassium phosphate buffer as well as in a pH 7.4 multi-electrolyte salt solution (Hanks) buffer which is similar in ionic composition to intestinal fluid. All the buffers are freshly prepared with de-ionized water and de-aerated by sparging with helium prior to use. The amount of linaclotide released from the dosage form can be determined by reverse phase gradient liquid chromatography as described in example 4.

### Example 6

### Linaclotide Tablet Release Profiles

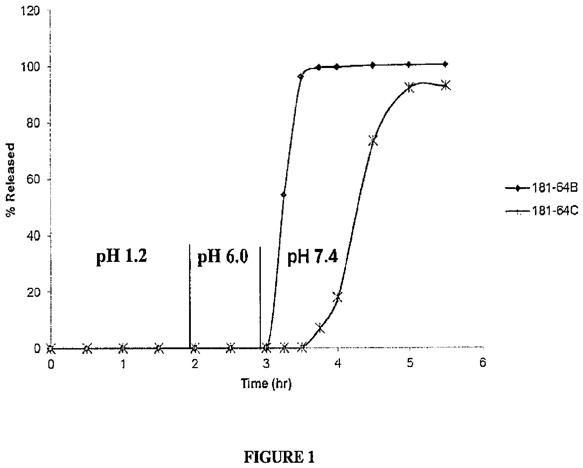

Linaclotide tablet release profiles have been tested at various pHs for three delayed release formulations. Sample181-64B contains a 100 µg dose of linaclotide, a subcoat of Opadry® II, and a functional coat of Eudragit® FS 30D and talc. Sample 181-64C contains a 100 µg dose of linaclotide, a subcoat of Opadry II, and a functional coat of Eudragit® FS 30D and Eudragit® PlasAcryl™. The release profiles for Lot 181-64B and Lot 181-64C are provided in Figure 1.

### Example 7

### Linaclotide Tablet Preparation

Delayed release tablets may be prepared by first preparing the following core tablet components: a placebo base, a linaclotide 750 µg/225 mg base, and pre-granulated fillers.

### Granulation manufacturing process:

The tablet components may be prepared into separate granulations for blending before tablet compression. Use of separate tablet components, such as, the placebo base and pregranulated filler base provided, among other things, advantageous properties for stability and release profiles for the tablets. For example, all the tablets components listed in Table 2 could be separately prepared by wet granulation and blended before compression or blended together and processed as a mixture for wet granulation. In another process, the tablets components listed in Table 2 could be separately prepared by dry granulation and blended before compression or blended together and processed as a mixture for dry granulation. In another process, the tablet components are direct blended for compression. In a preferred process, the pregranulated filler base and/or placebo base are prepared through wet granulation and dried before mixing with the 750 µg/225 mg linaclotide base. The linaclotide base could be prepared by wet granulation processes or by Wurster coating process. This preferred process, exhibited further gains in stability for the tablet by reducing moisture exposure to linaclotide during processing and minimizing residue moisture in the tablet core.

**Table 2: Components for various tablet strengths (the 25 µg-dose is not according to the invention)**

| Strength | Placebo | 25 µg | 30µg | 50 µg | 75 µg | 100 µg | 150 µg | 290 µg | 300µg |
|---|---|---|---|---|---|---|---|---|---|
| Placebo base (%) | 20.00 | 16.67 | 16 | 13.33 | 10.00 | 6.67 | 3.33 | 0.00 | 0 |
| Linaclotide base (i.e.750 ug/225 mg base (%)) | 0.00 | 3.33 | 4 | 6.67 | 10.00 | 13.33 | 16.67 | 38.65 | 40 |
| Pregranulated fillers (%) | 78.75 | 78.75 | 78.75 | 78.75 | 78.75 | 78.75 | 78.75 | 60.10 | 58.75 |
| Magnesium Stearate (%) | 1.25 | 1.25 | 1.25 | 1.25 | 1.25 | 1.25 | 1.25 | 1.25 | 1.25 |
| Total (%) | 100.0 | 100.0 | 100 | 100.0 | 100.0 | 100.0 | 100.0 | 100.0 | 100 |

Then compress the above blends on a suitable tablet press to target core tablet weight of 225 mg. In a perforated pan coater, add a sub-coat (OPADRY® II) at a weight gain of 4% w/w. Coating conditions should be set and monitored so that moisture uptake during coating is kept to a minimum. When measured by loss on drying (LOD), the sub-coated tablets should have no more than 1.5% LOD. In a perforated pan coater, add a functional coat on the subcoated tablets. The functional coat is either Eudragit® FS30D, Eudragit® S100, or Eudragit® L100. Apply the functional coat at 5 mg polymer weight/cm² of the tablet surface. This comes to be approximately 4.5% total polymer weight gain during functional coating. Coating conditions should be set and monitored so that moisture uptake during coating is kept to a minimum. When measured by loss on drying, the functionally coated tablet should have no more than 2.0% LOD.

### Placebo Base Preparation:

Table 3 represents the formulation for the placebo base granulation:

**Table 3: Formulation of the placebo base granulation**

| Component | %w/w | Quantity (g) |
|---|---|---|
| L-Histidine | 2.26 | 112.9 |
| Calcium Chloride Dihydrate | 1.07 | 53.5 |
| polyvinyl alcohol | 1.50 | 75.0 |
| microcrystalline cellulose | 95.17 | 4758.6 |
| Hydrochloric acid, pure, fuming, 37% solution in water | | |
| Treated water | | |
| Total | 100 | 5000 |

The placebo base preparation may be prepared by first dispensing the raw materials of Table 4.

**Table 4: Raw materials for placebo base preparation**

| Component | Quantity (g) |
|---|---|
| L-Histidine | 242.2 |
| Calcium Chloride Dihydrate | 114.8 |
| polyvinyl alcohol | 160.9 |
| microcrystalline cellulose | 4758.6 |

Tare the mix container and add 2682.1 ± 5.0 g of treated water into the container. Set up a mixer and begin to stir the water. Add the EMPROVE® to the water while stirring and start the timer. Cover and heat solution to 70C while stirring and maintain temperature until material is visually dissolved.

Adjust the pH of solution to 1.5 with hydrochloric acid. Add calcium chloride dihydrate to the solution while stirring. Mix until dissolved. Add L-Histidine to the solution while stirring. Stir for approximately 15 minutes. Record the initial pH. Adjust pH of solution to 5.0 with hydrochloric acid. Record final pH of solution and hydrochloric acid addition. Mix until all material is dissolved. While mixing, adjust the pH solution to 2.5 with hydrochloric acid. Record final pH of solution and hydrochloric acid addition. Ensure that the high shear granulator is set up properly for granulating with the 25L bowl, mixing blade and chopper. Pass microcrystalline cellulose through 16 mesh screen into granulator bowl. Calculate the net weight of granulation solution to add. Pump the granulation solution into the granulator at a rate of approximately 300g/min while mixing with the below parameters: Impeller speed 1(290 rpm, 5.5m/s tip speed), Chopper speed 1(1760 rpm). Stop the granulator and scrape down the sides and the bottom of the bowl. Mix for an additional 3 minutes according to the following parameters: Impeller speed 1(290 rpm, 5.5m/s tip speed), Chopper speed 1(1760 rpm). Tare a poly bag and discharge the completed wet granulation into it. Weigh the granulation. Transfer the wet granulation to the FLM-3 fluid bed for drying. Dry the granulation using the following approximate settings. Dry until the granulation LOD is no more than 1.2% moisture. Discharge the dried granulation into a tared poly bag.

| **Parameters** | **Target Range** |
|---|---|
| Product Temperature | 40°C |
| Process (Drying) Air | 30 - 60 CFM |
| Inlet Temperature | 60°C |

Settings are suggested settings only and may be adjusted for optimum drying.

Screen the dried granulation through a #30 mesh sieve. Tare a poly bag and discharge the dried granulation into it. Weigh the granulation. Package dried granulation into foil sealed bags with desiccant.

### Linaclotide Base Preparation (i.e. 750µg/225mg):

Table 5 represents the formulation for the 750µg/225mg base granulation:

**Table 5: Formulation for the 750µg/225mg base granulation**

| Component | % w/w | Quantity (g) |
|---|---|---|
| Linaclotide | 0.39 | 19.3 |
| L-Histidine | 2.26 | 112.9 |
| Calcium Chloride Dihydrate | 1.07 | 53.5 |
| polyvinyl alcohol | 1.50 | 75.0 |
| microcrystalline cellulose | 94.79 | 4,739.3 |
| Hydrochloric acid, pure, fuming, 37% solution in water | ----- | ----- |
| Treated water | ----- | ----- |
| Total | 100.00 | 5,000.0 |

The 750µg/225mg base granulation may be prepared by first dispensing the raw materials of Table 6.

**Table 6: Raw materials of the linaclotide base granulation**

| Raw Material | Required Quantity (g) |
|---|---|
| Linaclotide | 19.3 |
| microcrystalline cellulose | 4,739.3 |
| Granulation solution | |

While mixing, add the linaclotide to the granulation solution. Mix until dissolved. Ensure that the high shear granulator is set up properly for granulating with the 25L bowl, mixing blade and chopper. Pass microcrystalline cellulose through 16 mesh screen into granulator bowl. Pump the granulation solution into the granulator at a rate of approximately 300g/min while mixing with the below parameters: Impeller speed 1(290 rpm, 5.5m/s tip speed), Chopper speed 1(1760 rpm). Tare a poly bag and discharge the completed wet granulation into it. Weigh the granulation. Transfer the wet granulation to the fluid bed for drying. Dry the granulation using the following approximate settings. Dry until the granulation LOD is no more than 1.2% moisture. Discharge the dried granulation into a tared poly bag.

| **Parameters** | **Target Range** |
|---|---|
| Process (Drying) Air | 30 - 60 CFM |
| Inlet Temperature | 80°C |

| | |
|---|---|
| Note: Settings are suggested settings only and may be adjusted for optimum drying. | |

Screen the dried granulation through a #30 mesh sieve. Tare a poly bag and discharge the dried granulation into it. Weigh the granulation. Package dried granulation into foil sealed bags with desiccant.

### Pregranulated Filler Preparation:

Table 7 represents the formulation for the pregranulated fillers.

**Table 7: Formulation of the fillers granulation**

| Component | % w/w | Qty per sublot (g) |
|---|---|---|
| microcrystalline cellulose | 19.4 | 1,358 |
| croscarmellose sodium | 5.1 | 357 |
| mannitol | 71.7 | 5,019 |
| polyvinyl alcohol | 3.8 | 266 |
| Treated water | ----- | ----- |
| Total | 100.0 | 7,000 |

The fillers preparation may be prepared by first dispensing the raw materials of Table 8.

**Table 8: Raw materials for preparation of fillers granulation**

| Raw Material | Required Quantity (g) |
|---|---|
| polyvinyl alcohol | 266 |
| microcrystalline cellulose | 1,358 |
| croscarmellose sodium | 357 |
| mannitol | 5,019 |
| Total | --- |

Then record the tare weight of the stainless steel container. Tare the container and weigh the required quantity of treated water into the container. Transfer the water into a jacketed kettle. Set up the mixer and begin to stir the water in the kettle. Add the EMPROVE® (polyvinyl alcohol) to the water while stirring and start the timer. Cover and heat solution to 70°C while stirring and maintain temperature until material is visually dissolved. Calculate weight of water lost due to evaporation during heating. Add this amount of treated water to the solution. Add each of microcrystalline cellulose, croscarmellose sodium), and mannitol to a high shear granulator bowl. Mix for approximately 2 minutes according to the following parameters: Impeller speed 1(290 rpm, 5.5m/s tip speed), Chopper speed 1(1760 rpm). Pump 2217 ± 5 g of the granulation solution into the granulator at a rate of approximately 300g/min while mixing with the below parameters: Impeller speed 1(290 rpm, 5.5m/s tip speed), Chopper speed 1(1760 rpm). Stop the granulator and scrape down the sides and the bottom of the bowl. Mix for an additional 30 seconds to 1 minute according to the following parameters: Impeller speed 1(290 rpm, 5.5m/s tip speed), Chopper speed 1(1760 rpm). Tare a poly bag and discharge the completed wet granulation into it. Weigh the granulation. Pass the wet granulation through the Comil with 2A375Q03763 screen with 5-10% power. Transfer the wet granulation to the FLM-3 fluid bed for drying. Dry the granulation using the following approximate settings. Dry until the granulation LOD is no more than 1.0% moisture. Discharge the dried granulation into a tared poly bag.

| Parameters | Target Range |
|---|---|
| Process (Drying) Air | 30 - 60 CFM |
| Inlet Temperature | 80°C |

### Settings are suggested settings only and may be adjusted for optimum drying.

Mill the granulation with Comil, round impeller, 2A045R03137 screen. Tare a poly bag and discharge the dried and milled granulation into it. Weigh the granulation. Package dried granulation into foil sealed bags with desiccant.

### Example 8

### 25µg Tablet Blending and Compression (Reference Example)

According to the procedure of Example 7, a 25µg dose tablet of linaclotide may be prepared with the formulation of Table 9.

**Table 9: Formulation of a 25 µg Linaclotide Tablet:**

| Component | % w/w | Quantity (g) |
|---|---|---|
| Pregranulated Fillers | 78.750 | 5118.8 |
| Linaclotide Base | 3.333 | 216.7 |
| Placebo Base | 16.667 | 1083.4 |
| Magnesium Stearate | 1.250 | 81.3 |
| Total | 100 | 6500 |

First, dispense the raw materials of Table 10.

**Table 10: Raw materials for preparation of 25µg linaclotide tablet**

| Raw Material | Required Quantity (g) |
|---|---|
| Pregranulated Fillers | 5118.8 |
| Linaclotide Base | 227.5 |
| Placebo Base | 1137.5 |
| Magnesium Stearate | 81.2 |
| Total | 6565.0 |

Preblend: Set up an 8 qt blender and add the 750µg/225mg Base and Placebo Base. Close lids and blend for 10 minutes. Tare a poly bag, and discharge the preblend into it.

Sub-Blend A: Add 650g of the preblend to a 16 qt v-blender. Add the Pregranulated Fillers for sub-blend A to the 16 qt v-blender. Close lids and blend for 10 minutes. Pass the Magnesium Stearate for sub-blend A through the 40 mesh screen. Add to the 16 qt v-blender. Close lids and blend for 3 minutes. Tare a poly bag, and discharge the blend into it.

Sub-Blend B: Add 650g of the preblend to a 16 qt v-blender. Add the Pregranulated Fillers for sub-blend B to the 16 qt v-blender. Close lids and blend for 10 minutes. Pass the Magnesium Stearate for sub-blend B through the 40 mesh screen. Add to the 16 qt v-blender. Close lids and blend for 3 minutes. Tare a poly bag, and discharge the blend into it.

Compression: Ensure a Korsch XL 100 tablet press with 0.32" sc toolings is set up with 0.32" round concave tooling (10 stations desired), paddle feeder, and that all other components are properly secured. Manually rotate die table at least one full revolution to ensure proper installation. Visually confirm presence of all dies and punches, and that dies are flush with the die table. Verify that the tablet specifications meet the In-Process Tablet Specifications of Table 11.

**Table 11: In-Process Tablet Specifications**

| Parameters | Target |
|---|---|
| Average Tablet Weight | 225 mg |
| Individual Thickness | 4.0 - 4.5 mm (guideline only) |
| Individual Tablet Hardness | 10 kP |
| Friability (100 drops) | NO MORE THAN 0.6% |
| Individual Disintegration | NO MORE THAN 15 min |

Charge the blend into the hopper. Set the turret speed to an appropriate speed. Adjust the die fill amount and compression parameters to yield a tablet with a target weight of 225 mg and with a target hardness of 8-12 kP. All waste material should be collected in the tableting waste poly bag. Start timer and compress tablets.

### Example 9

### 100µg Tablet Blending and Compression

According to the procedure of Example 7, a 100µg dose tablet of linaclotide may be prepared with the formulation of Table 12.

**Table 12: Formulation of a 100 µg Linaclotide Tablet:**

| Component | % w/w | Quantity (g) |
|---|---|---|
| Pregranulated Fillers | 78.750 | 9843.8 |
| Linaclotide Base | 13.333 | 1666.6 |
| Placebo Base | 6.667 | 833.4 |
| Magnesium Stearate | 1.250 | 156.3 |
| Total | 100 | 12500 |

First, dispense the raw materials of Table 13.

**Table 13: Raw materials for preparation of 100µg linaclotide tablet**

| Raw Material | Required Quantity (g) |
|---|---|
| Pregranulated Fillers | 9843.8 |
| Linaclotide Base | 1750.0 |
| Placebo Base | 875.0 |
| Magnesium Stearate | 156.3 |
| Total | 12,625.0 |

Preblend: Set up an 16 qt blender and add the 750µg/225mg Base and Placebo Base. Close lids and blend for 10 minutes. Tare a poly bag, and discharge the preblend into it.

Add 2500g of the preblend to a 1 cubic foot v-blender. Add the Pregranulated Fillers to the 1 cubic foot v-blender. Close lids and blend for 10 minutes. Pass the Magnesium Stearate through the 40 mesh screen. Add to the 1 cubic foot v-blender. Close lids and blend for 3 minutes. Tare a poly bag, and discharge the blend into it.

Compression: Ensure a Korsch XL 100 tablet press with 0.32" sc toolings is set up with 0.32" round concave tooling (10 stations desired), paddle feeder, and that all other components are properly secured. Manually rotate die table at least one full revolution to ensure proper installation. Visually confirm presence of all dies and punches, and that dies are flush with the die table. Verify that the tablet specifications meet the In-Process Tablet Specifications of Table 14.

**Table 14: In-Process Tablet Specifications**

| Parameters | Target |
|---|---|
| Average Tablet Weight | 225 mg |
| Individual Thickness | 4.0 - 4.5 mm (guideline only) |
| Individual Tablet Hardness | 10 kP |
| Friability (100 drops) | NO MORE THAN 0.6% |
| Individual Disintegration | NO MORE THAN 15 min |

Charge the blend into the hopper. Set the turret speed to an appropriate speed. Adjust the die fill amount and compression parameters to yield a tablet with a target weight of 225 mg and with a target hardness of 8-12 kP. All waste material should be collected in the tableting waste poly bag. Start timer and compress tablets.

### Example 10

### 290µg Tablet Blending and Compression

According to the procedure of Example 7, a 290µg dose tablet of linaclotide may be prepared with the formulation of Table 15.

**Table 15: Formulation of a 290 µg Linaclotide Tablet:**

| Component | % w/w | Quantity (g) |
|---|---|---|
| Pregranulated Fillers | 60.10 | 3906.5 |
| Linaclotide Base | 38.65 | 2512.3 |
| Magnesium Stearate | 1.25 | 81.3 |
| Total | 100 | 6500 |

First, dispense the raw materials of Table 16.

**Table 16: Raw materials for preparation of 290µg linaclotide tablet**

| Raw Material | Required Quantity (g) |
|---|---|
| Pregranulated Fillers | 3906.6 |
| Linaclotide Base | 2512.2 |
| Magnesium Stearate | 81.2 |
| Total | 6500.0 |

Sub-blend A: Add the Pregranulated Fillers and Linaclotide base for sub-blend A to a 16 qt v-blender. Close lids and blend for 10 minutes. Pass the Magnesium Stearate for sub-blend A through the 40 mesh screen. Add to the 16 qt v-blender. Close lids and blend for 3 minutes. Tare a poly bag, and discharge the blend into it.

Sub-blend B: Add the Pregranulated Fillers and Linaclotide Base for sub-blend B to a 16 qt v-blender. Close lids and blend for 10 minutes. Pass the Magnesium Stearate for sub-blend B through the 40 mesh screen. Add to the 16 qt v-blender. Close lids and blend for 3 minutes. Tare a poly bag, and discharge the blend into it.

Compression: Ensure a Korsch XL 100 tablet press with 0.32" sc toolings is set up with 0.32" round concave tooling (10 stations desired), paddle feeder, and that all other components are properly secured. Manually rotate die table at least one full revolution to ensure proper installation. Visually confirm presence of all dies and punches, and that dies are flush with the die table. Verify that the tablet specifications meet the In-Process Tablet Specifications of Table 17.

**Table 17: In-Process Tablet Specifications**

| Parameters | Target |
|---|---|
| Average Tablet Weight | 225 mg |
| Individual Thickness | 4.0 - 4.5 mm (guideline only) |
| Individual Tablet Hardness | 10 kP |
| Friability (100 drops) | NO MORE THAN 0.6% |
| Individual Disintegration | NO MORE THAN 15 min |

Charge the blend into the hopper. Set the turret speed to an appropriate speed. Adjust the die fill amount and compression parameters to yield a tablet with a target weight of 225 mg and with a target hardness of 8-12 kP. All waste material should be collected in the tableting waste poly bag. Start timer and compress tablets.

### Example 11

### Sub-Coating of Linaclotide 25 µg Tablets (Reference Example)

The 25µg tablets of Example 8 may be sub-coated with an Opadry® II sub-coating. The formula of Table 18 represents the amounts needed to prepare the coating material, which is prepared in excess to account for losses in the coating process.

**Table 18: Sub-Coating formula for 25µg tablets**

| Component | %w/w | g/batch |
|---|---|---|
| Opadry II White | 20 | 400 |
| Purified water | 80 | 1600 |
| Total | 100 | 2000 |

Dispense 1600g of purified water into a suitably sized container. Dispense 400g of Opadry® II into a suitably sized container. Add Opadry® to the water. Calculate the theoretical amount of solution needed to apply a 4.0% weight gain, with 85% theoretical efficiency. Prepare a poly bag for the waste tablets collected during the coating process. Ensure the Compu-Lab has been set up with the 19 inch pan and plenum assembly. Verify the liquid feed lines are Tygon 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Verify initial tablet weight by weighing 100 uncoated tablets. Calculate the average weight of a single tablet by dividing the weight of the tablets by 100. Test the initial tablet moisture by crushing approximately 1 gram of tablets and running LOD for 10 minutes at 105°C. Adjust the pump so that the liquid flow rate is approximately 10 g/min. Prime the lines past the guns and verify that there is no leaking in the lines or gun. Charge the tablets into the coating pan and begin warming for 20 minutes with an inlet temperature of 50°C and airflow of 350 CFM. Jog occasionally during warm-up.

Once target bed temperature is reached, begin spraying the coating suspension according to the target process parameters outlined in Table 19 below. Test and report tablet moisture content periodically as desired. Once the theoretical amount of solution has been applied check the tablets for weight gain. When 4% weight gain has been achieved, stop spray and dry tablets for 30 minutes with an inlet temperature of 50°C, reducing pan speed to a minimum or jogging. Discharge the tablets and determine the new weight of the coated tablets.

**Table 19: Target Process Parameters**

| Parameters | Target |
|---|---|
| Spray rate | 10 g/min |
| Inlet temperature | 65°C |
| Airflow | 350 CFM |
| Atomization air | 40 PSI |
| Pan speed | 11 RPM |
| Exhaust temperature | 47°C |
| Bed Temperature | 50°C |

### Example 12

### Sub-Coating of 100 µg Linaclotide Tablets

The 100µg tablets of Example 9 may be sub-coated with an Opadry® II coating. The formula of Table 20 represents the amounts needed to prepare the coating material, which is prepared in excess to account for losses in the coating process.

**Table 20: Sub-Coating formula for 100µg tablets**

| Component | %w/w | g/batch |
|---|---|---|
| Opadry II White | 20 | 1000 |
| Purified water | 80 | 4000 |
| Total | 100 | 5000 |

Dispense 4000g of purified water into a suitably sized container. Dispense 1000g of Opadry® II into a suitably sized container. Add Opadry® to the water. Calculate the theoretical amount of solution needed to apply a 4.0% weight gain, with 85% theoretical efficiency. Ensure the Compu-Lab has been set up with the 24 inch pan and plenum assembly. Verify the liquid feed lines are Tygon 17 tubing. Verify the gun assembly is installed in the pan. The spray gun assembly should consist of 2 x ¼ JAU spray gun mounted with a 40100 AB liquid spray nozzle and matching air cap. The gun assembly should be mounted as far as possible from the tablet bed, with the spray angle perpendicular to the top third of the bed. Verify initial tablet weight by weighing 100 uncoated tablets. Calculate the average weight of a single tablet by dividing the weight of the tablets by 100. Test the initial tablet moisture by crushing approximately 1 gram of tablets and running LOD for 10 minutes at 105°C. Adjust the pump so that the liquid flow rate is approximately 20 g/min. Prime the lines past the guns and verify that there is no leaking in the lines or gun. Charge the tablets into the coating pan and begin warming for 20 minutes with an inlet temperature of 50°C and airflow of 400 CFM. Jog occasionally during warm-up.

Once target bed temperature is reached, begin spraying the coating suspension according to the target process parameters outlined in Table 21 below. Test and report tablet moisture content periodically as desired. Once the theoretical amount of solution has been applied check the tablets for weight gain. When 4% weight gain has been achieved, stop spray and dry tablets for 30 minutes with an inlet temperature of 50°C, reducing pan speed to a minimum or jogging. Discharge the tablets and determine the new weight of the coated tablets.

**Table 21: Target Process Parameters**

| Parameters | Target |
|---|---|
| Spray rate | 20 g/min |
| Inlet temperature | 65°C |
| Airflow | 400 CFM |
| Atomization air | 40 PSI |
| Pan speed | 10 RPM |
| Exhaust temperature | 47°C |
| Bed Temperature | 50°C |

### Example 13

### Sub-Coating of 290 µg Linaclotide Tablets

The 290µg tablets of Example 10 may be sub-coated with an Opadry® II coating. The formula of Table 22 represents the amounts needed to prepare the coating material, which is prepared in excess to account for losses in the coating process.

**Table 22: Sub-Coating formula for 100µg tablets**

| Component | %w/w | g/batch |
|---|---|---|
| Opadry II White | 20 | 400 |
| Purified water | 80 | 1600 |
| Total | 100 | 2000 |

Dispense 1600g of purified water into a suitably sized container. Dispense 400g of Opadry® II into a suitably sized container. Add Opadry® to the water. Calculate the theoretical amount of solution needed to apply a 4.0% weight gain, with 85% theoretical efficiency. Ensure the Compu-Lab has been set up with the 19 inch pan and plenum assembly. Verify the liquid feed lines are Tygon 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Verify initial tablet weight by weighing 100 uncoated tablets. Calculate the average weight of a single tablet by dividing the weight of the tablets by 100. Test the initial tablet moisture by crushing approximately 1 gram of tablets and running LOD for 10 minutes at 105°C. Adjust the pump so that the liquid flow rate is approximately 10 g/min. Prime the lines past the guns and verify that there is no leaking in the lines or gun. Charge the tablets into the coating pan and begin warming for 20 minutes with an inlet temperature of 50°C and airflow of 350 CFM. Jog occasionally during warm-up.

Once target bed temperature is reached, begin spraying the coating suspension according to the target process parameters outlined in Table 23 below. Test and report tablet moisture content periodically as desired. Once the theoretical amount of solution has been applied check the tablets for weight gain. When 4% weight gain has been achieved, stop spray and dry tablets for 30 minutes with an inlet temperature of 50°C, reducing pan speed to a minimum or jogging. Discharge the tablets and determine the new weight of the coated tablets.

**Table 23: Target Process Parameters**

| Parameters | Target |
|---|---|
| Spray rate | 10 g/min |
| Inlet temperature | 65°C |
| Airflow | 350 CFM |
| Atomization air | 40 PSI |
| Pan speed | 1 RPM |
| Exhaust temperature | 47°C |
| Bed Temperature | 50°C |

### Example 14

### Functional or Enteric Coating of Linaclotide Tablets

The tablets of previous examples may be prepared with a functional coating. The formulation of Table 24 represents the amounts needed to prepare the coating material, which is prepared in excess to account for losses in the coating process:

**Table 24: Formulation for functional coating process**

| **Component** | **%w/w** |
|---|---|
| Eudragit ® S100 | 8.25 |
| Eudragit ® L100 | 1.69 |
| IN NH₃ | 6.75 |
| Triethyl Citrate | 4.97 |
| Talc | 4.97 |
| Purified water | 73.37 |
| Total | 100 |

To prepare the functional coating, dispense the required quantity of Eudragit® S100 and Eudragit® L100 into a suitably sized container. Dispense 2/3 of the required quantity of purified water into a suitably sized container. Begin agitation of water until a vortex is achieved. Add Eudragit® S100 and Eudragit® L100 slowly to the water and mix until the powder is thoroughly wetted and lump or foam formation has dissipated (about 5 minutes).

Dispense the required quantity of IN NH3 into a suitable sized container. Add the IN NH3 slowly into the Eudragit® suspension and mix for a minimum of 60 minutes. Dispense the required quantity of Triethyl Citrate into a suitable sized container. Add the Triethyl Citrate into the Eudragit® suspension and mix for a minimum of 60 minutes. Dispense the required quantity of Talc into a suitable sized container. Homogenize the talc in the remaining 1/3 of purified water for 10 minutes (or until homogenous) using a Silverson Homogenizer. Pour the talc suspension into the Eudragit® suspension while mixing. Mix for no longer than 5 minutes. Screen the coating suspension through a #30 mesh screen.

Calculate the theoretical amount of solution needed to apply a 9.0% weight gain, with 90% theoretical efficiency. Ensure the Compu-Lab has been set up with the 19 inch pan and plenum assembly. Verify the liquid feed lines are Tygon 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Charge the tablets into the coating pan. Warm the tablets with an inlet temperature of 30 degrees. Ensure bed temperature reaches approximately 30 degrees before proceeding to next step. Adjust the pump so that the liquid flow rate is approximately 12 g/min. Prime the lines past the guns and verify that there is no leaking in the lines or gun. Test the starting tablet moisture by crushing approximately 2 gram of tablets and running LOD for 10 minutes at 105°C.

Once target bed temperature is reached, begin spraying the coating suspension according to the target process parameters outlined in Table 25 below. Test and report tablet moisture content periodically as desired. Once the theoretical amount of solution has been applied check the tablets for weight gain. When 9% weight gain has been achieved, stop spray and dry tablets for 5-10 minutes with an inlet temperature of 40°C, reducing pan speed to a minimum or jogging.

**Table 25: Target process parameters for spray coating process**

| **Parameters** | **Target** |
|---|---|
| Spray rate | 12 - 20 g/min¹ |
| Inlet temperature | 43°C¹ |
| Airflow | 300 CFM |
| Atomization air | 35 PSI |
| Pan speed | 12 RPM |
| Exhaust temperature | 33°C |
| Bed Temperature | 35°C |

| | |
|---|---|
| ¹ Adjust as needed to maintain bed temperature. | |

Test the final tablet moisture by crushing approximately 2 gram of tablets and running LOD for 10 minutes at 105°C. Moisture should be ≤ the initial tablet moisture. Discharge the tablets and determine the new weight of the coated tablets. Dry tablets for at least 2 hours in a mechanical convection oven with the temperature set to 40°C. Bulk package tablets in foil bag with desiccants and store at 5°C.

### Example 15

### Organic Coating of Linaclotide Tablets

An organic coating may be provided for the linaclotide tablets of the examples above. For the coating of 100 µg tablets, the formula of Table 26 was used.

**Table 26: Organic coating material formula for linaclotide tablets.**

| **Component** | **%w/w** | **g/batch** |
|---|---|---|
| Eudragit S100 | 2.941 | 88.2 |
| Eudragit L100 | 2.941 | 88.2 |
| Triethyl Citrate | 1.177 | 35.3 |
| Talc | 2.941 | 88.2 |
| Acetone | 34.290 | 1028.7 |
| Isopropanol | 51.420 | 1542.6 |
| Purified Water | 4.290 | 128.7 |
| Total | 100 | 3000 |

Dispense the required quantity of purified water into a suitable sized container. Dispense the required quantity of acetone into a suitable sized container. Begin mixing acetone and add the water. Dispense the required quantity of isopropanol into a suitable sized container. Add isopropanol to the water and acetone to create the diluent mixture. Pour approximately half of the diluent mixture into a second container and resume mixing the first half of the diluent mixture. Dispense the required quantity of Eudragit S100 into a suitable sized container. Begin mixing the second half of diluent mixture and add the Eudragit S100. Dispense the required quantity of Eudragit L100 into a suitable sized container.

Add the Eudragit L100 to the diluent mixture containing the Eudragit S100 and start timer. Stir until the polymers are completely dissolved. Add the triethyl citrate to the first half of the diluent mixture while mixing with a high shear mixer. Dispense the required quantity of talc into a suitable sized container. Add the talc to the first half of the diluent mixture while mixing with a high shear mixer to create the excipient suspension. Start the timer and mix for at least 10 minutes. Record the mixing time of the Eudragit solution. While continuing to mix the Eudragit solution, slowly pour the excipient suspension into the Eudragit solution. Once the coating suspension is uniform pass it through a 35 mesh screen. Resume mixing. Calculate the theoretical amount of solution needed to apply a 8.5% weight gain, with 88% theoretical efficiency.

Prepare a poly bag for the waste tablets collected during the coating process. Ensure the Compu-Lab has been set up with the 15 inch pan and plenum assembly. Verify the liquid feed lines are solvent resistant 17 tubing. Verify the gun assembly is installed in the pan. The spray gun assembly should consist of 1 x ¼ JAU spray gun mounted with a 40100 AB liquid spray nozzle and matching air cap. The gun assembly should be mounted as far as possible from the tablet bed, with the spray angle perpendicular to the top third of the bed. Adjust the pump so that the liquid flow rate is approximately 28 g/min. Prime the lines past the guns and verify that there is no leaking in the lines or gun. Charge the tablets into the coating pan and begin warming with an inlet temperature of 38°C and airflow of 200 CFM. Jog occasionally during warm-up. Once the product temperature reaches about 27°C, begin spraying the coating suspension according to the target process parameters outlined below. When 8.5% weight gain has been achieved, stop spray and dry tablets for 10 minutes with an inlet temperature of 40°C, reducing pan speed to a minimum or jogging.

| Parameters | Target |
|---|---|
| Spray rate | 27-30 g/min |
| Inlet temperature | 30-40°C |
| Airflow | 200 CFM |
| Atomization air | 17 PSI |
| Pan speed | 13 RPM |
| Exhaust temperature | 25-28°C |
| Bed Temperature | 26-27°C |

Place tablets onto trays and dry for at least 24 hours in a mechanical convection oven with the temperature set to 45°C.

### Example 16 (Reference Example)

### Effects of linaclotide in an animal model of overactive bladder/interstitial cystitis

The aims of this non-clinical non-GLP study were to investigate the underlying mechanisms of TNBS induced bladder overactivity and the ability of linaclotide to reduce TNBS induced changes in bladder function by:

Determining changes in bladder afferent mechanosensitivity in chronic visceral hypersensitivity (CVH) mice compared to healthy mice.

Examining changes in the chemo-sensitivity of bladder afferents and detrusor smooth muscle from healthy and CVH mice to exogenous agonists, such as an acetylcholine receptor agonist (carbachol), purinergic receptor agonists (αβMe-ATP), and TRPV1 agonist (capsaicin).

Determining the excitability of retrogradely traced bladder DRG neurons from both thoracolumbar and lumbosacral regions in healthy and CVH mice. Determining the effect of chronically administered once daily linaclotide for 2 weeks on the excitability of retrogradely traced bladder DRG neurons from both thoracolumbar and lumbosacral regions in healthy and CVH mice.

The TNBS model used in this study was previously described and demonstrated to induce chronic visceral hypersensitivity (CVH) to mechanical stimulation of colonic afferents at 28 days post-TNBS administration (Hughes et al., (2009) Gut, 58: 1333-41.; Castro et al., (2013) Gastroenterology, 145: 1334-1346).

### Animals

C57/BL6 mice were sourced from SAHMRI bio resources as per our standard practice. Mice were initially group-housed under standard light/dark cycles and had free access to water and standard diet. Mice were individually housed during the 28-day experimental period.

*Healthy mice:* Male C57/BL6 mice between 13-17 weeks of age.

*Induction of colitis (TNBS recovery chronic visceral hypersensitivity (CVH) mice):*

Male C57 BL/6 mice were used in all experiments. Colitis was induced by administration of TNBS as described previously (Brierley et al., (2011) J. Physiol, 15: 3575-3593). Briefly, 13-week-old mice were administered an intra-colonic enema of 0.1 mL TNBS (130 µg/mL in 30% EtOH) via a polyethylene catheter inserted 3 cm from the anus of isoflurane anaesthetized mice. Mice were then individually housed and observed daily for changes in body weight, physical appearance and behavior. Histological examination of mucosal architecture, cellular infiltrate, crypt abscesses, and goblet cell depletion confirmed TNBS-induced significant damage by day 3-post treatment, largely recovered by day 7 and fully recovered at the day 28-time point (Brierley et al., (2008) Gastroenterology, 134: 2059-69). High-threshold nociceptors from mice at the 28 day time point display significant mechanical hypersensitivity, lower mechanical activation thresholds and display hyperalgesia and allodynia. As such, they are therefore termed 'chronic visceral hypersensitivity' (CVH) mice.

### Ex-vivo afferent recordings from bladder pelvic afferents

Pelvic bladder afferents are classified by their ability to respond to urinary bladder ramp distension. During bladder distension there is an increase in intravesical pressure and a corresponding increase in the afferent neuron activity. The mechanosensitivity of these afferents was tested in bladder preparations from healthy and CVH mice as well as CVH mice that received either a 2 week treatment of once daily oral gavage of vehicle or of linaclotide (3µg/kg/day) by ramp distension to a final intravesical pressure of 30mmHg.

Recordings were made from healthy and CVH mice, which had been chronically administered either vehicle or linaclotide (3µg/kg/day) for a total duration to 2 weeks, and which were euthanized via CO₂ inhalation and cervical dislocation. The animal was cut in two at the level of L2, removing vascular connections via the aorta and vena cava, yet ensuring the kidneys and ureters remained intact. The tail and hind limbs were removed and the remaining pelvic section consisting of bladder, testes, urethra, ureter and kidneys was placed into ice-cold Krebs solution whilst being transported to the recording chamber. The recording chamber was continually perfused with oxygenated (95% O2 and 5% CO₂) Krebs-bicarbonate solution (composition, mM: NaCl 118.4, NaHCO₃ 24.9, CaCl₂ 1.9, MgSO₄ 1.2, KH₂PO₄ 1.2, Glucose 11.7) at a stable temperature of 35°C to maintain functioning tissue.

Under a dissection microscope (WPI, PZMIII), the ureters were identified and ligated using silk suture (US 4/0) to prevent back flow. Excess tissue surrounding the bladder neck was removed to expose the pubic symphasis. The pubic symphasis was cut along the rostral/caudal axis of the urethra and connective tissue attaching them together was removed. The pelvic bone on either side of the urethra was cut away to the point of attachment to the hind legs to provide increased visibility and ease of dissection for the pelvic nerves. The urethral ending was cut and a catheter (OD 0.03IN) attached to a syringe pump (Genie, Kent, multi-phaser™ model NE-1000) was inserted into the bladder and tied in place using silk suture. The bladder was then filled with 0.9% saline to create a small amount of pressure in order to pierce the bladder dome with a syringe needle (BD microlance™, 19G 2"). A double lumen catheter, connected to both a pressure transducer (DTX™ plus DT-XX, Becton Dickinson, Singapore), to allow recordings of intravesical pressure, and an outflow (two-way) tap was then inserted into the bladder dome through the incision and secured in place with silk suture. The open or closed state of the tap allowed control over filling and emptying the bladder.

Following catheterisation, bladder nerve bundles containing both pelvic and hypogastric afferent nerves exiting the bladder were located around the base of the bladder, proximal to the point at which it connects to the urethra. The bladder nerve bundles converge with urethral bundles as they run dorsally towards the spinal cord and it is here that they are most easily identified under the microscope. Gently removing connective and fatty tissue exposed the nerve bundles which were carefully divided into individual strands. A nerve strand was selected and inserted into a suction electrode (tip diameter 25-50µm) attached to a Neurolog headstage (NL100, digitimer, Ltd, UK), which allowed the recording of multi-unit afferent nerve activity. The NL100 was connected to an AC pre-amp (NL014) allowing the amplification of the nerve signals (x10,000) before they were filtered (NL125, band pass filter) and passed through a 50/60Hz electrical noise eliminator (Humbug, Quest Scientific) and finally passed via a micro 1401 analogue to digital interface and visualised on a computer using Spike 2 software (version 7.1, Cambridge Electronic Design, UK). Mechanosensitivity is measured as the number of action potentials passing a pre-set threshold for a given pressure. The number of action potentials per one second bin is converted to a rate histogram from which total values for the number of action potentials/second can be plotted against pressure.

### Bladder contraction/relaxation experiments and the exogenous chemical stimulation of bladder muscle

The chemosensitivity of bladder afferents was also tested following isolation of pelvic afferents and catheterisation of the bladder described above. Following an equilibration period, when bladder afferent responses to ramp distension are stable, the bladder was distended to a physiological pressure of 6mmHg above baseline. At this point, the saline perfusion is stopped but the outflow tap remains closed as to maintain the volume in the bladder. The bladder is maintained in this state to accommodate the increase in volume for 45 minutes whilst the baseline intravesical pressure and afferent firing stabilise.

Following the equilibration period, the bath perfusion is briefly turned off and a bolus dose of agonist is applied. Carbachol (1µM), αβMe-ATP (30µM), and capsaicin (10µM) were applied in series with a 1hr washout period between each dose.

Chemosensitive responses were measured as the number of action potentials crossing a pre-set threshold at a given time following agonist administration. The intravesical pressure was determined as the change from baseline pressure at a given time following agonist administration.

### Retrograde tracing of bladder DRG neurons.

Laparotomy was performed on anaesthetized (2-3% isofluorane/0.3L/min O₂) mice. AlexaFluor 488 conjugated Cholera Toxin Subunit B (10ul of 5ug/ul in sterile PBS; ThermoFisher product nr. C22842 (former LifeTechnologies)) was used for subserosal injection (2-3ul per injection) in descending colon or dorsal and lateral bladder using a 30G Hamilton needle. Incision was closed with Prolene 6/0 suture (Johnson&Johnson product nr. 8636G). All mice were given 100ul of buprenorphine (1:12) and Baytril (1:20) for postoperative care and left for 4 days before humanely killed for DRG cell culture.

### Patch clamp recordings from retrogradely traced bladder DRG neurons

Recordings were made from healthy and CVH mice. CVH mice received chronic administration of either vehicle or linaclotide (3µg/kg/day) for a total duration of 2 weeks. Mice were euthanized via CO₂ inhalation and DRGs from thoracolumbar (T10-L1) and lumbosacral (L6-S1) regions removed and digested with 3mg mL-1 collagenase II (Gibco) and 4 mg mL-1 dispase (Gibco) for 30 min at 37°C, followed by 3mg mL-1 collagenase II only for 10 min at 37°C. Neurons were mechanically dissociated into a single cell suspension via trituration through fire-polished Pasteur pipettes. Neurons were resuspended in Dulbecco's modified Eagle's medium (DMEM; Gibco) containing 10% fetal calf serum (FCS; Invitrogen), 2 mM L-glutamine (Gibco), 100 uM minimum essential medium (MEM) non-essential amino acids (Gibco) and 100 mg mL-1 penicillin-streptomycin (Invitrogen). Nerve growth factor (NGF) at 100 ng mL-1 (Sigma-Aldrich) was also included. Neurons were spot-plated on 8mm HCl treated coverslips coated with poly D-lysine (800 µg mL-1) and laminin (20 µg mL-1) and maintained at 37°C in 5% CO₂ for 48 hours.

Recordings were made similarly to those we have described previously (Brierley et al., 2011, ibid). Briefly, whole-cell patch clamp recordings were made from thoracolumbar (T10-L1) or lumbosacral (L6-S1) DRG neurons, which displayed fluorescence following retrograde tracer (CTB-AF488) injection into the bladder wall. 48 h after plating, using fire-polished glass electrodes with a resistance of 5-7 MΩ. All recordings were performed at room temperature (20-22°C). Signals were amplified by using an Axopatch 200A amplifier, digitised with a Digidata 1322A and recorded using pCLAMP 9 software (Molecular Devices, Sunnyvale, CA, USA). For all neurons, baseline holding potential was - 70 mV. Intracellular solutions contained (mM): KCl, 135; MgCl₂, 2; MgATP, 2; EGTA-Na, 5; Hepes-Na, 10; adjusted to pH 7.4. Extracellular solutions contained (mM): NaCl, 140; KCl, 4; MgCl₂, 2; CaCl₂, 2; Hepes-Na, 10; glucose, 5; adjusted to pH 7.4. In current clamp studies a series of depolarising pulses (500 ms, 10 pA step) were applied from a holding potential (65 mV) and the rheobase (or amount of current (pA) required for action potential generation) determined. The number of action potentials at rheobase and 2x rheobase was also determined and the resting membrane potential was noted. These measurements were made in normal external bath solution.

Using both healthy and CVH mice, the effects of chronic colonic hypersensitivity on bladder contraction and bladder afferent pathways were determined, as well as the effects of chronic daily dosing of linaclotide (oral gavage; 3µg/kg/day) for 2 weeks. For these studies, individually housed mice randomly assigned to each experimental group were used.

### Ex-vivo afferent recordings

Data are expressed as mean with the SEM, and *n* is the number of animals. Data were analysed using unpaired t-test to determine significance when comparing single points or a two-way analysis of variance (ANOVA) when comparing between experimental groups throughout a ramp distension or exogenous agonist protocol. Differences were considered significant at *P*<0.05. Mechanosensory responses represent action potential discharge displayed as spikes/second at a given pressure. Muscle compliance is calculated by plotting intravesical pressure against intravesical volume, which is in turn determined from the time taken to distend to a particular pressure. Chemosensory responses represent action potential discharge displayed as spikes/second and intravesical pressure at 20 second intervals following agonist application and are plotted for the duration of the response.

Whole nerve multifiber afferent nerve activity was quantified using the spike 2 software, which counted the number of action potentials crossing a pre-set threshold. Data are expressed as mean ± SEM. n indicates the number of individual animals. Data were analysed using Prism 5 software (GraphPad Software, San Diego, CA, USA) where appropriate, un-paired t-tests were used, or two-way analysis of variance (ANOVA) followed by *posthoc* tests. Differences were considered significant at a level of * *P*<0.05, ** *P*<0.01 and *** *P*<0.001.

### Patch clamp recordings

Action potentials were recorded in current clamp mode and the rheobase, or the amount of injected current (pA) required to elicit an action potential determined. The numbers of action potentials at rheobase and 2x rheobase were also recorded as well as noting the resting membrane potential. Data were analysed using Prism 5 software (GraphPad Software, San Diego, CA, USA) where appropriate, un-paired t-tests or one-way analysis of variance (ANOVA) followed by *posthoc* tests. Differences were considered significant at a level of * *P*<0.05, ** *P*<0.01 and *** *P*<0.001.

CVH mice show significantly increased bladder afferent responses to ramp bladder distension protocols compared to healthy controls. There were no changes in bladder compliance when comparing bladder responses from CVH mice and healthy CVH mice also show significantly increased bladder afferent responses to carbachol, αβMe-ATP and capsaicin, but no associated change in muscle contraction. Bladder traced TL and LS DRG neurons from CVH mice show a significantly reduced rheobase (amount of current required to fire an action potential) compared to healthy controls.

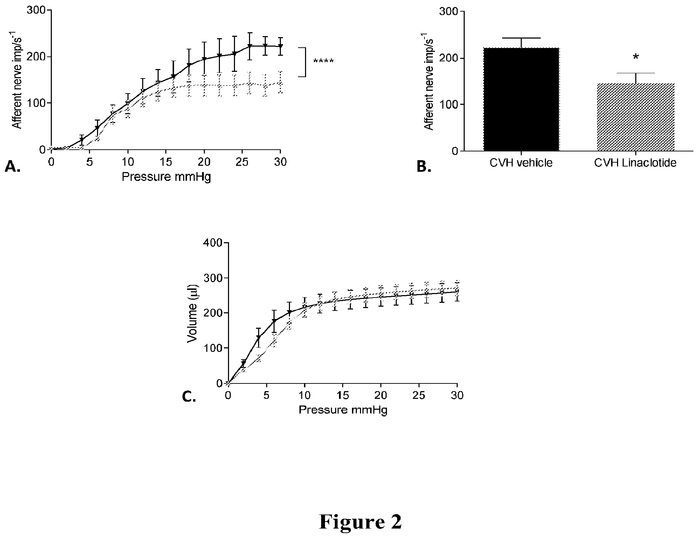

Chronic daily dosing of linaclotide for 2 weeks significantly reduced bladder afferent responses to ramp distension in CVH mice compared to vehicle controls. In CVH mice administered once daily oral gavage of linaclotide for two weeks, there was a significant decrease in afferent nerve activity to both the time course of ramp distension (Fig. 2A, P≤0.001) and peak afferent response to distension (Fig. 2B, P≤0.05) compared with mice administered once daily oral gavage of vehicle for two weeks. The effects of linaclotide were more apparent at higher distension pressures (Fig. 2A). There was no change in compliance between vehicle and linaclotide groups (Fig. 2C).

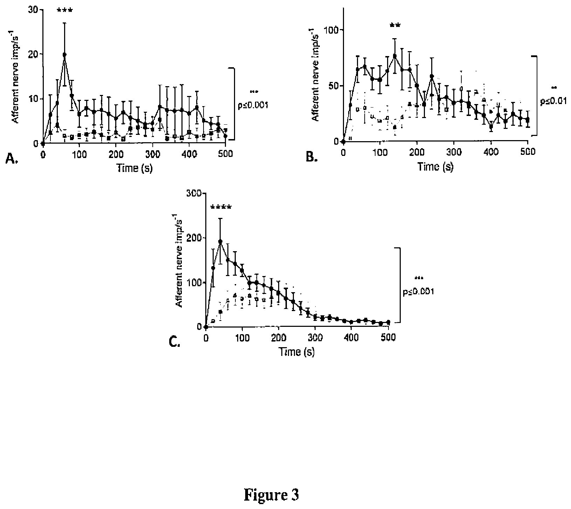

Chronic daily dosing of linaclotide for 2 weeks also significantly reduced afferent and contraction responses to carbachol and αβMe-ATP, and afferent responses only to capsaicin. Figure 3 shows bladder afferent responses to: A, carbachol (1µM) p≤0.001, B, capsaicin (10µM) p ≤0.01, C, αβMe-ATP (30µM) p≤0.001 in CVH mice with once daily oral gavage of vehicle (black, n=7) or linaclotide (grey, n=7) (2-way ANOVA Bonferroni multiple comparisons test).

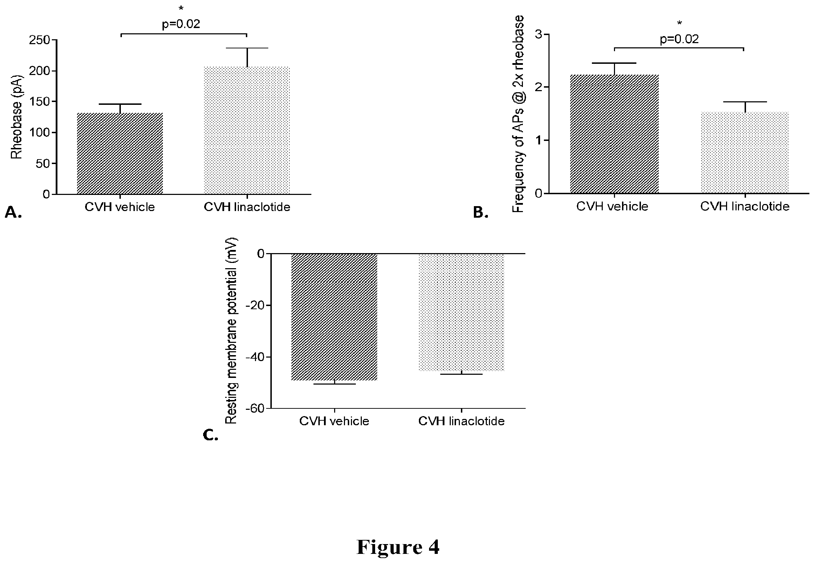

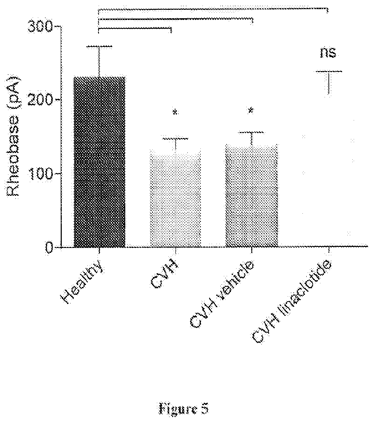

Chronic daily dosing of linaclotide for 2 weeks reversed the reduced rheobase of bladder sensory neurons observed in CVH mice. Figure 4 shows electrophysiological properties of retrogradely traced bladder thoracolumbar (TL) DRG neurons from CVH mice with once daily oral gavage of vehicle (n=27) or linaclotide (n=25). A, linaclotide significantly increases the rheobase compared to vehicle group (p=0.02) B, linaclotide significantly decreases the number of action potentials at 2X rheobase compared to vehicle group (p=0.02) C, linaclotide has no effect on resting membrane potential compared to mice receiving vehicle gavage. Figure 5 shows rheobase of retrogradely traced bladder thoracolumbar (TL) DRG neurons from healthy control mice (n=19), CVH mice (n=22), and CVH mice with once daily oral gavage of vehicle (n=27) or linaclotide (n=25). Rheobase is significantly reduced in CVH mice and this effect is reversed by oral gavage of linaclotide (p≤0.05, one-way ANOVA).

TNBS colitis induces considerable MPO activity in the colon as well as extensive damage to the colonic mucosa, oedema and mast cell infiltration at both day 3 and day 7 post TNBS compared with healthy controls. In contrast, no observable changes in MPO activity nor mucosal damage occurred in the bladder at these time points.

Induction of a CVH state via intra-colonic administration of TNBS results in significant increases in bladder afferent firing in response to mechanical stretch and chemical stimulation and decreases in the rheobase of DRG neurons innervating the bladder. Notably, chronic oral administration of linaclotide (3µg/kg/day) for 2 weeks is able to normalize these effects.

Oral treatment with linaclotide (3µg/kg/day) for 2 weeks is able to significantly attenuate TNBS-induced bladder hyperactivity in CVH mice by reducing the number of action potentials in bladder afferents arising from bladder distension and exogenous purinergic, muscarinic and TRPV1 activation. In addition, linaclotide treatment is able to reverse the reductions in rheobase observed in retrogradely traced bladder DRG neurons from CVH mice.

### Example 17

### Efficacy in Clinical Trial

The primary purpose of this study is to compare the efficacy of IR and DR formulations of linaclotide in the treatment of overactive bladder symptoms, compared with placebo.

### Inclusion Criteria

The subject patient is willing and able to complete the micturition diary and questionnaires correctly. The subject has symptoms of overactive bladder (urinary frequency and urgency with or without urge incontinence) for ≥ 3 months. The subject experiences frequency of micturition on average ≥ 8 times per 24-hour period during the 3-day micturition diary period. The subject must experience at least 3 episodes of urgency (grade 3 or 4) with or without incontinence, during the 3-day micturition diary period.

### Exclusion Criteria

The subject is breastfeeding, pregnant, intends to become pregnant during the study, or of childbearing potential, sexually active and not practicing a highly reliable method of birth control. The subject has significant stress incontinence or mixed stress/urge incontinence where stress is the predominant factor. The subject has an indwelling catheter or practices intermittent self-catheterization. The Subject has diabetic neuropathy. The subject has evidence of a symptomatic urinary tract infection, chronic inflammation such as interstitial cystitis, bladder stones, previous pelvic radiation therapy or previous or current malignant disease of the pelvic organs. The subject receives non-drug treatment including electro-stimulation therapy. The subject has severe hypertension. The subject has a known or suspected hypersensitivity to tolterodine, other anticholinergics, YM178, other beta-adrenoreceptor (β-AR) agonists, or lactose or any of the other inactive ingredients. The subject has been treated with any investigational drug or device within 30 days (90 days in the UK). The subject had an average total daily urine volume > 3000 mL as recorded in the 3-day micturition diary period. The subject has serum creatinine >150 umol/L, aspartate aminotransferase (AST) and/or alanine aminotransferase (ALT) > 2x upper limit of normal range (ULN), or gamma glutamyl transferase (γ-GT) > 3x ULN. The subject has a clinically significant abnormal electrocardiogram (ECG).

Subjects meeting the inclusion criteria listed above and not having any of the exclusion criteria are selected. Subjects are randomized and placed in three treatment groups: placebo, linaclotide IR, or linaclotide DR. The dose of linaclotide IR is 290 µg, given orally once per day. The dose of linaclotide DR is 300 µg, also given orally once per day. Treatment is for a twelve week period.

A primary efficacy parameter is the change from baseline to end of treatment (final visit) in mean number of incontinence episodes per 24 hours. The average number of incontinence episodes (any involuntary leakage of urine) per day is derived from the number of incontinence episodes recorded by the patient in a micturition diary for 3-days before the Baseline and Week 12 clinic visits. Least Squares (LS) Means are generated from an analysis of covariance (ANCOVA) model with treatment group, gender, and geographic region as fixed factors and baseline as a covariate.

Another primary efficacy parameter is the change from baseline to end of treatment (final visit) in mean number of micturitions per 24 hours. The average number of micturitions (urinations) per 24 hours is derived from the number of times a patient urinates (excluding incontinence only episodes) per day recorded by the patient in a micturition diary for 3-days before the Baseline and Week 12 clinic visits. LS means are generated from an ANCOVA model with treatment group, gender, and geographic region as fixed factors and baseline as a covariate.

Secondary outcome measures can include one or more of the following.
- Change From Baseline to Final Visit in Mean Volume Voided Per Micturition, calculated from the volume of each micturition measured by the patient and recorded in a micturition diary for 3 days before the Baseline and Week 12 clinic visits.
- Change From Baseline to Week 4 in Mean Number of Incontinence Episodes Per 24 Hours, derived from the number of incontinence episodes recorded by the patient in a micturition diary for 3-days before the Baseline and Week 4 clinic visits.
- Change From Baseline to Week 4 in Mean Number of Micturitions Per 24 Hours, calculated from the number of micturitions recorded by the patient in a micturition diary for 3-days before the Baseline and Week 4 clinic visits.
- Change From Baseline to Week 8 and Week 12 in Mean Number of Incontinence Episodes Per 24 Hours, derived from the number of incontinence episodes recorded by the patient in a micturition diary for 3-days before the Baseline, Week 8 and Week 12 clinic visits.
- Change From Baseline to Week 8 and Week 12 in Mean Number of Micturitions Per 24 Hours, calculated from the number of micturitions recorded by the patient in a micturition diary for 3-days before the Baseline, Week 8 and 12 clinic visits.
- Change From Baseline to Week 4, Week 8 and Week 12 in Mean Volume Voided Per Micturition, calculated from the volume of each micturition measured by the patient and recorded in a micturition diary for 3 days before the Baseline and Week 4, 8 and 12 clinic visits.
- Change From Baseline to Week 4, Week 8, Week 12 and Final Visit in Mean Number of Urgency Incontinence Episodes Per 24 Hours, derived from the number of incontinence episodes classified by the patient in a 3-day micturition diary as 3 or 4 on the Patient Perception of Intensity of Urgency Scale: 0 = No urgency; 1 = Mild urgency; 2 = Moderate urgency, could postpone voiding a short while; 3 = Severe urgency, could not postpone voiding; 4 = Urge incontinence, leaked before arriving to the toilet.
- Change From Baseline to Week 4, Week 8, Week 12 and Final Visit in Mean Number of Urgency Episodes (Grades 3 or 4) Per 24 Hours, derived from urgency episodes classified by the patient in a 3-day micturition diary as grade 3 or 4 on the Patient Perception of Intensity of Urgency Scale: 0: No urgency; 1: Mild urgency; 2: Moderate urgency, could delay voiding a short while; 3: Severe urgency, could not delay voiding; 4: Urge incontinence, leaked before arriving to the toilet.
- Change From Baseline to Week 4, Week 8, Week 12 and Final Visit in Mean Level of Urgency, based on average of patients' ratings on the degree of urgency associated with each micturition and/or incontinence episode recorded in a 3-day micturition diary according to the following 5-point categorical scale (Patient Perception of Intensity of Urgency Scale): 0: No urgency; 1: Mild urgency; 2: Moderate urgency, could delay voiding a short while; 3: Severe urgency, could not delay voiding; 4: Urge incontinence, leaked before arriving to the toilet.
- Change From Baseline to Week 4, Week 8, Week 12 and Final Visit in Mean Number of Nocturia Episodes Per 24 Hours; nocturia is defined as waking at night one or more times to void. The average number of times a patient urinated (excluding incontinence only episodes) during sleeping time per day was derived from the 3-day patient micturition diary.
- Change From Baseline to Week 4, Week 8, Week 12 and Final Visit in Mean Number of Pads Used Per 24 Hours During The 3-Day Micturition Diary Period.
- Percentage of Participants With Zero Incontinence Episodes at Week 4, Week 8, Week 12 and the Final Visit for the 3 days prior to each clinic visit derived from the micturition diary recorded by the patient.
- Percentage of Participants With ≥ 50% Reduction in Incontinence Episodes at Weeks 4, 8, 12 and the Final Visit (the percentage of participants with at least 50% decrease from baseline in mean number of incontinence episodes per 24 hours during the 3 days prior to each clinic visit derived from the patient micturition diary).
- Change From Baseline to Week 4, Week 8, Week 12 and Final Visit in Symptom Bother Score (overactive bladder symptoms are assessed using the symptom bother scale of the overactive bladder questionnaire. The symptom bother scale consists of 8 questions answered by the participant on a scale from 1-6. The total symptom bother score is calculated from the 8 answers and then transformed to range from 0 to 100, with 100 indicating worst severity. A negative change from Baseline in symptom bother score indicates improvements).
- Change From Baseline to Week 4, Week 8, Week 12 and Final Visit in Health-related Quality of Life (HRQL) Total Score, assessed by the HRQL subscales (coping, concern, sleep and social interaction) of the overactive bladder questionnaire (OABq). The HRQL total score was calculated by adding the 4 HRQL subscale scores, and transforming to a scale from 0 to 100, with higher scores indicating better quality of life. A positive change from Baseline in HRQL score indicates improvements.
- Change From Baseline to Week 12 and Final Visit in Work Productivity and Activity Impairment (WPAI): Percent Work Time Missed. The WPAI:SHP questionnaire is used to assess the degree and extent to which overactive bladder (OAB) symptoms interfered with work productivity in the last 7 days. Percent of work time missed is derived from the number of hours of work missed due to OAB symptoms as a percentage of total hours that should have been worked. A higher percentage indicates more hours missed. A negative change from baseline indicates improvement.
- Change From Baseline to Week 12 and Final Visit in Work Productivity and Activity Impairment (WPAI): Percent Impairment While Working
- Change From Baseline to Week 12 and Final Visit in Work Productivity and Activity Impairment (WPAI): Percent Overall Work Impairment
- Change From Baseline to Week 12 and Final Visit in Work Productivity and Activity Impairment (WPAI): Percent Activity Impairment
- Change From Baseline to Final Visit in European Quality of Life-5 Dimensions (EQ-5D) Mobility Score. The EQ-5D is an international, standardized, nondisease-specific (i.e., generic) instrument for describing and valuing health status. Participants are asked to indicate which of the following statements best describes their health state:
- Change From Baseline to Final Visit in European Quality of Life-5 Dimensions (EQ-5D) Self-care Score
- Change From Baseline to Final Visit in European Quality of Life-5 Dimensions (EQ-5D) Usual Activities Score
- Change From Baseline to Final Visit in European Quality of Life-5 Dimensions (EQ-5D) Pain/Discomfort Score
- Change From Baseline to Final Visit in European Quality of Life-5 Dimensions (EQ-5D)Anxiety/Depression Score
- Change From Baseline to Week 4, Week 8, Week 12 and Final Visit in the European Quality of Life-5 Dimensions (EQ-5D) Visual Analog Scale (VAS)
- Change From Baseline to Week 12 and Final Visit in Patient Perception of Bladder Condition (PPBC)
- Change From Baseline to Week 12 and Final Visit in Treatment Satisfaction on Visual Analog Scale (TS-VAS). The TS-VAS is a visual analog scale (VAS) that asks patients to rate their satisfaction with treatment by placing a vertical mark on a 10 cm line where the endpoints are labeled 'No, not at all' on the left (=0) to 'Yes, completely satisfied' on the right (=10). LS means are from an ANCOVA model with treatment group, gender, and geographical regions as fixed factors and baseline as a covariate. A positive change from baseline indicates improvement.
- Change From Baseline to Week 4, Week 8, Week 12 and Final Visit in Number of Non-study Related Visits to Physician (the number of times the patient visited a physician's office during the 4 weeks prior to each study visit (excluding study visits) because of the patient's bladder condition).
- Percentage of Participants With Improvement in Patient Perception of Bladder Condition (PPBC) at Week 12 and Final Visit. The PPBC scale is a global assessment tool that asks patients to rate their impression of their current bladder condition on a 6-point scale from 1: 'Does not cause me any problems at all'; 2: 'Causes me some very minor problems'; 3: 'Causes me some minor problems'; 4: 'Causes me (some) moderate problems'; 5: 'Causes me severe problems' and 6: 'Causes me many severe problems'. Improvement was defined as at least a 1 point improvement from Baseline to post-baseline and a major improvement was defined as at least a 2 point improvement from Baseline to post-baseline in PPBC score.

### Example 18

### Alternative Linaclotide Tablet Preparation

Delayed release tablets were prepared by first preparing the following core tablet components: a placebo base, a linaclotide 1000 µg/225 mg base, and pre-granulated fillers.

### Granulation manufacturing process:

The tablet components were prepared essentially as described above in Example 7, but with slight modifications as described below. The placebo base as described in Table 28 was used to provide core tablets with the components listed in Table 27. A final pH of 2.25 was used for placebo and active base granulations.

**Table 27: Components for various tablet strengths (the 25 µg-dose is not according to the invention)**

| Strength | Placebo | 25 µg | 30µg | 50 µg | 75 µg | 100 µg | 150 µg | 290 µg | 300µg |
|---|---|---|---|---|---|---|---|---|---|
| Placebo base (%) | 30 | 27.5 | 27 | 25 | 22.5 | 20 | 15 | 1 | 0 |
| Linaclotide base (i.e., 1000 ug/225 mg base (%)) | 0 | 2.5 | 3 | 5 | 7.5 | 10 | 15 | 29 | 30 |
| Pregranulated fillers (%) | 68.75 | 68.75 | 68.75 | 68.75 | 68.75 | 68.75 | 68.75 | 68.75 | 68.75 |
| Magnesium Stearate (%) | 1.25 | 1.25 | 1.25 | 1.25 | 1.25 | 1.25 | 1.25 | 1.25 | 1.25 |
| Total (%) | 100 | 100 | 100 | 100 | 100 | 100 | 100 | 100 | 100 |

### Placebo Base Preparation:

Table 28 represents the formulation for the placebo base granulation:

**Table 28: Formulation of the placebo base granulation**

| Component | %w/w | Quantity (g) |
|---|---|---|
| L-Histidine | 4.97 | 248.5 |
| Calcium Chloride Dihydrate | 2.35 | 117.5 |
| polyvinyl alcohol | 3.96 | 198 |
| microcrystalline cellulose | 88.72 | 4436 |
| Hydrochloric acid, pure, fuming, 37% solution in water | | |
| Treated water | | |
| Total | 100 | 5000 |

The placebo base preparation was prepared by first dispensing the raw materials of Table 29.

**Table 29: Raw materials for placebo base preparation**

| Component | Quantity (g) |
|---|---|
| L-Histidine | 248.4 |
| Calcium Chloride Dihydrate | 117.7 |
| polyvinyl alcohol | 198 |
| microcrystalline cellulose | 4435.9 |

The mix container is tared and 2200 ± 5.0 g of treated water (rather than 2682.1 ± 5.0 g) is added into the container.

### Linaclotide Base Preparation (1000µg/225mg):

Table 30 represents the formulation for the 1000µg/225mg base granulation:

**Table 30: Formulation for the 1000µg/225mg base granulation**

| Component | % w/w | Quantity (g) |
|---|---|---|
| Linaclotide | 0.444 | 23 |
| L-Histidine | 4.97 | 248.4 |
| Calcium Chloride Dihydrate | 2.35 | 117.7 |
| polyvinyl alcohol | 3.96 | 198 |
| microcrystalline cellulose | 88.27 | 4413.7 |

| | | |
|---|---|---|
| A balance of 0.02% represents Linaclotide impurities. | | |

The 1000µg/225mg base granulation may be prepared by first dispensing the raw materials of Table 31.

**Table 31: Raw materials of the linaclotide base granulation**

| Raw Material | Required Quantity (g) |
|---|---|
| Linaclotide | 23 |
| microcrystalline cellulose | 4413.7 |
| Granulation solution | |

### Pregranulated Filler Preparation:

Table 32 represents the formulation for the pregranulated fillers.

**Table 32: Formulation of the fillers granulation**

| Component | % w/w | Qty per sublot (g) |
|---|---|---|
| microcrystalline cellulose | 14.6 | 1018 |
| croscarmellose sodium | 2.9 | 203.6 |
| mannitol | 79.1 | 5533.2 |
| polyvinyl alcohol | 3.5 | 245 |
| Treated water | ----- | ----- |
| Total | 100.0 | 7.000 |

The fillers preparation are prepared by first dispensing the raw materials of Table 33.

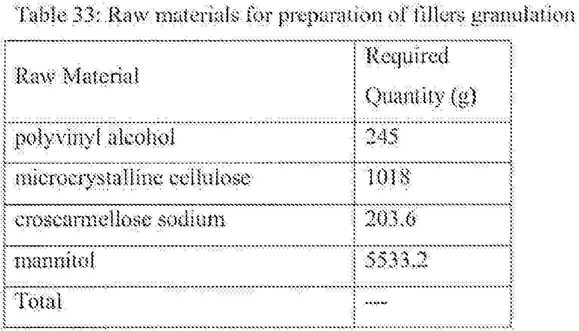

### Example 19

### Effect of Altering Tablet Composition on Stability and Impurities

In order to determine the influence of various components on the stability of linaclotide, a number of different formulations containing different amounts of PVA, CaCl2, Histidine and CCS were prepared and tested for loss of drug substance and/or appearance of degradants. The formulations were prepared as shown in Table 34.

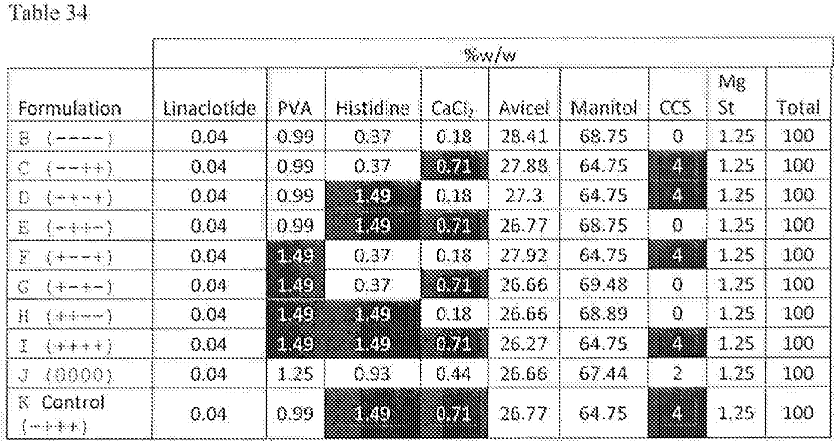

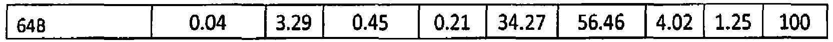

As shown above, formulations contained high or low concentrations of PVA (1.49 or 0.99% w/w), Histidine (1.49 or 0.37% w/w), CaCl₂ (0.71 or 0.18% w/w) or CCS (4 or 0% w/w).

The uncoated core tablets of the formulations described above were packaged in 60cc white HDPE bottles with 3g silica gel dessicant, heat sealed with inner foil liner and closed with 33mm white polypropylene child-resistant caps. Sealed bottles were placed in chambers at 40°C at 75% relative humidity (RH). Samples were taken at different time intervals and quantitated for the amount of linaclotide, as well as for the ketone and immidazolidinone degradative products, which are described, for example, in US 8,933,030. Linaclotide and the degradative products were quantitated using HPLC as outlined below in Tables 35 and 36, respectively and compared with T = 0 samples (unstressed controls).

**Table 35.**

| **DR Tablets Assay Method LC/2015014/AS-02** | | | |
|---|---|---|---|
| Column | YMC Pack Pro C18, 3x150mm, 3um, YMC AS-12S03-1503WT | | |
| Mobile phase | A: Water: ACN : TFA = 98 : 2 : 0.1 (v/v) | | |
| | B: Water: ACN : TFA = 5 : 95 : 0.1 (v/v) | | |
| Flow rate | 0.6 mL/min | | |
| Column temperature | 40°C | | |
| Auto sampler temperature | 4°C | | |
| Detector | UV 220 nm | | |
| Standard concentration | 5 ug/mL | | |
| Sample concentration | 1.5 ug/mL (30ug tab), 2.5 ug/mL (100ug tab), 5 ug/mL (300ug tab) | | |
| Injection | 100 uL | | |
| Run time | 15 min | | |

| Gradient | Time(min) | %B | Curve |
|---|---|---|---|
| | 0.0 | 18 | - |
| | 5.0 | 25 | 6 |
| | 5.01 | 100 | 1 |
| | 8.00 | 100 | 6 |
| | 8.01 | 18 | 1 |
| | 15.0 | 18 | 6 |
| Sample Preparation (duplicate preps): 5 DR tablets washed with 20 mL MeOH 1min; 20 mL ACN 30 sec, twice; then extracted with 0.1 N HCl on a shaker for 2hr; filter with 0.45 um PVDF syringe filters, discard 1st 2mL. | | | |

**Table 36.**

| **DR Tablets Purity Method** | | |
|---|---|---|
| | Column: YMC Pro C18, 150 mm x 3.0 mm ID, 3 µm, | |
| Column | Guard column: YMC Guard Cartridge, ProC18, 20 x 4.0 mm I.D., 3µm | |
| Mobile phase | Mobile Phase A: Water:Acetonitrile:TFA (98:2:0.1) | |
| | Mobile Phase B: Acetonitrile:Water:TFA (95:5:0.1) | |
| Flow rate | 0.6 mL/min | |
| Column temperature | 40°C | |
| Auto sampler temperature | 4°C | |
| Detector | UV 220 nm | |
| Sample concentration | 25 ug/mL | |
| Injection | 400 uL | |
| Run time | 69 min | |

| Gradient | Time(min) | %B |
|---|---|---|
| | 0 | 0 |
| | 4 | 0 |
| | 9 | 10 |
| | 43 | 23 |
| | 49 | 34 |
| | 59 | 80 |
| | 59.1 | 100 |
| | 62 | 100 |
| | 62.1 | 0 |
| | 69 | 0 |
| Sample Preparation: 5 DR tablets washed with 20 mL MeOH 1min; 20 mL ACN 30 sec, twice; then extracted with 0.1 N HCl on a shaker for 1hr; filter with 0.2 um PVDF syringe filters | | |

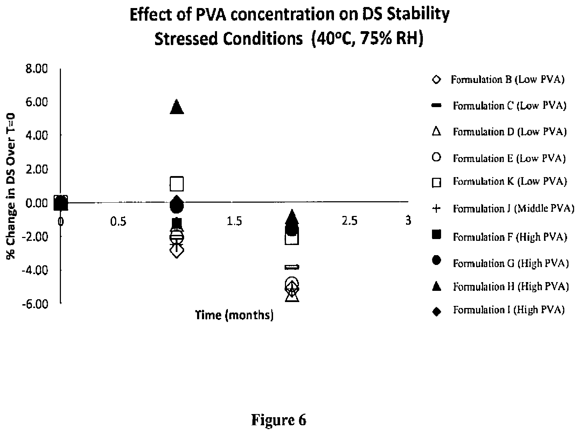

As shown in Figure 6, tablets containing lower concentrations [0.99% (w/w), formulations B, C, D, E, K] of PVA showed loss of linaclotide in samples placed under stressed conditions for 1 or 2 months, whereas tablets with higher concentrations [1.25% (w/w) - Formulation J, or 1.49% (w/w) - Formulations F, G, H, I] of PVA showed less reduction.

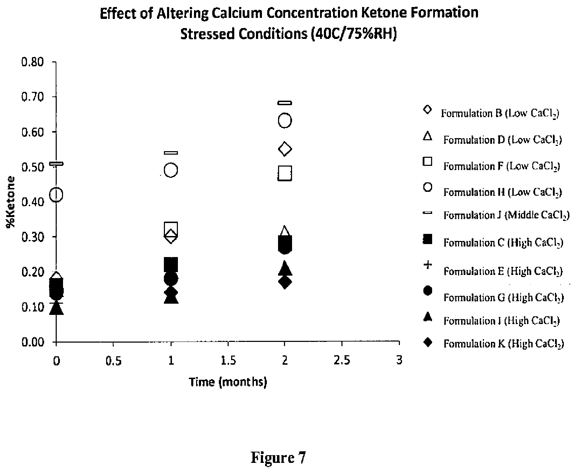

Similarly, certain formulations were found to result in an increase in ketone formation. As shown in Figure 7, tablets containing low levels [0.18% (w/w), formulations B, D, F, H] or moderate levels [0.44, formulation J] of CaCl₂ were shown to result in an increase in ketone degradative products in samples placed in stressed conditions for one or two months. In contrast, samples containing 0.71% (w/w) of CaCl₂ showed a decrease in ketone formation.

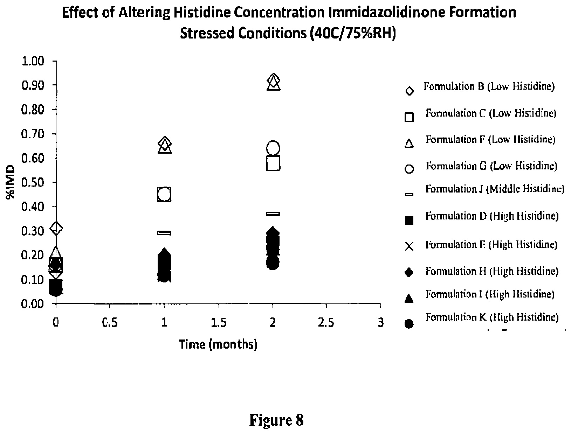

Histidine concentration was found to influence the amount of immidazolidinone formation in tablets (Figure 8), where low concentration of histidine (Formulations B, C, F, & G) resulted in higher levels of this degradative product, whereas tablets with moderate [0.93% (w/w), formulation J] or high [1.49% (w/w), formulations D, E, H, I & K] levels of histidine resulted in decreased formation of this degradative product.

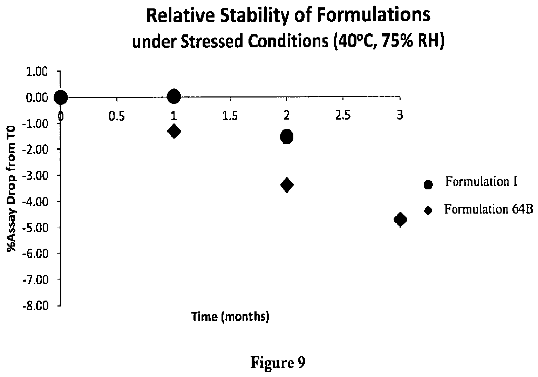

Finally, the formulation I, which is also described in Example 17, was compared with earlier formulations for linaclotide stability. Figure 9 compares stability of linaclotide in Formulation I compared with Formulation 64B.

### OTHER EMBODIMENTS

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims. It is further to be understood that all values are approximate, and are provided for description.

## Claims

1. A delayed release composition comprising an enteric coated tablet, wherein the tablet comprises:
linaclotide;
Ca²⁺;
histidine; and
polyvinyl alcohol (PVA),
wherein the linaclotide is present in the composition in an amount between 30 µg and 300 µg.

2. The composition of claim 1, wherein the linaclotide is present in an amount of 30 µg, 100 µg or 300 µg.

3. The composition of claim 1 or claim 2, wherein the enteric coating comprises methyl acrylate-methacrylic acid copolymers (e.g. Eudragit®).

4. The composition of any one of claims 1-3, wherein the enteric coating comprises Eudragit® S100, Eudragit®L100, or Eudragit® S100/Eudragit®L100 mixture.

5. The composition of any one of claims 1-4, further comprising a protective polymer film or subcoating.

6. The composition of claim 5, wherein the subcoating comprises Opadry II®.

7. A unit dosage form comprising the pharmaceutical composition of claim 1.

8. A method of making the composition of any one of claims 1-6, comprising:
i) preparing a linaclotide base, pregranulated filler, and placebo base;
ii) blending and compressing the linaclotide base, pregranulated filler, and placebo base into a tablet; and
iii) applying an enteric coating to the tablet.

9. The method of claim 8, wherein the pregranulated filler is prepared through wet granulation and dried before blending and compressing into a tablet.

10. The method of claim 8 or claim 9, wherein the method further comprises applying a subcoat to the tablet.

11. The method of claim 10, wherein the subcoat comprises Opadry II®.

12. The method of any of claims 8-11, wherein the enteric coating comprises Eudragit® S100, Eudragit®L100, or Eudragit® S100/Eudragit®L100 mixture.

13. A composition prepared by the method of any one of claims 8-12.

14. A composition of any of claims 1-6 or 13 for use in a method of treating a gastrointestinal disorder in a patient.

15. The composition for use of claim 14, wherein the gastrointestinal disorder is selected from the group consisting of: irritable bowel syndrome (IBS), constipation, a functional gastrointestinal disorder, gastroesophageal reflux disease, functional heartburn, dyspepsia, diverticulitis, visceral pain, abdominal pain, gastroparesis, chronic intestinal pseudo-obstruction, colonic pseudo-obstruction, Crohn's disease, ulcerative colitis, and inflammatory bowel disease.

16. The composition for use of claim 14, wherein the gastrointestinal disorder is constipation.

17. The composition for use of claim 16, wherein the constipation is chronic constipation, idiopathic constipation, chronic idiopathic constipation, constipation due to post-operative ileus, or constipation caused by opiate use.

18. The composition for use of claim 14, wherein the gastrointestinal disorder is irritable bowel syndrome (IBS).

19. The composition for use of claim 18, wherein the irritable bowel syndrome is constipation-predominant irritable bowel syndrome (IBS-c), diarrhea-predominant irritable bowel syndrome (IBS-d) or mixed irritable bowel syndromes (IBS-m).

20. The composition for use of claim 14, wherein the gastrointestinal disorder is visceral pain.

21. The composition for use of claim 14, wherein the gastrointestinal disorder is abdominal pain.

22. A composition of any of claims 1-6 or 13 for use in a method of treating or relieving pain in a patient, wherein the pain is selected from visceral pain; diverticulitis pain; pelvic pain; abdominal pain; or pain associated with gastrointestinal disorders, venereal diseases, bladder pain syndrome, or interstitial cystitis; or wherein the pain is selected from pain associated with irritable bowel syndrome (IBS), chronic or acute radiation proctopathy (also referred to as radiation proctitis), rectal pain, chronic proctalgia, proctalgia fugax, anal pain, chronic anal fissure, post-operative anal pain, overactive bladder syndrome, stress incontinence, pain associated with cancer, pain associated with gastrointestinal tract neoplasms, endometriosis, orchialgia, chronic prostatitis, prostatodynia, vulvodynia, urethral syndrome, penile pain, perianal pain, and pain associated with ulcerative colitis, ulcerative proctitis, or Crohn's disease.

23. A composition of any of claims 1-6 or 13 for use in a method of treating overactive bladder syndrome, bladder hypersensitivity or colitis induced bladder afferent hyperactivity in a patient.

## Patentansprüche

1. Zusammensetzung mit verzögerter Freisetzung, umfassend eine magensaftresistente Tablette, wobei die Tablette Folgendes umfasst:
Linaclotid;
Ca²⁺;
Histidin; und
Polyvinylalkohol (PVA),
wobei das Linaclotid in der Zusammensetzung in einer Menge zwischen 30 µg und 300 µg vorliegt.

2. Zusammensetzung nach Anspruch 1, wobei das Linaclotid in einer Menge von 30 µg, 100 µg oder 300 µg vorliegt.

3. Zusammensetzung nach Anspruch 1 oder Anspruch 2, wobei der magensaftresistente Überzug Methylacrylat-Methacrylsäure-Copolymere (z. B. Eudragit®) umfasst.

4. Zusammensetzung nach einem der Ansprüche 1 bis 3, wobei der magensaftresistente Überzug Eudragit® S100, Eudragit® L100 oder ein Eudragit® S100/Eudragit® L100-Gemisch umfasst.

5. Zusammensetzung nach einem der Ansprüche 1 bis 4, weiter umfassend einen protektiven Polymerfilm oder einen Basisüberzug (Subcoating).

6. Zusammensetzung nach Anspruch 5, wobei der Basisüberzug Opadry II® umfasst.

7. Einzeldarreichungsform, umfassend die pharmazeutische Zusammensetzung nach Anspruch 1.

8. Verfahren zur Herstellung der Zusammensetzung nach einem der Ansprüche 1 bis 6, umfassend:
(i) Zubereiten einer Linaclotid-Basis, eines vorgranulierten Füllstoffs und einer Placebo-Basis;
(ii) Mischen und Verpressen der Linaclotid-Basis, des vorgranulierten Füllstoffs und der Placebo-Basis zu einer Tablette; und
(iii) Auftragen eines magensaftresistenten Überzugs auf die Tablette.

9. Verfahren nach Anspruch 8, wobei der vorgranulierte Füllstoff mittels Feuchtgranulierung und Trocknen vor dem Mischen und Verpressen zu einer Tablette zubereitet wird.

10. Verfahren nach Anspruch 8 oder Anspruch 9, wobei das Verfahren weiter das Auftragen eines Basisüberzugs auf die Tablette umfasst.

11. Verfahren nach Anspruch 10, wobei der Basisüberzug Opadry II® umfasst.

12. Verfahren nach einem der Ansprüche 8 bis 11, wobei der magensaftresistente Überzug Eudragit® S100, Eudragit® L100 oder ein Eudragit® S100/Eudragit® L100-Gemisch umfasst.

13. Zusammensetzung, zubereitet mittels des Verfahrens nach einem der Ansprüche 8 bis 12.

14. Zusammensetzung nach einem der Ansprüche 1 bis 6 oder 13 zur Verwendung in einem Verfahren zur Behandlung einer gastrointestinalen Störung bei einem Patienten.

15. Zusammensetzung zur Verwendung nach Anspruch 14, wobei die gastrointestinale Störung aus der Gruppe ausgewählt ist, bestehend aus: dem Reizdarmsyndrom (IBS), Obstipation, einer gastrointestinalen Funktionsstörung, der gastroösophagealen Refluxkrankheit, funktionellem Sodbrennen, Dyspepsie, Divertikulitis, einem viszeralen Schmerz, einem abdominellen Schmerz, einer Gastroparese, einer chronischen intestinalen Pseudoobstruktion, einer kolonischen Pseudoobstruktion, Morbus Crohn, Colitis ulcerosa und einer entzündlichen Darmerkrankung.

16. Zusammensetzung zur Verwendung nach Anspruch 14, wobei die gastrointestinale Störung eine Obstipation ist.

17. Zusammensetzung zur Verwendung nach Anspruch 16, wobei die Obstipation eine chronische Obstipation, idiopathische Obstipation, idiopathische chronische Obstipation, durch einen postoperativen Ileus bedingte Obstipation oder eine durch Opiatanwendung verursachte Obstipation ist.

18. Zusammensetzung zur Verwendung nach Anspruch 14, wobei die gastrointestinale Störung das Reizdarmsyndrom (IBS) ist.

19. Zusammensetzung zur Verwendung nach Anspruch 18, wobei das Reizdarmsyndrom ein Reizdarmsyndrom vorwiegend mit Obstipation (IBS-C), ein Reizdarmsyndrom vorwiegend mit Durchfall (IBS-D) oder ein Reizdarmsyndrom des gemischten Typs (IBS-M) ist.

20. Zusammensetzung zur Verwendung nach Anspruch 14, wobei die gastrointestinale Störung ein viszeraler Schmerz ist.

21. Zusammensetzung zur Verwendung nach Anspruch 14, wobei die gastrointestinale Störung ein abdomineller Schmerz ist.

22. Zusammensetzung nach einem der Ansprüche 1 bis 6 oder 13 zur Verwendung in einem Verfahren zur Behandlung oder Linderung von Schmerzen bei einem Patienten, wobei der Schmerz ausgewählt ist aus einem viszeralen Schmerz; einem Divertikulitisschmerz; einem pelvinen Schmerz; einem abdominellen Schmerz; oder einem durch gastrointestinale Störungen bedingten Schmerz, venerischen Erkrankungen, dem Blasenschmerzsyndrom oder einer interstitiellen Cystitis; oder wobei der Schmerz ausgewählt ist aus durch das Reizdarmsyndrom (IBS) bedingte Schmerzen, einer chronischen oder akuten Strahlenproktopathie (auch als Strahlenproktitis bezeichnet), rektalen Schmerzen, chronischer Proktalgie, Proktalgie fugax, Analschmerzen, einer chronischen Analfissur, postoperativen Analschmerzen, dem überaktiven Blasensyndrom, Stressinkontinenz, krebsbedingten Schmerzen, mit Neoplasien des Gastrointestinaltrakts assoziierten Schmerzen, Endometriose, Orchialgie, chronischer Prostatitis, Prostatodynie, Vulvodynie, dem Urethralsyndrom, penilen Schmerzen, perianalen Schmerzen und durch Colitis ulcerosa bedingte Schmerzen, ulzerativer Proktitis oder Morbus Crohn.

23. Zusammensetzung nach einem der Ansprüche 1 bis 6 oder 13 zur Verwendung in einem Verfahren zur Behandlung des überaktiven Blasensyndroms, einer Blasenüberempfindlichkeit oder einer durch Colitis induzierten blasenafferenten Hyperaktivität bei einem Patienten.

## Revendications

1. Composition à libération retardée comprenant un comprimé à enrobage entérique, le comprimé comprenant :
du linaclotide ;
du Ca²⁺ ;
de l'histidine ; et
de l'alcool polyvinylique (PVA),
le linaclotide étant présent dans la composition dans une quantité de 30 µg à 300 µg.

2. Composition selon la revendication 1, dans laquelle le linaclotide est présent dans une quantité de 30 µg, 100 µg ou 300 µg.

3. Composition selon la revendication 1 ou la revendication 2, dans laquelle l'enrobage entérique comprend des copolymères d'acrylate de méthyle-acide méthacrylique (p. ex. de l'Eudragit®).

4. Composition selon l'une quelconque des revendications 1 à 3, dans laquelle l'enrobage entérique comprend de l'Eudragit® S100, de l'Eudragit® L100, ou un mélange d'Eudragit® S100 et d'Eudragit® L100.

5. Composition selon l'une quelconque des revendications 1 à 4, comprenant en outre un film de polymère ou un sous-enrobage protecteur.

6. Composition selon la revendication 5, dans laquelle le sous-enrobage comprend de l'Opadry II®.

7. Forme galénique unitaire comprenant la composition pharmaceutique selon la revendication 1.

8. Procédé de fabrication de la composition selon l'une quelconque des revendications 1 à 6, comprenant :
(i) la préparation d'une base de linaclotide, d'une charge prégranulée, et d'une base inactive ;
(ii) le mélange et la compression de la base de linaclotide, de la charge prégranulée, et de la base inactive pour former un comprimé ; et
(iii) l'application d'un enrobage entérique sur le comprimé.

9. Procédé selon la revendication 8, dans lequel la charge prégranulée est préparée par granulation à l'état humide et séchée avant le mélange et la compression pour former un comprimé.

10. Procédé selon la revendication 8 ou la revendication 9, le procédé comprenant en outre l'application d'un sous-enrobage sur le comprimé.

11. Procédé selon la revendication 10, dans lequel le sous-enrobage comprend de l'Opadry II®.

12. Procédé selon l'une quelconque des revendications 8 à 11, dans lequel l'enrobage entérique comprend de l'Eudragit® S100, de l'Eudragit® L100, ou un mélange d'Eudragit® S100 et d'Eudragit® L100.

13. Composition préparée par le procédé selon l'une quelconque des revendications 8 à 12.

14. Composition selon l'une quelconque des revendications 1 à 6 ou 13, destinée à une utilisation dans une méthode de traitement d'un trouble gastro-intestinal chez un patient.

15. Composition destinée à une utilisation selon la revendication 14, le trouble gastro-intestinal étant sélectionné dans le groupe constitué d'un syndrome du côlon irritable (SCI), d'une constipation, d'un trouble gastro-intestinal fonctionnel, d'une maladie de reflux gastro-œsophagien, des brûlures gastriques fonctionnelles, de la dyspepsie, de la diverticulite, des douleurs viscérales, des douleurs abdominales, de la gastroparésie, d'une pseudo-obstruction intestinale chronique, d'une pseudo-obstruction colique, de la maladie de Crohn, de la colite ulcéreuse, et d'une maladie inflammatoire de l'intestin.

16. Composition destinée à une utilisation selon la revendication 14, le trouble gastro-intestinal étant une constipation.

17. Composition destinée à une utilisation selon la revendication 16, la constipation étant une constipation chronique, une constipation idiopathique, une constipation idiopathique chronique, une constipation due à un iléus postopératoire, ou une constipation causée par l'utilisation d'opiacés.

18. Composition destinée à une utilisation selon la revendication 14, le trouble gastro-intestinal étant un syndrome du côlon irritable (SCI).

19. Composition destinée à une utilisation selon la revendication 18, le syndrome du côlon irritable étant un syndrome du côlon irritable à dominante constipation (SCI-c), un syndrome du côlon irritable à dominante diarrhée (SCI-d), ou un syndrome du côlon irritable mixte (SCI-m).

20. Composition destinée à une utilisation selon la revendication 14, le trouble gastro-intestinal étant des douleurs viscérales.

21. Composition destinée à une utilisation selon la revendication 14, le trouble gastro-intestinal étant des douleurs abdominales.

22. Composition selon l'une quelconque des revendications 1 à 6 ou 13, destinée à une utilisation dans une méthode de traitement ou de soulagement de la douleur chez un patient, la douleur étant sélectionnée parmi des douleurs viscérales ; des douleurs liées à une diverticulite ; des douleurs pelviennes ; des douleurs abdominales ; ou des douleurs associées à des troubles gastro-intestinaux, des maladies vénériennes, un syndrome de la vessie douloureuse, ou une cystite interstitielle ; ou la douleur étant sélectionnée parmi des douleurs associées à un syndrome du côlon irritable (SCI), une proctopathie radio-induite (que l'on appelle aussi proctite radique) chronique ou aiguë, des douleurs rectales, une proctalgie chronique, une proctalgie fugace, des douleurs anales, une fissure anale chronique, des douleurs anales postopératoires, une vessie hyperactive, une incontinence de stress, des douleurs associées à un cancer, des douleurs associées à des néoplasmes du tractus gastro-intestinal, une endométriose, une orchialgie, une prostatite chronique, une prostatodynie, une vulvodynie, un syndrome urétral, des douleurs péniennes, des douleurs périanales, et des douleurs associées à une colite ulcéreuse, à une proctite ulcéreuse, ou à la maladie de Crohn.

23. Composition selon l'une quelconque des revendications 1 à 6 ou 13, destinée à une utilisation dans une méthode de traitement d'une vessie hyperactive, d'une hypersensibilité vésicale ou d'une hyperactivité vésicale afférente induite par une colite chez un patient.
